# EUROPEAN PATENT APPLICATION

(11) **EP 3 578 648 A1**
(43) Date of publication of application: **11.12.2019**
(21) Application number: 18748611.3
(22) Date of filing: 12.01.2018
(51) Int. Cl.: C12N 9/42, C12P 7/08

(54) **POLYPEPTIDES WITH POLYSACCHARIDE MONOOXYGENASE ACTIVITY AND USE THEREOF FOR THE PRODUCTION OF FERMENTABLE SUGARS**

(30) Priority: 01.02.2017 ES 201730113
(71) Applicant: Abengoa Bioenergía Nuevas Tecnologías, S. A., 41014 Sevilla (ES)
(72) Inventor: MARTÍN PÉREZ, Lucía, 41014 Sevilla (ES); DÍEZ GARCÍA, Bruno, 41014 Sevilla (ES); REYES SOSA, Francisco Manuel, 41014 Sevilla (ES); VALBUENA CRESPO, Noelia, 41014 Sevilla (ES); MORENO PÉREZ, Antonio Javier, 41014 Sevilla (ES); PÉREZ GÓMEZ, Dolores, 41014 Sevilla (ES); PLATERO GÓMEZ, Ana Isabel, 41014 Sevilla (ES); GAVALDÁ MARTÍN, Sandra, 41014 Sevilla (ES); VIÑAS DE LA CRUZ, Laura, 41014 Sevilla (ES); ROCHA MARTÍN, Javier, 41014 Sevilla (ES); SÁNCHEZ ZAMORANO, Laura, 41014 Sevilla (ES); BERMÚDEZ ALCÁNTARA, María de los Angeles, 41014 Sevilla (ES); LEDESMA GARCÍA, Laura, 41014 Sevilla (ES); RAMOS MARTÍN, Juan Luis, 41014 Sevilla (ES)
(74) Representative: Pons
(86) International application number: PCT/ES2018/070023
(87) International publication number: WO 2018/142002

(57) **Abstract**

The invention refers to polypeptides with polysaccharide monooxygenase activity, to a host cell that expresses them, preferably a *Myceliophthora thermophila* cell that recombinantly expresses at least one of these polypeptides, to an enzymatic composition comprising at least one of these polypeptides, preferably together with other cellulolytic enzymes, the use of this host cell, of at least one of the polypeptides with polysaccharide monooxygenase activity or of the enzymatic composition for the degradation of cellulosic biomass and to a process for the production of bioproducts, preferably bioethanol, including the use of this host cell, of at least one of the polypeptides of the invention or of the enzymatic composition of the invention.

LISTADO DE SECUENCIAS

<110> Abengoa Bioenergía Nuevas Tecnologías, S.A.

<120> POLIPÉPTIDOS CON ACTIVIDAD POLISACÁRIDO MONOOXIGENASA Y SU USO
PARA LA PRODUCCIÓN DE AZÚCARES FERMENTABLES

<130> PCT1861.71

<150> P201730113
<151> 2017-02-01

<160> 70

<170> PatentIn version 3.5

<210> 1
<211> 1147
<212> DNA
<213> Byssochlamys spectabilis

<220>
<221> sig_peptide
<222> (1)..(63)

<220>
<221> Intron
<222> (102)..(153)

<400> 1
atgccgttct ctaagatagc tatcctcgct accattcttg gtaccgcatc tctggtttca 60

ggacatggat atgtccaagg gattgttgcc gatggaaaat agtatgttct gtagctatac 120

ctgacagtga gcgggtgctt actatcttta cagctatggc ggatatatcg tgacgcagta 180

tccgtacacc aacgaccctc ccgagctcgt cgcctggtct accgaagcaa ccgatcttgg 240

atttgtggac ggaagtggct atgcttcagg tgatatcatc tgccacaaga gcgcacagcc 300

tgccgccctg gctgctgagg taccagctgg agggaaagta gagcttcaat ggaccgaatg 360

gcctgagagc caccacggtc ctgtgcttga ttaccttgct ccttgcaacg ggaattgcgc 420

cgacgttgac aagaccgccc tggaattctt caagattgac cagggtggtt tggtcgatga 480

cagcagccct ccaggcacct gggcctctga tcaattgatt tccaacaaca actcctggac 540

cgtcactatt ccctccacaa tcgccccagg taattacgtt ctgaggcacg aaatcattgc 600

ccttcactcc gccggaaaca aggatggagc tcagaattat cctcagtgta tcaacataaa 660

agtcactggt tccggtaccg attctccttc tggcactctc ggcacgtccc tttataagga 720

cactgatcct ggcattctgg tcaacattta tcaaacgctg tccgattatt ccattccagg 780

ccctgcgcta tatgaaggtg gcagtagctc tggcacatcc tctggctctg gaacttcgac 840

ggccacttcg acctctgtgg catcttccac ctcagttaag ccttccagta ttagcactcc 900

ttcctcagtt caagtggcta tcccatctgc caaaacctca gctgttcctg ctacgacgca 960

ggcacctacc acagctactc ctgcggtcac gattccaact gaaagcgcct tgaccgatta 1020

cctcaacagt atgagtgcag atgaggtcct gaatctcgtt cgtggtacta tgtcctggtt 1080

gttcacgaac aagaatcacg ctcgagattt atctgctagt tctgagctta ctgatcgccc 1140

catgtaa 1147

<210> 2
<211> 750
<212> DNA
<213> Penicillium oxalicum

<220>
<221> sig_peptide
<222> (1)..(63)

<400> 2
atggtatctt caaaagccac caccctatct gccatgttgg catgtgtctc tatggtcgca 60

ggccatggtt tcgtttccag cattatggca ggtggcaaaa actacactgg ctatctcgcc 120

aactcctacc catacatgag caatccaccc cagagcgtcg gctgggccac cacagctacc 180

gatctaggct ttgaagacgg caccgaatat caatcggcca acatcatctg ccaccgagat 240

ggcaagaatg ccgcactctc tgcacccgtc accgcaggct ccaaggtgga aatccaatgg 300

acctcatggc cagacagcca ccatggtccc gtcatcacat accttgcctc ctgtggtgga 360

gactgcagca cagtcgacaa gaccacgctc aagttcttca agatcgatga ggccggtctg 420

attgatgact ctaaccctcc cggaacgtgg gccacggatc agctcattgc agctggaaac 480

cgatggacgg tgactatccc caagagcatt gcatcgggga actatgtcat gcgtcacgag 540

atcatcgctc ttcattcttc gggttccaag gatggtgcac agaactatcc ccagtgtttg 600

aatctgcagg tcaccggtgg agggagcgct aagcctcagg gtactttggg tgaggctctg 660

tacaaggaca ctgatcctgg cattttgatc aatatctacc agtccttgaa gagctacgtg 720

attcccggtc cagctctgta cagcgcgtga 750

<210> 3
<211> 1032
<212> DNA
<213> Aspergillus clavatus

<220>
<221> sig_peptide
<222> (1)..(57)

<400> 3
atgtctgtca ctaagatcgc tggtatcctt ctcggctccg ccgccatggt cgctggtcac 60

ggtttcgtga ccggtgccgt tgtcgatggc acataccaca cgggttatct tgtcaacaac 120

tacccctact cgagcaaccc tcccaaaacc attggctggt ctgagaccgc caccgacctc 180

ggctttgtcg acggcactgg ctacgccagc ggcgacatca tttgccacaa gaacgctaag 240

cctggagccc tctctgcgga catcaaggct ggtggaaagg tggagttcca atggacccag 300

tggcccgagt ctcaccacgg ccctgtcatc acttatatgg ccaactgcaa tggcgactgc 360

gcctcggttg acaagtctca gctggagttc ttcaagattg aggagaaggg tctcatcggc 420

tccggacact gggcctcgga tgacctgatt gccaacaaca acagctggac cgtgaccgtc 480

cccagcagcc tggccgctgg caactatgtc atgcgtcacg agatcattgg cctccactcg 540

gctggcaacg cgaacggggc ccagaactac ccccagtgca tcaacctcaa ggttaccggt 600

ggtggcagcg acaagcctgt tggcaccctg ggcaccaagc tctacaaggc caccgacccc 660

ggtatccttg tcaacatcta cggcagcctg acctcctaca cgatccccgg ccctgctctc 720

tggaccggtg cttcttctgg cggctccgat gacaacggtt ccactcccgc ttccactccc 780

actcccaccg ccgccccctc ctccactgct gcccccagcg tgaccatcac cagcacccct 840

ggacagaaga cccagacccc tgtcgccacc cccgccccgt ctacccccag ctcgaccccg 900

ggtcttccca ccgacggcaa cctgaccgac tacttcaagt ccctgagccc tgagcagttc 960

ctcagcgtcc tcaaggagac cttttcctgg cttgttaccg agaaggttca cgctcgctcc 1020

ctctctgctt ag 1032

<210> 4
<211> 1178
<212> DNA
<213> Aspergillus fumigatus

<220>
<221> sig_peptide
<222> (1)..(57)

<220>
<221> Intron
<222> (325)..(380)

<400> 4
atgtctgtcc ctaagattgc agctgctctt ctcagctcgg ccgctctggt cgctggtcac 60

ggttttgtga cgggtgccgt tgttgatggc aaatactaca ctggatacct tgtcaaccaa 120

tacccttaca tgagcagccc tcctgacagc atcggttggt ctgaaaccgc taccgatttg 180

ggtttcgtcg acggcagcgg ctactctagc ggcgacatca tctgccacaa ggatgctaag 240

aatggtgcca tctctgccga gatcaaggcc ggtggaaagg ttgaattcca atggactgaa 300

tggcctgaat ctcaccacgg accggtatgt cttcgtaaga catccatgga acaattggtt 360

agggtattaa cttcgtaaag gtcattacct acatggccaa ctgcaatggt gattgcgcct 420

cagttgacaa gactactctg gagttcttca agattgatga gagtggtctg atcagtgact 480

ccaatgttcc tggcacctgg gcctctgaca acctgatcgc caacaacaac agctggaccg 540

tgactgttcc cagctccatc gccgctggta actatgtcat gcgtcacgaa attattgccc 600

tccactccgc tggcaaccag aatggtgccc agaactaccc tcagtgcatc aaccttaagg 660

tcaccggtgg tggcagcgac aagcctgcag gtaccctcgg cactgcgctc tacaagaaca 720

ctgatgctgg catcctggtc aacatctacc agagcctgag ctcctatgaa attcctggtc 780

ccgctctcta ctctggcgct tcttctggca gctccaacaa cggtggttcc gcctcgtcta 840

gcgccactgc tccttctgcc accatcactc agccctctac tgcagtcccg actagctctg 900

cgactgctta ccagccctcc actaccaccg aggctgtcac agtcaccagc attcctgccc 960

agcagagcta cgtccaggct cctaccgcca cccctagctc cactgccggc agctccggct 1020

ccggctccgg ttctagctcc agtggcactc ttcccagcag cagcaacctc actgagtact 1080

tcaactccct cagcgccgaa gagttcctga aggtccttaa gcagaccttc tcttggctgg 1140

ttacggagaa ggtgcacgct cgtgatctct ctgcttag 1178

<210> 5
<211> 1209
<212> DNA
<213> Aspergillus niger

<220>
<221> sig_peptide
<222> (1)..(57)

<400> 5
atgtctgtcg ctaagattgc tggtgtcgtc ctcggctcgg ccgcccttgt tgctggccac 60

ggttatgtgt ctggtgctgt catcgacggc gagtactacg gaggatacat cgtctcctcc 120

tacccctatg agagtgaccc tccggagacc attgcctggt ctaccacagc cactgacctt 180

ggcttcgtcg acggctctga atactcggac cctgacatca tctgccacaa gagcgctaag 240

cctggtgcta tctccgctga cgtcaaggcc ggtggtactg ttgagcttca gtggactgac 300

tggccttcca gccaccacgg tcccgtcctg acttaccttg ccaactgcaa cggtgactgc 360

tccgatgtca ccaagactga ccttgagttc ttcaagatcg acgagagcgg cctcattagc 420

gacactgaag tccccggaac ctgggccact gacaacttga tctccaacaa caacagctgg 480

actgtgacca ttccctcgac cctcgaggct ggtaactacg tcctgcgcca cgagatcatt 540

gccctgcact ccgctgagaa caaggatggc gcccagaact acccccagtg tctcaacctg 600

aaggttaccg gcagtggtag ctccacctac tccggcacca agggtgaagc cctctacaag 660

gacaccgacc cgggtatcct ggtcaacatc tacgagaccc tgagctccta cgacatcccc 720

ggtcctgcta tgtacaacgc cacttcctcc agcagctctt ctggcagctc ctccggtgcc 780

tcttctacca gctccgctgc cgctgccgcc gccactactt ccagtgcctc gacttccagc 840

gcttccaccc ccaccaccag cgctgccgct gcgactagct ccgccgtaag cgccaccacc 900

actgctgctg ctgccgttgt caccgacgtt gaaaccaccg ttcagactgc ggtcgccact 960

gcttaccaga cttcgactga cgttgtgcag gtcactgtca ctggcagcgc cccccagcag 1020

acccacatcc aggtcgccag ctccagcgcc gctgcttcca cccccagcag ctccagtgtc 1080

gccgtctcca gcgacaacct caccagctac ttcagctctc tgagcgccga ccagttcctc 1140

agcgtcctga aggagacctt ctcttggctg gtcactgaga agaagcacgc ccgtgacctc 1200

aacgcctaa 1209

<210> 6
<211> 1209
<212> DNA
<213> Aspergillus niger

<220>
<221> sig_peptide
<222> (1)..(57)

<220>
<221> Intron
<222> (732)..(878)

<400> 6
atgtctgtcg ctaagattgc tggtgtcgtc ctcggctcgg ccgcccttgt tgctggccac 60

ggttatgtgt ctggtgctgt catcgacggc gagtactacg gaggatacat cgtctcctcc 120

tacgcctatg agagtgaccc tccggagacc attgcctggt ctaccacagc cactgacctt 180

ggcttcgtcg acggctctga atactcggac cctgacatca tctgccacaa gagcgctaag 240

cctggtgcta tctccgctga cgtcaaggcc ggtggtactg ttgagcttca gtggactgac 300

tggccttcca gccaccacgg tcccgtcctg acttaccttg ccaactgcaa cggtgactgc 360

tccgatgtca ccaagactga tcttgagttc ttcaagatcg acgagagcgg cctcatcagc 420

gacactgaag tccccggaac ctgggccact gacaacttga tctccaacaa caacagctgg 480

actgtgacca ttccctcgac cctcgaggct ggtaactacg tcctgcgcca cgagatcatt 540

gccctgcact ccgctgagaa caaggatggc gcccagaact accctcaatg tctcaacctg 600

aaggttaccg gcagtggtag ctccacctac tccggcacca agggtgaagc cctctacaag 660

gacaccgacc cgggtatcct ggtcaacatc tacgagaccc tgagctccta cgacatcccc 720

ggtcctgcta tgtacaacgc cacttcctcc agcagctctt ccggcagctc ctccggtgcc 780

tcttctacca gctccgctgc cgctgccgcc gccactactt ccagtgcctc gacttccagc 840

gcttccaccc ccaccaccag cgctgccgct gcgactagct ccgccgtaag cgccaccacc 900

actgctgctg ctgccgttgt caccgacgtt gaaaccaccg ttcagactgc ggtcgccact 960

gcttaccaga cttcgactga cgttgtgcag gtcactgtca ctggcagcgc cccccagcag 1020

acccacatcc aggtcgccag ctccagcgcc gctgcttcca cccccagcag ctccagtgtc 1080

gccgtctcca gcgacaacct caccagctac ttcagctctc tgagcgccga ccagttcctc 1140

agcgtcctga aggagacctt ctcttggctg gtcactgaga agaagcacgc ccgtgacctc 1200

aacgcctaa 1209

<210> 7
<211> 1035
<212> DNA
<213> Aspergillus ruber

<220>
<221> sig_peptide
<222> (1)..(57)

<400> 7
atgtctgtca ccaagatcac tggtcttctt ctcggctcgg ccgctatggt cgctggtcac 60

ggttatgtct ccggtgccgt tgtggatggc caatactacg gaggatacat cgttacctcc 120

tacgcttaca ccgacagccc cccagaaacc attggatggt cgaccgaagc tactgatctg 180

ggctttgtct ctcccagctc cttctctgac ccggatatta tctgtcacaa gagtgcgcag 240

cccggtgcca tctctgcttc ggtggcagct ggtaaggatg ttgagcttca gtggactgaa 300

tggccggaga gtcaccatgg tcctgtcatc acctacctgg ccaactgcaa cggtgactgc 360

tccaaggtcg acaagacctc gctcgagttc ttcaagatcg accagaaggg tctcgtcgac 420

gacagcaacg tccctggcac atgggctagc gacaatctga tttccaacaa caacagctac 480

accgtcacta ttcccagcga cattgctgcc ggtaactacg tcctgcgtca tgaaattatt 540

gccctgcact ccgctggaaa cgaggacggt gcccagagct accctcaatg tgtcaacctc 600

aaggttactg gtggcggcag cgcatctccc tcgggtactc ttggtactaa gctctacagc 660

gaggacgacc cgggtatcct cgtcaacatt taccagcagc tcgactccta cgaaatccct 720

ggccctgctc tgtactctgg cgcttcttcg tcctccaact ctggctcttc ttccagcact 780

gcttcggctt ctactactgc cacttctgcc actacttcgt ctgcctcgtc cacccaagct 840

tccgctaccc ctgcctccca agccaaggcc caagtctcta gctctactcc cagcgcttcg 900

tccgttgcga cttctggtag cttgtccgac tacttcagct ccctgagcgc tgaggagttt 960

ctcaacgtta tcagcgagac tctgtcctgg ttggttactg acaagatcca cgcccgtgac 1020

atctcgaccg cataa 1035

<210> 8
<211> 1209
<212> DNA
<213> Aspergillus terreus

<220>
<221>  sig_peptide
<222>  (1)..(57)

<400>  8
atgtctgtcg ctaagattgc tggcgttgtc ctcggctctg cggcccttgt cgctggccat      60

ggttacgtta cgggtgccgt cgtcgatggc aagtactatg ctggctacga tgtcacctct     120

gccccttact ctagcgaccc tccggccacc atcggctggt cgaccaccgc caccgacctc     180

ggcttcgttg acggcaccga gtactccgag cctgacatca tctgccacaa ggacgctaag     240

cctggctctc tctctgctga aattactgcc ggtggcaagg tcgagttgca gtggaccgaa     300

tggcccgaga gccaccacgg ccctgtcatt acctacctgg ccaactgcaa cggcgactgc     360

tcctcggtcg acaagacttc gctggagttc ttcaagattg atcagaaggg tctcattgag     420

ggcaacacct gggctagtga ccagctgatc agcaacaaca acagttggac tgtcaccatc     480

cccagcagca ttgcttctgg caactatgtc ctgcgccacg agatcattgg ccttcactcc     540

gctggcaaca aggatggcgc ccagaactac cctcagtgca tgaacctcaa ggttactggt     600

ggcggcagcg acaagccggc tggcaccctt ggtaccgctc tgtacaagga cactgaccct     660

ggtatcctcg tcaacatcta ccagactctg tcctcgtacg tgatccccgg tcctgccctc     720

tactctggca gctcatccgg ctcgggttcg ggctcagcta gctccagcgc tgctgctact     780

ccttcctcca gcagtgttcc ctccagcacc agcattgccg ttcccaccag ctctgctcct     840

gcccccacct ccgccgctcc ttcggctcaa accagcactg ctactgccta ccagactgcc     900

accactattg agagtgtcac cgttaccgcc gaagcgcccc agcagaccaa ggtgcaggac     960

cctagctccg gctctagctc tggttccaac tctggctcca gctccggttc tagctctgga    1020

tccagcactg gctctagctc cggctccagc actggcactg gctcctccag tgttcccact    1080

cctagtggta gcagcagcct gtctgactac ttcaactccc tgagtgccga gcagttcctg    1140

aaccttctcc gccagactct ttcttggctg atcaccgaca agaagcacgc ccgtgacctc    1200

tctgcctag                                                            1209


<210>  9
<211>  1160
<212>  DNA
<213>  Neosartorya fischeri


<220>
<221>  sig_peptide
<222>  (1)..(57)

<400>  9
atgtctgtct ctaagattgc agctgttctt ctcagctcgg ccgctctggt tgctggtcac      60

ggattcgtgt cgggtgccgt tgttgatggc aaatactaca ctggatacct cgtcaaccaa 120

tacccttaca tgagcagccc tcctgagagc atcggttggt ctgaaaccgc taccgatctg 180

ggtttcgtcg acggtagcgg ctactctagc ggcgacatca tctgccacaa gagtgctaag 240

aatggtgcca tctctgccga gatcaaggcc ggtggaaagg ttgaattcca atggactgaa 300

tggcctgaat ctcaccacgg accggtatgt cttcgtaaga catccatgga ataatcggtt 360

agggtattaa cttagtacag gtcattacct acatggccaa ctgcaatggt gattgcgcct 420

cagttgacaa gaccagtctg gaattcttca agattgatga gagtggtctg atcagtgact 480

ctaatgttcc tggcacctgg gcctccgaca acctaatcgc caacaacaac agctggacgg 540

tgactgttcc cagctccatc gccgctggta actatgtcat gcgtcacgaa atcattgccc 600

tccactccgc tggaaaccag aacggtgccc agaactaccc tcagtgcatc aaccttaagg 660

tcaccggtgg tggcagcgac aagcctgcag gtaccctcgg cactgcgctc tacaagaaca 720

ctgacgccgg catccttgtc aatatctacc agagcctgag ctcctatgac attcctggtc 780

ccgctctcta ctctggcgct tcttctggca gctccaacag cggtggttcc gcctcgtcta 840

gcgccgctgc tccttctgcc accatcactc agacctctac tgcagtccag actagctctg 900

cgactgttta ccagccctcc actaccaccg aggctgtcac cgtcaccagc actcctgccc 960

agcagagcta cgtccaggct cctaccgcca cccctagctc cactgccggc agctccagct 1020

ccagcggcac tcttcccagc ggcagcagcc tcaccgagta cttcaactcc ctcagcgccg 1080

aggagttcct gaaggtcctt aaggagacct tctcttggct ggttacggag aaggtgcacg 1140

ctcgtgatct ctctgcctag 1160

<210> 10
<211> 1203
<212> DNA
<213> Aspergillus kawachii

<220>
<221> sig_peptide
<222> (1)..(57)

<400> 10
atgtctgtcg ctaagattgc tggtgttgtc ctcggttcgg ccgcccttgt tgctggccac 60

ggttatgtgt ctggtgctgt cgttgacggc cagtactacg gaggatacat cgtctcctcc 120

tacgcctatg agagtgaccc tccggagacc attgcctggt ctaccgaagc cactgacctt 180

ggctttgtcg acggttctga atacgccgag cctgacatca tctgccacaa gagcgctaag 240

cctggtgcta tctccgctga cgtcaaggct ggtggtactg tcgagcttca gtggactgac 300

tggccctcca gccaccacgg tcccgtcctg acttaccttg ccaactgcaa cggtgactgc 360

tccgatgtca ccaagactga ccttgagttc ttcaagatcg acgagagcgg ccttatcagc 420

gacactgaag tccctggaac ctgggccacc gacaacttga tctccaacaa caacagctgg 480

actgtgacca tcccctcgac cctcgagtct ggtaactacg tcctgcgtca cgagatcatt 540

gccctgcact ccgctggaaa caaggatggc gcccagaact accctcagtg cctcaacctg 600

aaggttaccg gtagcggtag ctccacctac tcaggcacca agggtgaagc cctctacaag 660

gacaccgacc cgggtatcct ggtcaacatc taccagagcc tgagctccta cgacatcccc 720

ggccctgcta tgtacaacgc cacttcttcc ggcagctcct ccggcagctc ctccagtgcc 780

tcttctacga gctccgctgc cgctgccgct gctactactt ccagcgcttc cagctccacc 840

accagcgctg ctgctgcgac tagctccgcc gtaagcgcca ccaccaccag cgctgctgct 900

gctgccgttg tcaccgacgt tgaaaccacc gtccagactg cggtcgccac tgcttaccag 960

acttcgactg acgttgtgca ggtcactgtc actggcagcg ccccccagca aacccacatc 1020

caggttgcca gctccagcgc cgccgctgtt gctaccccca gcagctccag cgtcgccgtc 1080

tccagcgaca acctcaccag ctacttcagc tctctgagcg ccgaccagtt cctcagtgtc 1140

ctgaaggaga ccttctcctg gctggtcacc gagaagaagc acgcccgtga cctcaacgcc 1200

taa 1203

<210> 11
<211> 1357
<212> DNA
<213> Aspergillus nidulans

<220>
<221> sig_peptide
<222> (1)..(57)

<220>
<221> Intron
<222> (320)..(371)

<400> 11
atgtctgtcg ctagaactgc tggttttgcc ctcgcctccg cggccatcgt cgccggtcac 60

ggttatgtca cgggaatcgt cgctgacggc acatactacg gcggttacct cgttaaccag 120

tacccttaca gcaacgaccc ccctgcagtt gttggatggg ctgaggatgc taccgacctg 180

ggtttcgttg atggctccgg ctacacctct ggcgatatca tctgccacaa ggacgctacc 240

aacgcccagg cttctgccac cgtcgctgct ggtggcaccg ttgagctgca gtggactgag 300

tggcctgaat cccaccacgg tgagtacaaa ctttagctct ctgaacatcg cattcactta 360

tacatgcaaa ggtcccgtca tcgactacat tgccagctgc aacggtgact gcacaaccgt 420

cgacaagacc accctcgaat gggtcaagat tagcgagagc ggtctcgttg acggcagctc 480

tgcccctggc acctgggcct ctgacaacct gatctccaac aacaacagct ggaccgtgac 540

catccccagc agcctcgccg ccggtggcta cgtcctccgc cacgagatca tcgccctcca 600

ctccgccggc aacgagaatg gcgcccagaa ctacccccag tgtgtcaacc tcgaggtcac 660

tggcggtggc agcgcctctc cctctggtac cgtcggtact gagctttaca cccccactga 720

ccctggtatc ctggtcaaca tctacacctc cctcgactcc tacaccatcc ccggccctgc 780

tctctgggac ggtgcctctt ccagcggcgg aaacagcggc agtggtagcg cctcctccag 840

cgccgccgcc acctccaccc ctaccactcc cagcgtaagc gtccctgtca tccccaccgc 900

tagctctggt gcctcctcta cccctctcgt gcccaccccc agcgcgcctg ctgtgacccc 960

ttcggtccct gccggcaacc aggcccctca gcccacttac acctccacct acattgagac 1020

tgagactttg cctggtcaga ccgttacctc caccacgacc gagtacgctt ccgagcctac 1080

ccagcccgct gtcgagaccc aggttgctca gccctctgag accgaggctg ccacatccac 1140

ttccactgtc accgagactg cctctgccac cgccgccccg actggctcct ctggctctag 1200

ctctggatct ggctccagca gcaccgagct ccccaccgac agctccagcc tctccgacta 1260

cttctcctcc ctgagcgcag aggagttcct caaccttctg aaggagaccc ttaagtggtt 1320

ggtcaccgac aaggtccacg ctcgttccct ccactaa 1357

<210> 12
<211> 806
<212> DNA
<213> Aspergillus oryzae

<220>
<221> sig_peptide
<222> (1)..(63)

<220>
<221> Intron
<222> (102)..(154)

<400> 12
atggctatgt ccaagatcgt gtcgcttact ggcctcctgg cctctgcctc tctcgtcgcc 60

ggccacggtt atgtgtctgg agtcgtcatt gatggtcaat agtatgattc taaattcgca 120

aaaaagtcat tataaaggct aacacccacc atagctatgg tggatacctt gttgacaagt 180

acgcctactc ggacaatgcc ccagatacca ttggctggac cacctctgcc accgaccttg 240

gattcgtgga cggcactgga taccagtccc ccgatatcat ctgtcacaag gacggagcgc 300

caggtgcttt gactgctgaa gttgctgctg gtggcaaaat tgaactgcag tggactgagt 360

ggcctgaaag tcaccacggc cctgtcctca attacctcgc accctgcggc ggcgagtgct 420

ccgcggtgga caagacaact ttagagttct tcaagatcga ggccaagggc ctgattgacg 480

gcaccacccc cccgggtcag tgggctaccg atgacttgat cagcaacaac aacagctaca 540

ctgtgaccat cccgacatcc atccaagagg gcaactatgt tctccgccac gagatcatcg 600

gccttcactc tgccggtcag aaggatggtg ctcagaacta cccccagtgc atcaatatca 660

aggtcactgg cggcggagac gccacgccgg caggtaccgc cggcgaggcc ctttacaagg 720

acactgacgc tggtattctg ttcgacatct acagtgacct cagcggcggc taccccatcc 780

ctggccctga ggttttcagc gcttaa 806

<210> 13
<211> 1113
<212> DNA
<213> Aspergillus oryzae

<220>
<221> sig_peptide
<222> (1)..(57)

<400> 13
atgtctatcg ctaagattgc tggtgtcgtc ctcggctcgg ccgctttggt cgccggtcac 60

ggttacgtct ctggtgccgt cgttgacgga cagtactact ctggatatga catgtcctac 120

cactatatgt ccaacccccc tcaggtcatt ggatggtcga ccgatgccac cgacctcgga 180

ttcgttgacg gtagctcata tgccgatgcc gacattatct gccacaagaa cgccaaaaac 240

ggcgctatct ctgctgagat cgctgctggc aagcaggtcg agctgcagtg gactgcctgg 300

cccgagagtc acaagggtcc tgtcatcacc tacctcgcca actgcaacgg cgactgcgcc 360

accgtcgaca agacccagct cgagttcttc aagatcgatg agaagggcct catcagcggc 420

agtgacaaca cctgggcctc cgacaatctg atctccagca acaacagctg gaccgtgacc 480

atccccagca gcattgctgc cggcaactat gtcatgcgtc acgaaatcat tgcccttcac 540

tccgctggta acaaggatgg tgctcagaac tacccccagt gtctcaactt caaggttacc 600

ggcggtggta gcgacaagcc cgagggtacc ctcggtactg ctctctacaa ggacaccgac 660

cctggtattg agatcaacat ctaccagacc ctgtcctcct acaccatccc cggccctgct 720

ctctacactg gcagctcttc cagcggctcc tccggctccg gcagctccag cgccgctccc 780

tcccctactg ccagtgcttc tgcttccgcc actgctgccc ctgtccagac tagcaccgct 840

actgcctacc agacctccac tgctgttgcc agcgtcactg ttactggctc tgctcctgct 900

cagacccacg tccaggccac cagctcttca gctgctgctt ccactcccac cgctagctct 960

ggcgccagca ctggctctgg ctctggctcc agctccagcg acctgaccag ctacttcaac 1020

tccttgagcg ctgacgagct cctgaacgtt atcaagcaga ctctgtcttg gcttgtcacc 1080

gacaagatcc acgctcgtga catctctgcc taa 1113

<210> 14
<211> 1474
<212> DNA

<213>  Baudoinia compniacensis

<220>
<221>  sig_peptide
<222>  (1)..(57)

<220>
<221>  Intron
<222>  (162)..(222)

<220>
<221>  Intron
<222>  (973)..(1227)

<400>  14
atgtcagtca tccagtctgc ggccatcctt gcgtcgctgg ttgcctccgt cgcagcacac      60

ggacatgtcc agggcatcgt cgctggtggc acctactaca ccggctacga tccttcgttc     120

caatactccc ccatcccacc catcgttatc ggctggtccg agtaagtccg cggactgccc     180

tgttacaaga ctgttcacgc aacgctaacg gcttcccacc agcccacaag acctggacaa     240

tggcttcata gcgccgagca actacaccaa tccggacata atctgccacc gcaacgccac     300

cgcggctggc acctccgccc gcatcgccgc cggcgacgtc gtcgaacccc agtggacgcc     360

ctggccgagc agtcaccacg gcccggttat tgactacctc gccaaatgcc ctgggtcctg     420

ctcagacgtc gataagacga ccctcgagtt cttcaagatc gatcaggttg gactgatcga     480

cgataccacg gagccaggta cttggggctc ggatcaactc ctcgccaaca acaacagctg     540

gaccgttacc atccctccct ccatcgcacc gggcaactac gtcttgcgcc acgaaatcat     600

cgcgctacac tctgcgggca cggcggacgg ggcgcagaat tacccgcagt gcgtcaacct     660

ggaaattacg gggagcggga ctgatactcc ggctggcacg ctggggcaga agctgtatgg     720

tgctaatgac tctgggattc tgatcaacat ctatcagagt ttgagcacgt atgaaatacc     780

gggtccgagt ttgtacagtg gcgctgtggc gatgagccag actcaggtgg ctattacggg     840

atatgcgccg cttagttctg gtggcgttgc ggtatcgatt gcttatagtt ctgccacaac     900

gacgacggcc gcggcaggat cttcgtctgt ggcgcgttcg actaccgctg cgtctgcgtc     960

tgcgtcgacc gcgttagtgg gaagctatgc gtggtcgtcg acaagtattc gggtgtctgg    1020

aacgccatct tcgacagctt taccggcggc atcatcttcg cacgtatctg cgagcaggcc    1080

aacagcctcg gtatcaccac cggcggtcat caccgtcgtc accacgctca acaccaccac    1140

taccattagc cctactacga ctcactcttc ccacaccact ctcacgaccg ccaccgtccc    1200

aacctccctc ctcgccgccg tctcctccgc cgtcgtaccc ctcgaaagca tcgtctctgc    1260

cctcccgagc acggcgttga cgcagacact gcccactgtc ttaccgactg gaacggcggt    1320

gtcgagcaac gacacaatcg ccggcttgcc gcccgcagag gcacctccgg caggcacgac    1380

attgcaggat ctcttcgcat ggctcgaata tatgcttgag gggctagcga gtactggagg    1440

acgacatcag agggatttta aagagcggaa gtaa                                1474

<210>  15
<211>  795
<212>  DNA
<213>  Penicillium roqueforti

<220>
<221>  sig_peptide
<222>  (1)..(54)

<220>
<221>  Intron
<222>  (93)..(149)

<400>  15
atgacacttt caaagctatc tacgctccta gcctctgcct ctcttgttgc aggccacggc      60

tatgtctcta gcatcgtagc tgatggcaag aagtaagtca atctaagaaa caaagtctcc     120

ataccaataa caactaacca cattatcagc tacactggct acatcgttga caaatacccc     180

tacatggcag acccccctga cagtgtcggc tgggcaacaa cggcaaccga cctcggtttt     240

gaagacggca ccgaatacca agatccgaat ataatctgcc accgaaacgg agtcaacgca     300

gctttgagcg ccaccgtcac cgctggctcc agtatcgatc tccaatggac gacctggcca     360

gactcacatc acggtcctct gataacttac ctcgcctcat gcaacggtga ctgcagcaca     420

gtcgacaaga cgacactaaa gttcttcaag attgaagaag atggtctcat cgatggtgca     480

actcctcctg gtacatgggc taccgatgat cttattgctg ctggaggcca gtggactgtg     540

aagatccccc ccactcttgc tgcgggtaac tatgtgatgc gccatgagat tatcgctctg     600

cactctgctg gccagttgaa tggcgctcag aactacccac agtgcttcaa tttggaaatc     660

actggcggtg gaagtgatgt gccggatggc acgtttggtg gggagttgta tatcaatact     720

gatcctggga tcctgttcga tatttattct acgcttactt cgtatattat cccgggaccg     780

gctttgtatg cctga                                                      795

<210>  16
<211>  1007
<212>  DNA
<213>  Sclerotinia sclerotiorum

<220>
<221>  sig_peptide
<222>  (1)..(54)

<400>  16
atgtctttct ctaagctcac cactttcgcc ggcctggtcg ccacagttgc tgcccatggt      60

tatgttactg gtattgtagc tggtggaact tagtaagtta aatcttggag ccatgtattc     120

aaacatactg attcaaaaat agtaccaaag gatggctcgt caactatgca taccaaaacc 180

caatgccaga gtcaattggt tggtctgacc aaaatctcga ccttggattc actgcaccat 240

ccgcctatgc cactggggat atcatctgtg ctaaaaatgg taccaatgct gctctttcag 300

ctaaggttgc tgccggtgac accgttgatt ttcaatggac tgtttggccc gattctcata 360

aaggcccagt catcacctac ttggcaaact gcaacggaga ctgcagtacc gtcgataaaa 420

ccaagcttga attcttcaag atagacgaag gaggattgat cgatgacacc actgtccctg 480

gaacctgggc ttctgacaac ttgatcacca ataacaatac atggacatcc accattccag 540

ccgatctagc tcctgggaac tacgtcgctc gtcatgaaat tatcgctctt cacggtgcgg 600

gaagtaatga tggtgctcaa aattaccctc agtgtatcaa cttggaagtc actggtagcg 660

gtactcttgc tccatctggt actctcggtg aaaagcttta cactcctacc gatccaggta 720

tcttggtcaa tatctatgct aaattgacct acactattcc tggtccacct atgtacaacg 780

caactggagc agcaactgga gcagcaactg gagccactac cccagccacc ggtaaaaccg 840

cctcagcaac ctccgccgcc gccgctcttt ccaccccagc tgttgtttct acccctccaa 900

ctccaaccac aatgattaca tccgtcgctc ctgccgctac ccctgctacc tctgctgccg 960

cttcatctgg tgctagtggt gatgatgacg acacctgtga agcatga 1007

<210> 17
<211> 1449
<212> DNA
<213> Penicillium roqueforti

<220>
<221> sig_peptide
<222> (1)..(57)

<400> 17
atgtctgtct caaaaattgc gggtctcctg cttagctcgg ccgccatggt cgctggtcac 60

ggtttcgtgt cgggtgccgt tgttgatggc acttaccacg ggggcttcat cgtcaataca 120

tacaactaca tgaccgaggt gcctgcgaac attggatgga gtgaaaaggc caccgacctc 180

ggctacatcg acggctctgg ctacactggc tccgacatta tttgccacaa ggaagccact 240

ccgggtgcga tctccgccga ggtcaaggcc ggtggctctg tcgagctgca gtggactgac 300

tggcccgaga gccaccacgg cccggtcatt acctacatgg ctaactgcaa tggcgactgc 360

tccgtggtcg acaagacaac cctcaagttt ttcaagatcg ccgaggccgg tctgatcgac 420

gacactaacg tccccggtac atgggcaagt gaccaactca ttgctgccaa caacagcgcc 480

actgtgacta tccccagcac catcgccgct ggtaactatg ttctccgtca cgagatcatt 540

gccctgcact ccgccggcga tgcaaacggc gcccaaaact acccccagtg tatcaacctg 600

aaggtcaccg gcggcggcag cgactcccct gacggcgtcc tcggcacagc gctctacacg 660

cccaccgatc ccggtatttt gatcagcatc tacagcgcac tggagtccta tgtgatcccc 720

ggccccgctt tgtacaccgg cgcttcatcc ggctcttcat cgacctcctc gaccaccgcc 780

gcagccacta ccgaagccac taccgcgtct gctactgttt ctaccaccgc ggcccccacc 840

gtggctacta ccagtgcctc aaccgtgagt gcctctccta tccagagctc ctcttcgcac 900

cacctcaccc gcacccgcac ccgcactgcc cgtcctacgt acagccctac tttctcgtct 960

gctcccacgt ctgctgtctc aacttccgcc cctagtgggc cagattatga gcccactgat 1020

gctccttcca ccctcatcga gaatgcgact gccaccgctt cctctgctcc tcaggttact 1080

gacgagcctg ctacacagac cgccagcacc gccgacagca tcaccatcac caccaccgcc 1140

ggtggtctgt ctgcccagag ctccgcagcc gtcctcgagt ctagcgccac ttccactgtc 1200

actgccgagg ctactgctac tgctgcccct accgccgcag ccaccacttc cacctctagc 1260

accagctctg gatcttcttc ctccagctcc acctccagct ccggatcttc ttccactagc 1320

tccacctcta cctccaactc ctcgtacctc agctccctga gcgccgagaa gctcctcgag 1380

gtcatccggt cgaccctgaa gtggcttgtc tctgacaaga aggtgcacgc ccgtgctcta 1440

gcttactaa 1449

<210> 18
<211> 796
<212> DNA
<213> Penicillium italicum

<220>
<221> sig_peptide
<222> (1)..(54)

<400> 18
atgacacttt caaagctatc tatgctccta gcctccgcct ctcttgcggc aggccatggc 60

tacgtctcca gcatcgtagc taatggcaaa aagtgggtta atccaatacc aaaaatctcc 120

ataccaacaa gtaactaacc acatcaccag ttatactggc tacatcgttg acaaatacgc 180

ttacatgtca gacccccctg acagtgtcgg ctgggcaaca accgcaaccg atctcggatt 240

cgaagacggc actgaatacc aaaatccgaa cataatctgc catcgaggcg gagtcaacgc 300

agctttgagt gccactgtcg ccgctggctc cagcattgat attcaatgga ccacctggcc 360

agactcgcac catggtcctc tgatcacgta cctcgcctcg tgcaacggtg actgcagcac 420

agtcgacaaa accacactga aattcttcaa gatcgaagaa gatggcctca tccatggcgc 480

aactcctccc ggtacgtggg ccaccgatga tcttattgct tctgggggcc agtggactgt 540

gaggattcct tccaccatcg ctacgggtaa ttatgtcatg cgccatgaga ttatcgctct 600

gcactctgct ggccagttga atggcgctca gaactacccg cagtgtttca atttgcaggt 660

cactggtggt ggaggtaatg cgcctaatgg cacgcttggt gagaagttgt atactaatac 720

tgatcctggg atcttgttca atatttactc tacgttgact tcttatgtga tcccgggacc 780

ggctttgtat gcctga 796

<210> 19
<211> 1119
<212> DNA
<213> Aspergillus flavus

<220>
<221> sig_peptide
<222> (1)..(60)

<400> 19
atgtctatcg ccaagattgc tggtatcgtc ctcggctcgg ccgctttggt cgccgctcac 60

ggtcacgtct ctggtgccgt cgttgacgga cagtactacc ctggatatga catatcctac 120

cactacatgc ccgatccccc taaggtcatt ggatggtcga ccgatgctac cgacaatgga 180

ttcgttgacg gtagctcata tgccgatgcc gacattatct gccacaagaa cgccaagaac 240

ggcgctatct ctgctgagat cgctgctggc aagcaggtcg agctgcagtg gactgcctgg 300

cccgagagtc acaagggtcc tgtcatcact tacctcgcca actgcaacgg cgactgcgcc 360

accgtcgaca agacccagct tgagttcttc aagatcgatg agaagggcct catcagcggc 420

agtgacaaca cttgggccac cgacaacctg atcgccagca acaacagctg gaccgtgacc 480

atccccagca gcattgctgc cggcaactat gtcatgcgtc acgaaatcat tggccttcac 540

tccgctggcc agaaggatgg tgctcagaac tacccccagt gtctcaactt caaggtcacc 600

ggcggtggta gcgacaagcc cgagggtacc ctcggtactg ctctctacaa ggacaccgac 660

cctggtatcc agatcaacat ctaccagact ctgacctctt acaccatccc cggccctgct 720

ctctactctg gcagctcttc cagcggctcc tccggctccg gcagctccag cgccgctccc 780

tccgctaccg ccagtgcttc tgcttctgct tctgccactg ctgcccctgt tcagactagc 840

accgctactg cctaccagac ctcaactgct gttgccagcg tcactgttac tggctctgct 900

cccgctcaga cccacgtcca ggccacgagc tcttcggctg ctgcttccac tcccaccgct 960

agctctggcg ccagcactgg ctctggctct ggctccagct ccagcgacct gaccggctac 1020

ttcaactcct tgagcgctga cgagctcctg aacgttatca agcagactct gtcttggctt 1080

gtcaccgaca agatccacgc ccgtgacatc tctgcctaa 1119

<210> 20
<211> 798
<212> DNA
<213> Penicillium crysogenum

<220>
<221>  sig_peptide
<222>  (1)..(54)

<400>  20
atgacacttt caaagctatc tacgctccta gcctctgcca ctcttgttgc aggccatggc      60

tatgtctcca gcatcgtagc cgatggcaaa aggtgagtca atctaagacc aaagcctcta     120

tgcaaacaac gaactaatca catcatcatc agctacactg gctacctcgt tgacaaatat     180

gcctacatgg cagaccctcc tgacagcgtc ggctggacta caacagcaac cgacctcggt     240

ttcgaggacg gcaccgaata ccaagatccc aacataatct gccaccgaga cggagccaac     300

gcagctttga gtgccactgt caaagctggc tccagcattg acctccaatg gacgacctgg     360

ccagactctc accacggccc tgtgattacc tatctcgcct cgtgcaacgg tgactgcacc     420

acagtcgaca agaccacgct ggaattcttc aagattgaag aagaaggtct tatcgatggc     480

gcaaaccctc ctggcacatg ggctaccgat gacctgattg ctgctggggg taagtggact     540

gtcaagatcc cttccactat cgctccgggt aactatgtca tgcgtcacga gattattgct     600

cttcactctg ctggccagac caatggcgct cagaactacc cgcagtgctt caacctggag     660

gtcactggtg gtggaagtga cacgccagat ggcacacctg gtatggagtt gtataccaac     720

actgatcctg ggatcttgtt cgatatttat tcttcgctga cttcgtatga tattccggga     780

ccggctttgt atgcctga                                                   798


<210>  21
<211>  796
<212>  DNA
<213>  Penicillium expansum


<220>
<221>  sig_peptide
<222>  (1)..(54)

<400>  21
atgacatttt caaagctatc tttgctccta gcctccgcct cttttgtggc aggtcatggc      60

tacgtctcca gcatcgtggc aaatggcaaa aagtaagcta aaacaattcc aagtgtctct     120

ataccaataa tcaactaaca acactatcag ttacactggc tacatcgttg acaaatacgc     180

ctacatgtca gatccccccg acagcgtcgg ctgggcaaca acggcaaccg acctcggttt     240

tgaagacggc accgaatacc aagatccgaa cataatctgc caccgcggcg gaattaacgc     300

agctttgagt gcccctgtca ccgctggctc cagcattgat atccaatgga cgacctggcc     360

agactcgcac catggtcctc tggtgacata ccttgcctcg tgcaacggtg actgcagcac     420

agtcgacaag accacgctga aattcttcaa gattgaagaa gacggtctca tcgatggtac     480

aactcctccc ggtacatggg ccactgatga tcttattgct gctgggggca agtggactgt     540

gagaattcct tctaccatcg ctgcgggtaa ctatgtcatg cgccatgaaa ttatcgctct 600

gcattctgct ggccagtcca atggcgccca gaactacccg cagtgtttca atttgcaggt 660

cactggtggt ggaagtgatg tacctgatgg cacgcttggt gagaagttgt ataccaacac 720

tgacccgggg atcttgttca atctatattc cacgttgact tcgtatatta tcccaggacc 780

ggctttatat gcttga 796

<210> 22
<211> 1347
<212> DNA
<213> Penicillium rubens

<220>
<221> sig_peptide
<222> (1)..(57)

<400> 22
atgtctgtct caaagatcgc gggtctcatg cttagctcgg ccgccatggt cgctggccac 60

ggtttcgtct caggtgctgt tgttgatggc acctaccacg gaggttacct cgtcaacaac 120

tacccctaca atgacaacca ccccgagact atcggatggg ccgagaaggc taccgatctc 180

ggcttcgtcg atggcagcgg ctactctggc cctgacatta tttgccacaa ggaggccact 240

cctggtgcca tctccgccga ggtcaaggcc ggtggtgatg tcgagctgca gtggaccgag 300

tggcccgaga gccaccacgg ccccgtcatc aactatctgg ctaactgcaa cggcgactgc 360

tccaaggttg acaagaagac cctcaagtgg ttcaagatcg ctgagtccgg tctgatcgac 420

ggcagcaacg cccccggtaa atgggcatct gacgagctca ttgccaacaa caacagcgcc 480

tccgtgacca tccccagctc catcgccgct ggcaactacg ttctccgtca cgagatcatc 540

gccctgcact ccgctggcca ggagaacggt gcccagaact acccccagtg tctcaacctg 600

aaggtcaccg gcggcggcag cgacgtcccc gagggtgtcg tcggaactga gctctacaag 660

cccgacgacg ccggcatttt ggtcagcatc tacaaccagc tgaaggacta caccatcccc 720

ggtcccgctc tgtacaaggg cgcctcctcc ggctccggct ccaagaccac cgacgctgcc 780

tccaccgcaa ctaccgccag tgcctcaacc gtgagtgcct ctcccatcca gagctcctct 840

tcgcaccacc tcacccgcac ccgcactgcc cgtcccactt acagcccctc cggcacccct 900

actccctcat ccagtgccgt gtctgctgcc tcaacctcca cccctattgg gtctggtgct 960

gagtccaccg acgctccttc ggtcgtcact gagactgcca ctgccactgc ttcttctgct 1020

cctcaggtta ctgacgagcc tgctacacag accgccagcg accccgccga cagcgtttcc 1080

atcaccacca gcgccggtgc ccagtctgcc cagagctccg tccccgcctc cggtggtgag 1140

gcctccgagc ctagcgctac ccctgccccc agctcctccg gctctagctc cggctccagc 1200

tccagctccg gatcttcctc cgagtccggc gactacgcct cctacctcag ctccctgagc 1260

gccgagaagc tcctcgaagt catccgctcc accctcaagt ggcttgtctc tgacaacaag     1320

gtgcacgccc gtgccctggc tcactaa                                         1347

<210>  23
<211>  364
<212>  PRT
<213>  Byssochlamys spectabilis

<220>
<221>  SIGNAL
<222>  (1)..(21)

<400>  23

Met Pro Phe Ser Lys Ile Ala Ile Leu Ala Thr Ile Leu Gly Thr Ala
1               5                   10                  15

Ser Leu Val Ser Gly His Gly Tyr Val Gln Gly Ile Val Ala Asp Gly
            20                  25                  30

Lys Tyr Tyr Gly Gly Tyr Ile Val Thr Gln Tyr Pro Tyr Thr Asn Asp
        35                  40                  45

Pro Pro Glu Leu Val Ala Trp Ser Thr Glu Ala Thr Asp Leu Gly Phe
    50                  55                  60

Val Asp Gly Ser Gly Tyr Ala Ser Gly Asp Ile Ile Cys His Lys Ser
65                  70                  75                  80

Ala Gln Pro Ala Ala Leu Ala Ala Glu Val Pro Ala Gly Gly Lys Val
                85                  90                  95

Glu Leu Gln Trp Thr Glu Trp Pro Glu Ser His His Gly Pro Val Leu
            100                 105                 110

Asp Tyr Leu Ala Pro Cys Asn Gly Asn Cys Ala Asp Val Asp Lys Thr
        115                 120                 125

Ala Leu Glu Phe Phe Lys Ile Asp Gln Gly Gly Leu Val Asp Asp Ser
    130                 135                 140

Ser Pro Pro Gly Thr Trp Ala Ser Asp Gln Leu Ile Ser Asn Asn Asn
145                 150                 155                 160

Ser Trp Thr Val Thr Ile Pro Ser Thr Ile Ala Pro Gly Asn Tyr Val
                165                 170                 175

Leu Arg His Glu Ile Ile Ala Leu His Ser Ala Gly Asn Lys Asp Gly

180                 185                 190

Ala Gln Asn Tyr Pro Gln Cys Ile Asn Ile Lys Val Thr Gly Ser Gly
        195                 200                 205

Thr Asp Ser Pro Ser Gly Thr Leu Gly Thr Ser Leu Tyr Lys Asp Thr
    210                 215                 220

Asp Pro Gly Ile Leu Val Asn Ile Tyr Gln Thr Leu Ser Asp Tyr Ser
225                 230                 235                 240

Ile Pro Gly Pro Ala Leu Tyr Glu Gly Gly Ser Ser Ser Gly Thr Ser
                245                 250                 255

Ser Gly Ser Gly Thr Ser Thr Ala Thr Ser Thr Ser Val Ala Ser Ser
            260                 265                 270

Thr Ser Val Lys Pro Ser Ser Ile Ser Thr Pro Ser Ser Val Gln Val
        275                 280                 285

Ala Ile Pro Ser Ala Lys Thr Ser Ala Val Pro Ala Thr Thr Gln Ala
    290                 295                 300

Pro Thr Thr Ala Thr Pro Ala Val Thr Ile Pro Thr Glu Ser Ala Leu
305                 310                 315                 320

Thr Asp Tyr Leu Asn Ser Met Ser Ala Asp Glu Val Leu Asn Leu Val
                325                 330                 335

Arg Gly Thr Met Ser Trp Leu Phe Thr Asn Lys Asn His Ala Arg Asp
            340                 345                 350

Leu Ser Ala Ser Ser Glu Leu Thr Asp Arg Pro Met
        355                 360

<210>  24
<211>  249
<212>  PRT
<213>  Penicillium oxalicum

<220>
<221>  SIGNAL
<222>  (1)..(21)

<400>  24

Met Val Ser Ser Lys Ala Thr Thr Leu Ser Ala Met Leu Ala Cys Val
1               5                   10                  15

Ser Met Val Ala Gly His Gly Phe Val Ser Ser Ile Met Ala Gly Gly
            20                  25                  30

Lys Asn Tyr Thr Gly Tyr Leu Ala Asn Ser Tyr Pro Tyr Met Ser Asn
        35                  40                  45

Pro Pro Gln Ser Val Gly Trp Ala Thr Thr Ala Thr Asp Leu Gly Phe
    50                  55                  60

Glu Asp Gly Thr Glu Tyr Gln Ser Ala Asn Ile Ile Cys His Arg Asp
65                  70                  75                  80

Gly Lys Asn Ala Ala Leu Ser Ala Pro Val Thr Ala Gly Ser Lys Val
                85                  90                  95

Glu Ile Gln Trp Thr Ser Trp Pro Asp Ser His His Gly Pro Val Ile
            100                 105                 110

Thr Tyr Leu Ala Ser Cys Gly Gly Asp Cys Ser Thr Val Asp Lys Thr
        115                 120                 125

Thr Leu Lys Phe Phe Lys Ile Asp Glu Ala Gly Leu Ile Asp Asp Ser
    130                 135                 140

Asn Pro Pro Gly Thr Trp Ala Thr Asp Gln Leu Ile Ala Ala Gly Asn
145                 150                 155                 160

Arg Trp Thr Val Thr Ile Pro Lys Ser Ile Ala Ser Gly Asn Tyr Val
                165                 170                 175

Met Arg His Glu Ile Ile Ala Leu His Ser Ser Gly Ser Lys Asp Gly
            180                 185                 190

Ala Gln Asn Tyr Pro Gln Cys Leu Asn Leu Gln Val Thr Gly Gly Gly
        195                 200                 205

Ser Ala Lys Pro Gln Gly Thr Leu Gly Glu Ala Leu Tyr Lys Asp Thr
    210                 215                 220

Asp Pro Gly Ile Leu Ile Asn Ile Tyr Gln Ser Leu Lys Ser Tyr Val
225                 230                 235                 240

Ile Pro Gly Pro Ala Leu Tyr Ser Ala
                245

<210>  25
<211>  343
<212>  PRT

<213>  Aspergillus clavatus

<220>
<221>  SIGNAL
<222>  (1)..(19)

<400>  25

Met Ser Val Thr Lys Ile Ala Gly Ile Leu Leu Gly Ser Ala Ala Met
1               5                   10                  15

Val Ala Gly His Gly Phe Val Thr Gly Ala Val Val Asp Gly Thr Tyr
            20                  25                  30

His Thr Gly Tyr Leu Val Asn Asn Tyr Pro Tyr Ser Ser Asn Pro Pro
        35                  40                  45

Lys Thr Ile Gly Trp Ser Glu Thr Ala Thr Asp Leu Gly Phe Val Asp
    50                  55                  60

Gly Thr Gly Tyr Ala Ser Gly Asp Ile Ile Cys His Lys Asn Ala Lys
65                  70                  75                  80

Pro Gly Ala Leu Ser Ala Asp Ile Lys Ala Gly Gly Lys Val Glu Phe
                85                  90                  95

Gln Trp Thr Gln Trp Pro Glu Ser His His Gly Pro Val Ile Thr Tyr
            100                 105                 110

Met Ala Asn Cys Asn Gly Asp Cys Ala Ser Val Asp Lys Ser Gln Leu
        115                 120                 125

Glu Phe Phe Lys Ile Glu Glu Lys Gly Leu Ile Gly Ser Gly His Trp
    130                 135                 140

Ala Ser Asp Asp Leu Ile Ala Asn Asn Asn Ser Trp Thr Val Thr Val
145                 150                 155                 160

Pro Ser Ser Leu Ala Ala Gly Asn Tyr Val Met Arg His Glu Ile Ile
                165                 170                 175

Gly Leu His Ser Ala Gly Asn Ala Asn Gly Ala Gln Asn Tyr Pro Gln
            180                 185                 190

Cys Ile Asn Leu Lys Val Thr Gly Gly Gly Ser Asp Lys Pro Val Gly
        195                 200                 205

Thr Leu Gly Thr Lys Leu Tyr Lys Ala Thr Asp Pro Gly Ile Leu Val
    210                 215                 220

Asn Ile Tyr Gly Ser Leu Thr Ser Tyr Thr Ile Pro Gly Pro Ala Leu
225                 230                 235                 240

Trp Thr Gly Ala Ser Ser Gly Gly Ser Asp Asp Asn Gly Ser Thr Pro
                245                 250                 255

Ala Ser Thr Pro Thr Pro Thr Ala Ala Pro Ser Ser Thr Ala Ala Pro
            260                 265                 270

Ser Val Thr Ile Thr Ser Thr Pro Gly Gln Lys Thr Gln Thr Pro Val
        275                 280                 285

Ala Thr Pro Ala Pro Ser Thr Pro Ser Ser Thr Pro Gly Leu Pro Thr
    290                 295                 300

Asp Gly Asn Leu Thr Asp Tyr Phe Lys Ser Leu Ser Pro Glu Gln Phe
305                 310                 315                 320

Leu Ser Val Leu Lys Glu Thr Phe Ser Trp Leu Val Thr Glu Lys Val
                325                 330                 335

His Ala Arg Ser Leu Ser Ala
            340

<210>  26
<211>  373
<212>  PRT
<213>  Aspergillus fumigatus

<220>
<221>  SIGNAL
<222>  (1)..(19)

<400>  26

Met Ser Val Pro Lys Ile Ala Ala Ala Leu Leu Ser Ser Ala Ala Leu
1               5                   10                  15

Val Ala Gly His Gly Phe Val Thr Gly Ala Val Val Asp Gly Lys Tyr
            20                  25                  30

Tyr Thr Gly Tyr Leu Val Asn Gln Tyr Pro Tyr Met Ser Ser Pro Pro
        35                  40                  45

Asp Ser Ile Gly Trp Ser Glu Thr Ala Thr Asp Leu Gly Phe Val Asp
    50                  55                  60

Gly Ser Gly Tyr Ser Ser Gly Asp Ile Ile Cys His Lys Asp Ala Lys

65                  70                  75                  80

Asn Gly Ala Ile Ser Ala Glu Ile Lys Ala Gly Gly Lys Val Glu Phe
                85                  90                  95

Gln Trp Thr Glu Trp Pro Glu Ser His His Gly Pro Val Ile Thr Tyr
            100                 105                 110

Met Ala Asn Cys Asn Gly Asp Cys Ala Ser Val Asp Lys Thr Thr Leu
        115                 120                 125

Glu Phe Phe Lys Ile Asp Glu Ser Gly Leu Ile Ser Asp Ser Asn Val
    130                 135                 140

Pro Gly Thr Trp Ala Ser Asp Asn Leu Ile Ala Asn Asn Asn Ser Trp
145                 150                 155                 160

Thr Val Thr Val Pro Ser Ser Ile Ala Ala Gly Asn Tyr Val Met Arg
                165                 170                 175

His Glu Ile Ile Ala Leu His Ser Ala Gly Asn Gln Asn Gly Ala Gln
            180                 185                 190

Asn Tyr Pro Gln Cys Ile Asn Leu Lys Val Thr Gly Gly Gly Ser Asp
        195                 200                 205

Lys Pro Ala Gly Thr Leu Gly Thr Ala Leu Tyr Lys Asn Thr Asp Ala
    210                 215                 220

Gly Ile Leu Val Asn Ile Tyr Gln Ser Leu Ser Ser Tyr Glu Ile Pro
225                 230                 235                 240

Gly Pro Ala Leu Tyr Ser Gly Ala Ser Ser Gly Ser Ser Asn Asn Gly
                245                 250                 255

Gly Ser Ala Ser Ser Ser Ala Thr Ala Pro Ser Ala Thr Ile Thr Gln
            260                 265                 270

Pro Ser Thr Ala Val Pro Thr Ser Ser Ala Thr Ala Tyr Gln Pro Ser
        275                 280                 285

Thr Thr Thr Glu Ala Val Thr Val Thr Ser Ile Pro Ala Gln Gln Ser
    290                 295                 300

Tyr Val Gln Ala Pro Thr Ala Thr Pro Ser Ser Thr Ala Gly Ser Ser
305                 310                 315                 320

Gly Ser Gly Ser Gly Ser Ser Ser Ser Gly Thr Leu Pro Ser Ser Ser
                325                 330                 335

Asn Leu Thr Glu Tyr Phe Asn Ser Leu Ser Ala Glu Glu Phe Leu Lys
            340                 345                 350

Val Leu Lys Gln Thr Phe Ser Trp Leu Val Thr Glu Lys Val His Ala
        355                 360                 365

Arg Asp Leu Ser Ala
    370

<210>  27
<211>  402
<212>  PRT
<213>  Aspergillus niger

<220>
<221>  SIGNAL
<222>  (1)..(19)

<400>  27

Met Ser Val Ala Lys Ile Ala Gly Val Val Leu Gly Ser Ala Ala Leu
1               5                   10                  15

Val Ala Gly His Gly Tyr Val Ser Gly Ala Val Ile Asp Gly Glu Tyr
            20                  25                  30

Tyr Gly Gly Tyr Ile Val Ser Ser Tyr Pro Tyr Glu Ser Asp Pro Pro
        35                  40                  45

Glu Thr Ile Ala Trp Ser Thr Thr Ala Thr Asp Leu Gly Phe Val Asp
    50                  55                  60

Gly Ser Glu Tyr Ser Asp Pro Asp Ile Ile Cys His Lys Ser Ala Lys
65                  70                  75                  80

Pro Gly Ala Ile Ser Ala Asp Val Lys Ala Gly Gly Thr Val Glu Leu
                85                  90                  95

Gln Trp Thr Asp Trp Pro Ser Ser His His Gly Pro Val Leu Thr Tyr
            100                 105                 110

Leu Ala Asn Cys Asn Gly Asp Cys Ser Asp Val Thr Lys Thr Asp Leu
        115                 120                 125

Glu Phe Phe Lys Ile Asp Glu Ser Gly Leu Ile Ser Asp Thr Glu Val
    130                 135                 140

Pro Gly Thr Trp Ala Thr Asp Asn Leu Ile Ser Asn Asn Asn Ser Trp
145                 150                 155                 160

Thr Val Thr Ile Pro Ser Thr Leu Glu Ala Gly Asn Tyr Val Leu Arg
                165                 170                 175

His Glu Ile Ile Ala Leu His Ser Ala Glu Asn Lys Asp Gly Ala Gln
            180                 185                 190

Asn Tyr Pro Gln Cys Leu Asn Leu Lys Val Thr Gly Ser Gly Ser Ser
        195                 200                 205

Thr Tyr Ser Gly Thr Lys Gly Glu Ala Leu Tyr Lys Asp Thr Asp Pro
    210                 215                 220

Gly Ile Leu Val Asn Ile Tyr Glu Thr Leu Ser Ser Tyr Asp Ile Pro
225                 230                 235                 240

Gly Pro Ala Met Tyr Asn Ala Thr Ser Ser Ser Ser Ser Ser Gly Ser
                245                 250                 255

Ser Ser Gly Ala Ser Ser Thr Ser Ser Ala Ala Ala Ala Ala Ala Thr
            260                 265                 270

Thr Ser Ser Ala Ser Thr Ser Ser Ala Ser Thr Pro Thr Thr Ser Ala
        275                 280                 285

Ala Ala Ala Thr Ser Ser Ala Val Ser Ala Thr Thr Thr Ala Ala Ala
    290                 295                 300

Ala Val Val Thr Asp Val Glu Thr Thr Val Gln Thr Ala Val Ala Thr
305                 310                 315                 320

Ala Tyr Gln Thr Ser Thr Asp Val Val Gln Val Thr Val Thr Gly Ser
                325                 330                 335

Ala Pro Gln Gln Thr His Ile Gln Val Ala Ser Ser Ser Ala Ala Ala
            340                 345                 350

Ser Thr Pro Ser Ser Ser Ser Val Ala Val Ser Ser Asp Asn Leu Thr
        355                 360                 365

Ser Tyr Phe Ser Ser Leu Ser Ala Asp Gln Phe Leu Ser Val Leu Lys
    370                 375                 380

Glu Thr Phe Ser Trp Leu Val Thr Glu Lys Lys His Ala Arg Asp Leu
385                 390                 395                 400

Asn Ala

<210> 28
<211> 353
<212> PRT
<213> Aspergillus niger

<220>
<221> SIGNAL
<222> (1)..(19)

<400> 28

Met Ser Val Ala Lys Ile Ala Gly Val Val Leu Gly Ser Ala Ala Leu
1               5                   10                  15

Val Ala Gly His Gly Tyr Val Ser Gly Ala Val Ile Asp Gly Glu Tyr
            20                  25                  30

Tyr Gly Gly Tyr Ile Val Ser Ser Tyr Ala Tyr Glu Ser Asp Pro Pro
        35                  40                  45

Glu Thr Ile Ala Trp Ser Thr Thr Ala Thr Asp Leu Gly Phe Val Asp
    50                  55                  60

Gly Ser Glu Tyr Ser Asp Pro Asp Ile Ile Cys His Lys Ser Ala Lys
65                  70                  75                  80

Pro Gly Ala Ile Ser Ala Asp Val Lys Ala Gly Gly Thr Val Glu Leu
                85                  90                  95

Gln Trp Thr Asp Trp Pro Ser Ser His His Gly Pro Val Leu Thr Tyr
            100                 105                 110

Leu Ala Asn Cys Asn Gly Asp Cys Ser Asp Val Thr Lys Thr Asp Leu
        115                 120                 125

Glu Phe Phe Lys Ile Asp Glu Ser Gly Leu Ile Ser Asp Thr Glu Val
    130                 135                 140

Pro Gly Thr Trp Ala Thr Asp Asn Leu Ile Ser Asn Asn Asn Ser Trp
145                 150                 155                 160

Thr Val Thr Ile Pro Ser Thr Leu Glu Ala Gly Asn Tyr Val Leu Arg
                165                 170                 175

His Glu Ile Ile Ala Leu His Ser Ala Glu Asn Lys Asp Gly Ala Gln

180                 185                 190

Asn Tyr Pro Gln Cys Leu Asn Leu Lys Val Thr Gly Ser Gly Ser Ser
        195                 200                 205

Thr Tyr Ser Gly Thr Lys Gly Glu Ala Leu Tyr Lys Asp Thr Asp Pro
    210                 215                 220

Gly Ile Leu Val Asn Ile Tyr Glu Thr Leu Ser Ser Tyr Asp Ile Pro
225                 230                 235                 240

Gly Pro Ala Ile Ser Ala Val Ser Ala Thr Thr Thr Ala Ala Ala Ala
                245                 250                 255

Val Val Thr Asp Val Glu Thr Thr Val Gln Thr Ala Val Ala Thr Ala
            260                 265                 270

Tyr Gln Thr Ser Thr Asp Val Val Gln Val Thr Val Thr Gly Ser Ala
        275                 280                 285

Pro Gln Gln Thr His Ile Gln Val Ala Ser Ser Ser Ala Ala Ala Ser
    290                 295                 300

Thr Pro Ser Ser Ser Ser Val Ala Val Ser Ser Asp Asn Leu Thr Ser
305                 310                 315                 320

Tyr Phe Ser Ser Leu Ser Ala Asp Gln Phe Leu Ser Val Leu Lys Glu
                325                 330                 335

Thr Phe Ser Trp Leu Val Thr Glu Lys Lys His Ala Arg Asp Leu Asn
            340                 345                 350

Ala

<210>  29
<211>  344
<212>  PRT
<213>  Aspergillus ruber

<220>
<221>  SIGNAL
<222>  (1)..(19)

<400>  29

Met Ser Val Thr Lys Ile Thr Gly Leu Leu Leu Gly Ser Ala Ala Met
1               5                   10                  15

Val Ala Gly His Gly Tyr Val Ser Gly Ala Val Val Asp Gly Gln Tyr
            20                  25                  30

Tyr Gly Gly Tyr Ile Val Thr Ser Tyr Ala Tyr Thr Asp Ser Pro Pro
        35                  40                  45

Glu Thr Ile Gly Trp Ser Thr Glu Ala Thr Asp Leu Gly Phe Val Ser
    50                  55                  60

Pro Ser Ser Phe Ser Asp Pro Asp Ile Ile Cys His Lys Ser Ala Gln
65                  70                  75                  80

Pro Gly Ala Ile Ser Ala Ser Val Ala Ala Gly Lys Asp Val Glu Leu
                85                  90                  95

Gln Trp Thr Glu Trp Pro Glu Ser His His Gly Pro Val Ile Thr Tyr
            100                 105                 110

Leu Ala Asn Cys Asn Gly Asp Cys Ser Lys Val Asp Lys Thr Ser Leu
        115                 120                 125

Glu Phe Phe Lys Ile Asp Gln Lys Gly Leu Val Asp Asp Ser Asn Val
    130                 135                 140

Pro Gly Thr Trp Ala Ser Asp Asn Leu Ile Ser Asn Asn Asn Ser Tyr
145                 150                 155                 160

Thr Val Thr Ile Pro Ser Asp Ile Ala Ala Gly Asn Tyr Val Leu Arg
                165                 170                 175

His Glu Ile Ile Ala Leu His Ser Ala Gly Asn Glu Asp Gly Ala Gln
            180                 185                 190

Ser Tyr Pro Gln Cys Val Asn Leu Lys Val Thr Gly Gly Gly Ser Ala
        195                 200                 205

Ser Pro Ser Gly Thr Leu Gly Thr Lys Leu Tyr Ser Glu Asp Asp Pro
    210                 215                 220

Gly Ile Leu Val Asn Ile Tyr Gln Gln Leu Asp Ser Tyr Glu Ile Pro
225                 230                 235                 240

Gly Pro Ala Leu Tyr Ser Gly Ala Ser Ser Ser Asn Ser Gly Ser
                245                 250                 255

Ser Ser Ser Thr Ala Ser Ala Ser Thr Thr Ala Thr Ser Ala Thr Thr
                260                 265                 270

Ser Ser Ala Ser Ser Thr Gln Ala Ser Ala Thr Pro Ala Ser Gln Ala
        275                 280                 285

Lys Ala Gln Val Ser Ser Ser Thr Pro Ser Ala Ser Ser Val Ala Thr
    290                 295                 300

Ser Gly Ser Leu Ser Asp Tyr Phe Ser Ser Leu Ser Ala Glu Glu Phe
305                 310                 315                 320

Leu Asn Val Ile Ser Glu Thr Leu Ser Trp Leu Val Thr Asp Lys Ile
                325                 330                 335

His Ala Arg Asp Ile Ser Thr Ala
            340

<210>  30
<211>  402
<212>  PRT
<213>  Aspergillus terreus

<220>
<221>  SIGNAL
<222>  (1)..(19)

<400>  30

Met Ser Val Ala Lys Ile Ala Gly Val Val Leu Gly Ser Ala Ala Leu
1               5                   10                  15

Val Ala Gly His Gly Tyr Val Thr Gly Ala Val Val Asp Gly Lys Tyr
            20                  25                  30

Tyr Ala Gly Tyr Asp Val Thr Ser Ala Pro Tyr Ser Ser Asp Pro Pro
        35                  40                  45

Ala Thr Ile Gly Trp Ser Thr Thr Ala Thr Asp Leu Gly Phe Val Asp
    50                  55                  60

Gly Thr Glu Tyr Ser Glu Pro Asp Ile Ile Cys His Lys Asp Ala Lys
65                  70                  75                  80

Pro Gly Ser Leu Ser Ala Glu Ile Thr Ala Gly Gly Lys Val Glu Leu
                85                  90                  95

Gln Trp Thr Glu Trp Pro Glu Ser His His Gly Pro Val Ile Thr Tyr
            100                 105                 110

Leu Ala Asn Cys Asn Gly Asp Cys Ser Ser Val Asp Lys Thr Ser Leu
        115                 120                 125

Glu Phe Phe Lys Ile Asp Gln Lys Gly Leu Ile Glu Gly Asn Thr Trp
    130                 135                 140

Ala Ser Asp Gln Leu Ile Ser Asn Asn Asn Ser Trp Thr Val Thr Ile
145                 150                 155                 160

Pro Ser Ser Ile Ala Ser Gly Asn Tyr Val Leu Arg His Glu Ile Ile
                165                 170                 175

Gly Leu His Ser Ala Gly Asn Lys Asp Gly Ala Gln Asn Tyr Pro Gln
            180                 185                 190

Cys Met Asn Leu Lys Val Thr Gly Gly Gly Ser Asp Lys Pro Ala Gly
        195                 200                 205

Thr Leu Gly Thr Ala Leu Tyr Lys Asp Thr Asp Pro Gly Ile Leu Val
    210                 215                 220

Asn Ile Tyr Gln Thr Leu Ser Ser Tyr Val Ile Pro Gly Pro Ala Leu
225                 230                 235                 240

Tyr Ser Gly Ser Ser Ser Gly Ser Gly Ser Gly Ser Ala Ser Ser Ser
                245                 250                 255

Ala Ala Ala Thr Pro Ser Ser Ser Ser Val Pro Ser Ser Thr Ser Ile
            260                 265                 270

Ala Val Pro Thr Ser Ser Ala Pro Ala Pro Thr Ser Ala Ala Pro Ser
        275                 280                 285

Ala Gln Thr Ser Thr Ala Thr Ala Tyr Gln Thr Ala Thr Thr Ile Glu
    290                 295                 300

Ser Val Thr Val Thr Ala Glu Ala Pro Gln Gln Thr Lys Val Gln Asp
305                 310                 315                 320

Pro Ser Ser Gly Ser Ser Ser Gly Ser Asn Ser Gly Ser Ser Ser Gly
                325                 330                 335

Ser Ser Ser Gly Ser Ser Thr Gly Ser Ser Ser Gly Ser Ser Thr Gly
            340                 345                 350

Thr Gly Ser Ser Ser Val Pro Thr Pro Ser Gly Ser Ser Ser Leu Ser
        355                 360                 365

Asp Tyr Phe Asn Ser Leu Ser Ala Glu Gln Phe Leu Asn Leu Leu Arg

370                 375                 380

Gln Thr Leu Ser Trp Leu Ile Thr Asp Lys Lys His Ala Arg Asp Leu
385                 390                 395                 400

Ser Ala

<210>  31
<211>  367
<212>  PRT
<213>  Neosartorya fischeri

<220>
<221>  SIGNAL
<222>  (1)..(19)

<400>  31

Met Ser Val Ser Lys Ile Ala Ala Val Leu Leu Ser Ser Ala Ala Leu
1               5                   10                  15

Val Ala Gly His Gly Phe Val Ser Gly Ala Val Val Asp Gly Lys Tyr
            20                  25                  30

Tyr Thr Gly Tyr Leu Val Asn Gln Tyr Pro Tyr Met Ser Ser Pro Pro
        35                  40                  45

Glu Ser Ile Gly Trp Ser Glu Thr Ala Thr Asp Leu Gly Phe Val Asp
    50                  55                  60

Gly Ser Gly Tyr Ser Ser Gly Asp Ile Ile Cys His Lys Ser Ala Lys
65                  70                  75                  80

Asn Gly Ala Ile Ser Ala Glu Ile Lys Ala Gly Gly Lys Val Glu Phe
                85                  90                  95

Gln Trp Thr Glu Trp Pro Glu Ser His His Gly Pro Val Ile Thr Tyr
            100                 105                 110

Met Ala Asn Cys Asn Gly Asp Cys Ala Ser Val Asp Lys Thr Ser Leu
        115                 120                 125

Glu Phe Phe Lys Ile Asp Glu Ser Gly Leu Ile Ser Asp Ser Asn Val
    130                 135                 140

Pro Gly Thr Trp Ala Ser Asp Asn Leu Ile Ala Asn Asn Asn Ser Trp
145                 150                 155                 160

Thr Val Thr Val Pro Ser Ser Ile Ala Ala Gly Asn Tyr Val Met Arg
                165                 170                 175

His Glu Ile Ile Ala Leu His Ser Ala Gly Asn Gln Asn Gly Ala Gln
            180                 185                 190

Asn Tyr Pro Gln Cys Ile Asn Leu Lys Val Thr Gly Gly Gly Ser Asp
        195                 200                 205

Lys Pro Ala Gly Thr Leu Gly Thr Ala Leu Tyr Lys Asn Thr Asp Ala
    210                 215                 220

Gly Ile Leu Val Asn Ile Tyr Gln Ser Leu Ser Ser Tyr Asp Ile Pro
225                 230                 235                 240

Gly Pro Ala Leu Tyr Ser Gly Ala Ser Ser Gly Ser Ser Asn Ser Gly
                245                 250                 255

Gly Ser Ala Ser Ser Ser Ala Ala Ala Pro Ser Ala Thr Ile Thr Gln
            260                 265                 270

Thr Ser Thr Ala Val Gln Thr Ser Ser Ala Thr Val Tyr Gln Pro Ser
        275                 280                 285

Thr Thr Thr Glu Ala Val Thr Val Thr Ser Thr Pro Ala Gln Gln Ser
    290                 295                 300

Tyr Val Gln Ala Pro Thr Ala Thr Pro Ser Ser Thr Ala Gly Ser Ser
305                 310                 315                 320

Ser Ser Ser Gly Thr Leu Pro Ser Gly Ser Ser Leu Thr Glu Tyr Phe
                325                 330                 335

Asn Ser Leu Ser Ala Glu Glu Phe Leu Lys Val Leu Lys Glu Thr Phe
            340                 345                 350

Ser Trp Leu Val Thr Glu Lys Val His Ala Arg Asp Leu Ser Ala
        355                 360                 365

<210>  32
<211>  400
<212>  PRT
<213>  Aspergillus kawachii

<220>
<221>  SIGNAL
<222>  (1)..(19)

<400>  32

Met Ser Val Ala Lys Ile Ala Gly Val Val Leu Gly Ser Ala Ala Leu
1               5                   10                  15

Val Ala Gly His Gly Tyr Val Ser Gly Ala Val Val Asp Gly Gln Tyr
            20                  25                  30

Tyr Gly Gly Tyr Ile Val Ser Ser Tyr Ala Tyr Glu Ser Asp Pro Pro
        35                  40                  45

Glu Thr Ile Ala Trp Ser Thr Glu Ala Thr Asp Leu Gly Phe Val Asp
    50                  55                  60

Gly Ser Glu Tyr Ala Glu Pro Asp Ile Ile Cys His Lys Ser Ala Lys
65                  70                  75                  80

Pro Gly Ala Ile Ser Ala Asp Val Lys Ala Gly Gly Thr Val Glu Leu
                85                  90                  95

Gln Trp Thr Asp Trp Pro Ser Ser His His Gly Pro Val Leu Thr Tyr
            100                 105                 110

Leu Ala Asn Cys Asn Gly Asp Cys Ser Asp Val Thr Lys Thr Asp Leu
        115                 120                 125

Glu Phe Phe Lys Ile Asp Glu Ser Gly Leu Ile Ser Asp Thr Glu Val
    130                 135                 140

Pro Gly Thr Trp Ala Thr Asp Asn Leu Ile Ser Asn Asn Asn Ser Trp
145                 150                 155                 160

Thr Val Thr Ile Pro Ser Thr Leu Glu Ser Gly Asn Tyr Val Leu Arg
                165                 170                 175

His Glu Ile Ile Ala Leu His Ser Ala Gly Asn Lys Asp Gly Ala Gln
            180                 185                 190

Asn Tyr Pro Gln Cys Leu Asn Leu Lys Val Thr Gly Ser Gly Ser Ser
        195                 200                 205

Thr Tyr Ser Gly Thr Lys Gly Glu Ala Leu Tyr Lys Asp Thr Asp Pro
    210                 215                 220

Gly Ile Leu Val Asn Ile Tyr Gln Ser Leu Ser Ser Tyr Asp Ile Pro
225                 230                 235                 240

Gly Pro Ala Met Tyr Asn Ala Thr Ser Ser Gly Ser Ser Ser Gly Ser
                245                 250                 255

Ser Ser Ser Ala Ser Ser Thr Ser Ser Ala Ala Ala Ala Ala Ala Thr
            260                 265                 270

Thr Ser Ser Ala Ser Ser Ser Thr Thr Ser Ala Ala Ala Ala Thr Ser
        275                 280                 285

Ser Ala Val Ser Ala Thr Thr Thr Ser Ala Ala Ala Ala Ala Val Val
    290                 295                 300

Thr Asp Val Glu Thr Thr Val Gln Thr Ala Val Ala Thr Ala Tyr Gln
305                 310                 315                 320

Thr Ser Thr Asp Val Val Gln Val Thr Val Thr Gly Ser Ala Pro Gln
                325                 330                 335

Gln Thr His Ile Gln Val Ala Ser Ser Ser Ala Ala Ala Val Ala Thr
            340                 345                 350

Pro Ser Ser Ser Ser Val Ala Val Ser Ser Asp Asn Leu Thr Ser Tyr
        355                 360                 365

Phe Ser Ser Leu Ser Ala Asp Gln Phe Leu Ser Val Leu Lys Glu Thr
    370                 375                 380

Phe Ser Trp Leu Val Thr Glu Lys Lys His Ala Arg Asp Leu Asn Ala
385                 390                 395                 400

<210>  33
<211>  434
<212>  PRT
<213>  Aspergillus nidulans

<220>
<221>  SIGNAL
<222>  (1)..(19)

<400>  33

Met Ser Val Ala Arg Thr Ala Gly Phe Ala Leu Ala Ser Ala Ala Ile
1               5                   10                  15

Val Ala Gly His Gly Tyr Val Thr Gly Ile Val Ala Asp Gly Thr Tyr
            20                  25                  30

Tyr Gly Gly Tyr Leu Val Asn Gln Tyr Pro Tyr Ser Asn Asp Pro Pro
        35                  40                  45

Ala Val Val Gly Trp Ala Glu Asp Ala Thr Asp Leu Gly Phe Val Asp

50                  55                  60

Gly Ser Gly Tyr Thr Ser Gly Asp Ile Ile Cys His Lys Asp Ala Thr
65                  70                  75                  80

Asn Ala Gln Ala Ser Ala Thr Val Ala Ala Gly Gly Thr Val Glu Leu
                85                  90                  95

Gln Trp Thr Glu Trp Pro Glu Ser His His Gly Pro Val Ile Asp Tyr
            100                 105                 110

Ile Ala Ser Cys Asn Gly Asp Cys Thr Thr Val Asp Lys Thr Thr Leu
        115                 120                 125

Glu Trp Val Lys Ile Ser Glu Ser Gly Leu Val Asp Gly Ser Ser Ala
    130                 135                 140

Pro Gly Thr Trp Ala Ser Asp Asn Leu Ile Ser Asn Asn Asn Ser Trp
145                 150                 155                 160

Thr Val Thr Ile Pro Ser Ser Leu Ala Ala Gly Gly Tyr Val Leu Arg
                165                 170                 175

His Glu Ile Ile Ala Leu His Ser Ala Gly Asn Glu Asn Gly Ala Gln
            180                 185                 190

Asn Tyr Pro Gln Cys Val Asn Leu Glu Val Thr Gly Gly Gly Ser Ala
        195                 200                 205

Ser Pro Ser Gly Thr Val Gly Thr Glu Leu Tyr Thr Pro Thr Asp Pro
    210                 215                 220

Gly Ile Leu Val Asn Ile Tyr Thr Ser Leu Asp Ser Tyr Thr Ile Pro
225                 230                 235                 240

Gly Pro Ala Leu Trp Asp Gly Ala Ser Ser Ser Gly Gly Asn Ser Gly
                245                 250                 255

Ser Gly Ser Ala Ser Ser Ser Ala Ala Ala Thr Ser Thr Pro Thr Thr
            260                 265                 270

Pro Ser Val Ser Val Pro Val Ile Pro Thr Ala Ser Ser Gly Ala Ser
        275                 280                 285

Ser Thr Pro Leu Val Pro Thr Pro Ser Ala Pro Ala Val Thr Pro Ser
    290                 295                 300

Val Pro Ala Gly Asn Gln Ala Pro Gln Pro Thr Tyr Thr Ser Thr Tyr
305                 310                 315                 320

Ile Glu Thr Glu Thr Leu Pro Gly Gln Thr Val Thr Ser Thr Thr Thr
                325                 330                 335

Glu Tyr Ala Ser Glu Pro Thr Gln Pro Ala Val Glu Thr Gln Val Ala
            340                 345                 350

Gln Pro Ser Glu Thr Glu Ala Ala Thr Ser Thr Ser Thr Val Thr Glu
        355                 360                 365

Thr Ala Ser Ala Thr Ala Ala Pro Thr Gly Ser Ser Gly Ser Ser Ser
    370                 375                 380

Gly Ser Gly Ser Ser Ser Thr Glu Leu Pro Thr Asp Ser Ser Ser Leu
385                 390                 395                 400

Ser Asp Tyr Phe Ser Ser Leu Ser Ala Glu Glu Phe Leu Asn Leu Leu
                405                 410                 415

Lys Glu Thr Leu Lys Trp Leu Val Thr Asp Lys Val His Ala Arg Ser
            420                 425                 430

Leu His

<210>  34
<211>  250
<212>  PRT
<213>  Aspergillus oryzae

<220>
<221>  SIGNAL
<222>  (1)..(21)

<400>  34

Met Ala Met Ser Lys Ile Val Ser Leu Thr Gly Leu Leu Ala Ser Ala
1               5                   10                  15

Ser Leu Val Ala Gly His Gly Tyr Val Ser Gly Val Val Ile Asp Gly
            20                  25                  30

Gln Tyr Tyr Gly Gly Tyr Leu Val Asp Lys Tyr Ala Tyr Ser Asp Asn
        35                  40                  45

Ala Pro Asp Thr Ile Gly Trp Thr Thr Ser Ala Thr Asp Leu Gly Phe
    50                  55                  60

Val Asp Gly Thr Gly Tyr Gln Ser Pro Asp Ile Ile Cys His Lys Asp
65                  70                  75                  80

Gly Ala Pro Gly Ala Leu Thr Ala Glu Val Ala Ala Gly Gly Lys Ile
                85                  90                  95

Glu Leu Gln Trp Thr Glu Trp Pro Glu Ser His His Gly Pro Val Leu
            100                 105                 110

Asn Tyr Leu Ala Pro Cys Gly Gly Glu Cys Ser Ala Val Asp Lys Thr
        115                 120                 125

Thr Leu Glu Phe Phe Lys Ile Glu Ala Lys Gly Leu Ile Asp Gly Thr
    130                 135                 140

Thr Pro Pro Gly Gln Trp Ala Thr Asp Asp Leu Ile Ser Asn Asn Asn
145                 150                 155                 160

Ser Tyr Thr Val Thr Ile Pro Thr Ser Ile Gln Glu Gly Asn Tyr Val
                165                 170                 175

Leu Arg His Glu Ile Ile Gly Leu His Ser Ala Gly Gln Lys Asp Gly
            180                 185                 190

Ala Gln Asn Tyr Pro Gln Cys Ile Asn Ile Lys Val Thr Gly Gly Gly
        195                 200                 205

Asp Ala Thr Pro Ala Gly Thr Ala Gly Glu Ala Leu Tyr Lys Asp Thr
    210                 215                 220

Asp Ala Gly Ile Leu Phe Asp Ile Tyr Ser Asp Leu Ser Gly Gly Tyr
225                 230                 235                 240

Pro Ile Pro Gly Pro Glu Val Phe Ser Ala
                245                 250

<210>  35
<211>  370
<212>  PRT
<213>  Aspergillus oryzae

<220>
<221>  SIGNAL
<222>  (1)..(19)

<400>  35

Met Ser Ile Ala Lys Ile Ala Gly Val Val Leu Gly Ser Ala Ala Leu
1               5                   10                  15

Val Ala Gly His Gly Tyr Val Ser Gly Ala Val Val Asp Gly Gln Tyr
            20                  25                  30

Tyr Ser Gly Tyr Asp Met Ser Tyr His Tyr Met Ser Asn Pro Pro Gln
        35                  40                  45

Val Ile Gly Trp Ser Thr Asp Ala Thr Asp Leu Gly Phe Val Asp Gly
    50                  55                  60

Ser Ser Tyr Ala Asp Ala Asp Ile Ile Cys His Lys Asn Ala Lys Asn
65                  70                  75                  80

Gly Ala Ile Ser Ala Glu Ile Ala Ala Gly Lys Gln Val Glu Leu Gln
                85                  90                  95

Trp Thr Ala Trp Pro Glu Ser His Lys Gly Pro Val Ile Thr Tyr Leu
            100                 105                 110

Ala Asn Cys Asn Gly Asp Cys Ala Thr Val Asp Lys Thr Gln Leu Glu
        115                 120                 125

Phe Phe Lys Ile Asp Glu Lys Gly Leu Ile Ser Gly Ser Asp Asn Thr
    130                 135                 140

Trp Ala Ser Asp Asn Leu Ile Ser Ser Asn Asn Ser Trp Thr Val Thr
145                 150                 155                 160

Ile Pro Ser Ser Ile Ala Ala Gly Asn Tyr Val Met Arg His Glu Ile
                165                 170                 175

Ile Ala Leu His Ser Ala Gly Asn Lys Asp Gly Ala Gln Asn Tyr Pro
            180                 185                 190

Gln Cys Leu Asn Phe Lys Val Thr Gly Gly Gly Ser Asp Lys Pro Glu
        195                 200                 205

Gly Thr Leu Gly Thr Ala Leu Tyr Lys Asp Thr Asp Pro Gly Ile Glu
    210                 215                 220

Ile Asn Ile Tyr Gln Thr Leu Ser Ser Tyr Thr Ile Pro Gly Pro Ala
225                 230                 235                 240

Leu Tyr Thr Gly Ser Ser Ser Ser Gly Ser Ser Gly Ser Gly Ser Ser
                245                 250                 255

Ser Ala Ala Pro Ser Pro Thr Ala Ser Ala Ser Ala Ser Ala Thr Ala

            260                 265                 270

Ala Pro Val Gln Thr Ser Thr Ala Thr Ala Tyr Gln Thr Ser Thr Ala
        275                 280                 285

Val Ala Ser Val Thr Val Thr Gly Ser Ala Pro Ala Gln Thr His Val
    290                 295                 300

Gln Ala Thr Ser Ser Ser Ala Ala Ala Ser Thr Pro Thr Ala Ser Ser
305                 310                 315                 320

Gly Ala Ser Thr Gly Ser Gly Ser Gly Ser Ser Ser Ser Asp Leu Thr
                325                 330                 335

Ser Tyr Phe Asn Ser Leu Ser Ala Asp Glu Leu Leu Asn Val Ile Lys
            340                 345                 350

Gln Thr Leu Ser Trp Leu Val Thr Asp Lys Ile His Ala Arg Asp Ile
        355                 360                 365

Ser Ala
    370

<210> 36
<211> 378
<212> PRT
<213> Baudoinia compniacensis

<220>
<221> SIGNAL
<222> (1)..(19)

<400> 36

Met Ser Val Ile Gln Ser Ala Ala Ile Leu Ala Ser Leu Val Ala Ser
1               5                   10                  15

Val Ala Ala His Gly His Val Gln Gly Ile Val Ala Gly Gly Thr Tyr
            20                  25                  30

Tyr Thr Gly Tyr Asp Pro Ser Phe Gln Tyr Ser Pro Ile Pro Pro Ile
        35                  40                  45

Val Ile Gly Trp Ser Asp Pro Gln Asp Leu Asp Asn Gly Phe Ile Ala
    50                  55                  60

Pro Ser Asn Tyr Thr Asn Pro Asp Ile Ile Cys His Arg Asn Ala Thr
65                  70                  75                  80

Ala Ala Gly Thr Ser Ala Arg Ile Ala Ala Gly Asp Val Val Glu Pro
                85                  90                  95

Gln Trp Thr Pro Trp Pro Ser Ser His His Gly Pro Val Ile Asp Tyr
            100                 105                 110

Leu Ala Lys Cys Pro Gly Ser Cys Ser Asp Val Asp Lys Thr Thr Leu
        115                 120                 125

Glu Phe Phe Lys Ile Asp Gln Val Gly Leu Ile Asp Asp Thr Thr Glu
    130                 135                 140

Pro Gly Thr Trp Gly Ser Asp Gln Leu Leu Ala Asn Asn Asn Ser Trp
145                 150                 155                 160

Thr Val Thr Ile Pro Pro Ser Ile Ala Pro Gly Asn Tyr Val Leu Arg
                165                 170                 175

His Glu Ile Ile Ala Leu His Ser Ala Gly Thr Ala Asp Gly Ala Gln
            180                 185                 190

Asn Tyr Pro Gln Cys Val Asn Leu Glu Ile Thr Gly Ser Gly Thr Asp
        195                 200                 205

Thr Pro Ala Gly Thr Leu Gly Gln Lys Leu Tyr Gly Ala Asn Asp Ser
    210                 215                 220

Gly Ile Leu Ile Asn Ile Tyr Gln Ser Leu Ser Thr Tyr Glu Ile Pro
225                 230                 235                 240

Gly Pro Ser Leu Tyr Ser Gly Ala Val Ala Met Ser Gln Thr Gln Val
                245                 250                 255

Ala Ile Thr Gly Tyr Ala Pro Leu Ser Ser Gly Gly Val Ala Val Ser
            260                 265                 270

Ile Ala Tyr Ser Ser Ala Thr Thr Thr Thr Ala Ala Ala Gly Ser Ser
        275                 280                 285

Ser Val Ala Arg Ser Thr Thr Ala Ala Ser Ala Ser Ala Ser Thr Ala
    290                 295                 300

Ile Val Ser Ala Leu Pro Ser Thr Ala Leu Thr Gln Thr Leu Pro Thr
305                 310                 315                 320

Val Leu Pro Thr Gly Thr Ala Val Ser Ser Asn Asp Thr Ile Ala Gly
                325                 330                 335

Leu Pro Pro Ala Glu Ala Pro Pro Ala Gly Thr Thr Leu Gln Asp Leu
            340                 345                 350

Phe Ala Trp Leu Glu Tyr Met Leu Glu Gly Leu Ala Ser Thr Gly Gly
        355                 360                 365

Arg His Gln Arg Asp Phe Lys Glu Arg Lys
    370                 375

<210>  37
<211>  245
<212>  PRT
<213>  Penicillium roqueforti

<220>
<221>  SIGNAL
<222>  (1)..(18)

<400>  37

Met Thr Leu Ser Lys Leu Ser Thr Leu Leu Ala Ser Ala Ser Leu Val
1               5                   10                  15

Ala Gly His Gly Tyr Val Ser Ser Ile Val Ala Asp Gly Lys Asn Tyr
            20                  25                  30

Thr Gly Tyr Ile Val Asp Lys Tyr Pro Tyr Met Ala Asp Pro Pro Asp
        35                  40                  45

Ser Val Gly Trp Ala Thr Thr Ala Thr Asp Leu Gly Phe Glu Asp Gly
    50                  55                  60

Thr Glu Tyr Gln Asp Pro Asn Ile Ile Cys His Arg Asn Gly Val Asn
65                  70                  75                  80

Ala Ala Leu Ser Ala Thr Val Thr Ala Gly Ser Ser Ile Asp Leu Gln
                85                  90                  95

Trp Thr Thr Trp Pro Asp Ser His His Gly Pro Leu Ile Thr Tyr Leu
            100                 105                 110

Ala Ser Cys Asn Gly Asp Cys Ser Thr Val Asp Lys Thr Thr Leu Lys
        115                 120                 125

Phe Phe Lys Ile Glu Glu Asp Gly Leu Ile Asp Gly Ala Thr Pro Pro
    130                 135                 140

Gly Thr Trp Ala Thr Asp Asp Leu Ile Ala Ala Gly Gly Gln Trp Thr
145                 150                 155                 160

Val Lys Ile Pro Pro Thr Leu Ala Ala Gly Asn Tyr Val Met Arg His
                165                 170                 175

Glu Ile Ile Ala Leu His Ser Ala Gly Gln Leu Asn Gly Ala Gln Asn
            180                 185                 190

Tyr Pro Gln Cys Phe Asn Leu Glu Ile Thr Gly Gly Gly Ser Asp Val
        195                 200                 205

Pro Asp Gly Thr Phe Gly Gly Glu Leu Tyr Ile Asn Thr Asp Pro Gly
    210                 215                 220

Ile Leu Phe Asp Ile Tyr Ser Thr Leu Thr Ser Tyr Ile Ile Pro Gly
225                 230                 235                 240

Pro Ala Leu Tyr Ala
                245

<210>  38
<211>  318
<212>  PRT
<213>  Sclerotinia sclerotiorum

<220>
<221>  SIGNAL
<222>  (1)..(18)

<400>  38

Met Ser Phe Ser Lys Leu Thr Thr Phe Ala Gly Leu Val Ala Thr Val
1               5                   10                  15

Ala Ala His Gly Tyr Val Thr Gly Ile Val Ala Gly Gly Thr Tyr Thr
            20                  25                  30

Lys Gly Trp Leu Val Asn Tyr Ala Tyr Gln Asn Pro Met Pro Glu Ser
        35                  40                  45

Ile Gly Trp Ser Asp Gln Asn Leu Asp Leu Gly Phe Thr Ala Pro Ser
    50                  55                  60

Ala Tyr Ala Thr Gly Asp Ile Ile Cys Ala Lys Asn Gly Thr Asn Ala
65                  70                  75                  80

Ala Leu Ser Ala Lys Val Ala Ala Gly Asp Thr Val Asp Phe Gln Trp
                85                  90                  95

Thr Val Trp Pro Asp Ser His Lys Gly Pro Val Ile Thr Tyr Leu Ala

            100                 105                 110

Asn Cys Asn Gly Asp Cys Ser Thr Val Asp Lys Thr Lys Leu Glu Phe
        115                 120                 125

Phe Lys Ile Asp Glu Gly Gly Leu Ile Asp Asp Thr Thr Val Pro Gly
    130                 135                 140

Thr Trp Ala Ser Asp Asn Leu Ile Thr Asn Asn Asn Thr Trp Thr Ser
145                 150                 155                 160

Thr Ile Pro Ala Asp Leu Ala Pro Gly Asn Tyr Val Ala Arg His Glu
                165                 170                 175

Ile Ile Ala Leu His Gly Ala Gly Ser Asn Asp Gly Ala Gln Asn Tyr
            180                 185                 190

Pro Gln Cys Ile Asn Leu Glu Val Thr Gly Ser Gly Thr Leu Ala Pro
        195                 200                 205

Ser Gly Thr Leu Gly Glu Lys Leu Tyr Thr Pro Thr Asp Pro Gly Ile
    210                 215                 220

Leu Val Asn Ile Tyr Ala Lys Leu Thr Tyr Thr Ile Pro Gly Pro Pro
225                 230                 235                 240

Met Tyr Asn Ala Thr Gly Ala Ala Thr Gly Ala Ala Thr Gly Ala Thr
                245                 250                 255

Thr Pro Ala Thr Gly Lys Thr Ala Ser Ala Thr Ser Ala Ala Ala Ala
            260                 265                 270

Leu Ser Thr Pro Ala Val Val Ser Thr Pro Pro Thr Pro Thr Thr Met
        275                 280                 285

Ile Thr Ser Val Ala Pro Ala Ala Thr Pro Ala Thr Ser Ala Ala Ala
    290                 295                 300

Ser Ser Gly Ala Ser Gly Asp Asp Asp Asp Thr Cys Glu Ala
305                 310                 315

<210> 39
<211> 404
<212> PRT
<213> Penicillium roqueforti

<220>
<221> SIGNAL

<222>  (1)..(19)

<400>  39

Met Ser Val Ser Lys Ile Ala Gly Leu Leu Leu Ser Ser Ala Ala Met
1               5                   10                  15

Val Ala Gly His Gly Phe Val Ser Gly Ala Val Val Asp Gly Thr Tyr
            20                  25                  30

His Gly Gly Phe Ile Val Asn Thr Tyr Asn Tyr Met Thr Glu Val Pro
        35                  40                  45

Ala Asn Ile Gly Trp Ser Glu Lys Ala Thr Asp Leu Gly Tyr Ile Asp
    50                  55                  60

Gly Ser Gly Tyr Thr Gly Ser Asp Ile Ile Cys His Lys Glu Ala Thr
65                  70                  75                  80

Pro Gly Ala Ile Ser Ala Glu Val Lys Ala Gly Gly Ser Val Glu Leu
                85                  90                  95

Gln Trp Thr Asp Trp Pro Glu Ser His His Gly Pro Val Ile Thr Tyr
            100                 105                 110

Met Ala Asn Cys Asn Gly Asp Cys Ser Val Val Asp Lys Thr Thr Leu
        115                 120                 125

Lys Phe Phe Lys Ile Ala Glu Ala Gly Leu Ile Asp Asp Thr Asn Val
    130                 135                 140

Pro Gly Thr Trp Ala Ser Asp Gln Leu Ile Ala Ala Asn Asn Ser Ala
145                 150                 155                 160

Thr Val Thr Ile Pro Ser Thr Ile Ala Ala Gly Asn Tyr Val Leu Arg
                165                 170                 175

His Glu Ile Ile Ala Leu His Ser Ala Gly Asp Ala Asn Gly Ala Gln
            180                 185                 190

Asn Tyr Pro Gln Cys Ile Asn Leu Lys Val Thr Gly Gly Gly Ser Asp
        195                 200                 205

Ser Pro Asp Gly Val Leu Gly Thr Ala Leu Tyr Thr Pro Thr Asp Pro
    210                 215                 220

Gly Ile Leu Ile Ser Ile Tyr Ser Ala Leu Glu Ser Tyr Val Ile Pro
225                 230                 235                 240

Gly Pro Ala Leu Tyr Thr Gly Ala Ser Ser Gly Ser Ser Ser Thr Ser
                245                 250                 255

Ser Thr Thr Ala Ala Ala Thr Thr Glu Ala Thr Thr Ala Ser Ala Thr
            260                 265                 270

Val Ser Thr Thr Ala Ala Pro Thr Val Ala Thr Thr Ser Ala Ser Thr
        275                 280                 285

Thr Ala Ser Thr Ala Asp Ser Ile Thr Ile Thr Thr Thr Ala Gly Gly
    290                 295                 300

Leu Ser Ala Gln Ser Ser Ala Ala Val Leu Glu Ser Ser Ala Thr Ser
305                 310                 315                 320

Thr Val Thr Ala Glu Ala Thr Ala Thr Ala Ala Pro Thr Ala Ala Ala
                325                 330                 335

Thr Thr Ser Thr Ser Ser Thr Ser Ser Gly Ser Ser Ser Ser Ser Ser
            340                 345                 350

Thr Ser Ser Ser Gly Ser Ser Ser Thr Ser Ser Thr Ser Thr Ser Asn
        355                 360                 365

Ser Ser Tyr Leu Ser Ser Leu Ser Ala Glu Lys Leu Leu Glu Val Ile
    370                 375                 380

Arg Ser Thr Leu Lys Trp Leu Val Ser Asp Lys Lys Val His Ala Arg
385                 390                 395                 400

Ala Leu Ala Tyr

<210>  40
<211>  245
<212>  PRT
<213>  Penicillium italicum

<220>
<221>  SIGNAL
<222>  (1)..(18)

<400>  40

Met Thr Leu Ser Lys Leu Ser Met Leu Leu Ala Ser Ala Ser Leu Ala
1               5                   10                  15

Ala Gly His Gly Tyr Val Ser Ser Ile Val Ala Asn Gly Lys Asn Tyr
            20                  25                  30

Thr Gly Tyr Ile Val Asp Lys Tyr Ala Tyr Met Ser Asp Pro Pro Asp
        35                  40                  45

Ser Val Gly Trp Ala Thr Thr Ala Thr Asp Leu Gly Phe Glu Asp Gly
    50                  55                  60

Thr Glu Tyr Gln Asn Pro Asn Ile Ile Cys His Arg Gly Gly Val Asn
65                  70                  75                  80

Ala Ala Leu Ser Ala Thr Val Ala Ala Gly Ser Ser Ile Asp Ile Gln
                85                  90                  95

Trp Thr Thr Trp Pro Asp Ser His His Gly Pro Leu Ile Thr Tyr Leu
            100                 105                 110

Ala Ser Cys Asn Gly Asp Cys Ser Thr Val Asp Lys Thr Thr Leu Lys
        115                 120                 125

Phe Phe Lys Ile Glu Glu Asp Gly Leu Ile His Gly Ala Thr Pro Pro
    130                 135                 140

Gly Thr Trp Ala Thr Asp Asp Leu Ile Ala Ser Gly Gly Gln Trp Thr
145                 150                 155                 160

Val Arg Ile Pro Ser Thr Ile Ala Thr Gly Asn Tyr Val Met Arg His
                165                 170                 175

Glu Ile Ile Ala Leu His Ser Ala Gly Gln Leu Asn Gly Ala Gln Asn
            180                 185                 190

Tyr Pro Gln Cys Phe Asn Leu Gln Val Thr Gly Gly Gly Gly Asn Ala
        195                 200                 205

Pro Asn Gly Thr Leu Gly Glu Lys Leu Tyr Thr Asn Thr Asp Pro Gly
    210                 215                 220

Ile Leu Phe Asn Ile Tyr Ser Thr Leu Thr Ser Tyr Val Ile Pro Gly
225                 230                 235                 240

Pro Ala Leu Tyr Ala
                245

<210>  41
<211>  372
<212>  PRT
<213>  Aspergillus flavus

<220>
<221>  SIGNAL
<222>  (1)..(20)

<400>  41

Met Ser Ile Ala Lys Ile Ala Gly Ile Val Leu Gly Ser Ala Ala Leu
1               5                   10                  15

Val Ala Ala His Gly His Val Ser Gly Ala Val Val Asp Gly Gln Tyr
            20                  25                  30

Tyr Pro Gly Tyr Asp Ile Ser Tyr His Tyr Met Pro Asp Pro Pro Lys
        35                  40                  45

Val Ile Gly Trp Ser Thr Asp Ala Thr Asp Asn Gly Phe Val Asp Gly
    50                  55                  60

Ser Ser Tyr Ala Asp Ala Asp Ile Ile Cys His Lys Asn Ala Lys Asn
65                  70                  75                  80

Gly Ala Ile Ser Ala Glu Ile Ala Ala Gly Lys Gln Val Glu Leu Gln
                85                  90                  95

Trp Thr Ala Trp Pro Glu Ser His Lys Gly Pro Val Ile Thr Tyr Leu
            100                 105                 110

Ala Asn Cys Asn Gly Asp Cys Ala Thr Val Asp Lys Thr Gln Leu Glu
        115                 120                 125

Phe Phe Lys Ile Asp Glu Lys Gly Leu Ile Ser Gly Ser Asp Asn Thr
    130                 135                 140

Trp Ala Thr Asp Asn Leu Ile Ala Ser Asn Asn Ser Trp Thr Val Thr
145                 150                 155                 160

Ile Pro Ser Ser Ile Ala Ala Gly Asn Tyr Val Met Arg His Glu Ile
                165                 170                 175

Ile Gly Leu His Ser Ala Gly Gln Lys Asp Gly Ala Gln Asn Tyr Pro
            180                 185                 190

Gln Cys Leu Asn Phe Lys Val Thr Gly Gly Gly Ser Asp Lys Pro Glu
        195                 200                 205

Gly Thr Leu Gly Thr Ala Leu Tyr Lys Asp Thr Asp Pro Gly Ile Gln
    210                 215                 220

Ile Asn Ile Tyr Gln Thr Leu Thr Ser Tyr Thr Ile Pro Gly Pro Ala
225                 230                 235                 240

Leu Tyr Ser Gly Ser Ser Ser Ser Gly Ser Ser Gly Ser Gly Ser Ser
                245                 250                 255

Ser Ala Ala Pro Ser Ala Thr Ala Ser Ala Ser Ala Ser Ala Ser Ala
            260                 265                 270

Thr Ala Ala Pro Val Gln Thr Ser Thr Ala Thr Ala Tyr Gln Thr Ser
        275                 280                 285

Thr Ala Val Ala Ser Val Thr Val Thr Gly Ser Ala Pro Ala Gln Thr
    290                 295                 300

His Val Gln Ala Thr Ser Ser Ser Ala Ala Ala Ser Thr Pro Thr Ala
305                 310                 315                 320

Ser Ser Gly Ala Ser Thr Gly Ser Gly Ser Gly Ser Ser Ser Ser Asp
                325                 330                 335

Leu Thr Gly Tyr Phe Asn Ser Leu Ser Ala Asp Glu Leu Leu Asn Val
            340                 345                 350

Ile Lys Gln Thr Leu Ser Trp Leu Val Thr Asp Lys Ile His Ala Arg
        355                 360                 365

Asp Ile Ser Ala
    370

<210>  42
<211>  245
<212>  PRT
<213>  Penicillium crysogenum

<220>
<221>  SIGNAL
<222>  (1)..(18)

<400>  42

Met Thr Leu Ser Lys Leu Ser Thr Leu Leu Ala Ser Ala Thr Leu Val
1               5                   10                  15

Ala Gly His Gly Tyr Val Ser Ser Ile Val Ala Asp Gly Lys Ser Tyr
            20                  25                  30

Thr Gly Tyr Leu Val Asp Lys Tyr Ala Tyr Met Ala Asp Pro Pro Asp
        35                  40                  45

Ser Val Gly Trp Thr Thr Thr Ala Thr Asp Leu Gly Phe Glu Asp Gly
    50                  55                  60

Thr Glu Tyr Gln Asp Pro Asn Ile Ile Cys His Arg Asp Gly Ala Asn
65                  70                  75                  80

Ala Ala Leu Ser Ala Thr Val Lys Ala Gly Ser Ser Ile Asp Leu Gln
                85                  90                  95

Trp Thr Thr Trp Pro Asp Ser His His Gly Pro Val Ile Thr Tyr Leu
            100                 105                 110

Ala Ser Cys Asn Gly Asp Cys Thr Thr Val Asp Lys Thr Thr Leu Glu
        115                 120                 125

Phe Phe Lys Ile Glu Glu Glu Gly Leu Ile Asp Gly Ala Asn Pro Pro
    130                 135                 140

Gly Thr Trp Ala Thr Asp Asp Leu Ile Ala Ala Gly Gly Lys Trp Thr
145                 150                 155                 160

Val Lys Ile Pro Ser Thr Ile Ala Pro Gly Asn Tyr Val Met Arg His
                165                 170                 175

Glu Ile Ile Ala Leu His Ser Ala Gly Gln Thr Asn Gly Ala Gln Asn
            180                 185                 190

Tyr Pro Gln Cys Phe Asn Leu Glu Val Thr Gly Gly Gly Ser Asp Thr
        195                 200                 205

Pro Asp Gly Thr Pro Gly Met Glu Leu Tyr Thr Asn Thr Asp Pro Gly
    210                 215                 220

Ile Leu Phe Asp Ile Tyr Ser Ser Leu Thr Ser Tyr Asp Ile Pro Gly
225                 230                 235                 240

Pro Ala Leu Tyr Ala
                245

<210>  43
<211>  245
<212>  PRT
<213>  Penicillium expansum

<220>
<221>  SIGNAL
<222>  (1)..(18)

<400>  43

Met Thr Phe Ser Lys Leu Ser Leu Leu Leu Ala Ser Ala Ser Phe Val
1               5                   10                  15

Ala Gly His Gly Tyr Val Ser Ser Ile Val Ala Asn Gly Lys Asn Tyr
            20                  25                  30

Thr Gly Tyr Ile Val Asp Lys Tyr Ala Tyr Met Ser Asp Pro Pro Asp
        35                  40                  45

Ser Val Gly Trp Ala Thr Thr Ala Thr Asp Leu Gly Phe Glu Asp Gly
    50                  55                  60

Thr Glu Tyr Gln Asp Pro Asn Ile Ile Cys His Arg Gly Gly Ile Asn
65                  70                  75                  80

Ala Ala Leu Ser Ala Pro Val Thr Ala Gly Ser Ser Ile Asp Ile Gln
                85                  90                  95

Trp Thr Thr Trp Pro Asp Ser His His Gly Pro Leu Val Thr Tyr Leu
            100                 105                 110

Ala Ser Cys Asn Gly Asp Cys Ser Thr Val Asp Lys Thr Thr Leu Lys
        115                 120                 125

Phe Phe Lys Ile Glu Glu Asp Gly Leu Ile Asp Gly Thr Thr Pro Pro
    130                 135                 140

Gly Thr Trp Ala Thr Asp Asp Leu Ile Ala Ala Gly Gly Gln Trp Thr
145                 150                 155                 160

Val Arg Ile Pro Ser Thr Ile Ala Ala Gly Asn Tyr Val Met Arg His
                165                 170                 175

Glu Ile Ile Ala Leu His Ser Ala Gly Gln Ser Asn Gly Ala Gln Asn
            180                 185                 190

Tyr Pro Gln Cys Phe Asn Leu Gln Val Thr Gly Gly Gly Ser Asp Val
        195                 200                 205

Pro Asp Gly Thr Leu Gly Glu Lys Leu Tyr Thr Asn Thr Asp Pro Gly
    210                 215                 220

Ile Leu Phe Asn Leu Tyr Ser Thr Leu Thr Ser Tyr Ile Ile Pro Gly
225                 230                 235                 240

Pro Ala Leu Tyr Ala

                245

<210>  44
<211>  393
<212>  PRT
<213>  Penicillium rubens

<220>
<221>  SIGNAL
<222>  (1)..(19)

<400>  44

Met Ser Val Ser Lys Ile Ala Gly Leu Met Leu Ser Ser Ala Ala Met
1               5                   10                  15

Val Ala Gly His Gly Phe Val Ser Gly Ala Val Val Asp Gly Thr Tyr
            20                  25                  30

His Gly Gly Tyr Leu Val Asn Asn Tyr Pro Tyr Asn Asp Asn His Pro
        35                  40                  45

Glu Thr Ile Gly Trp Ala Glu Lys Ala Thr Asp Leu Gly Phe Val Asp
    50                  55                  60

Gly Ser Gly Tyr Ser Gly Pro Asp Ile Ile Cys His Lys Glu Ala Thr
65                  70                  75                  80

Pro Gly Ala Ile Ser Ala Glu Val Lys Ala Gly Gly Asp Val Glu Leu
                85                  90                  95

Gln Trp Thr Glu Trp Pro Glu Ser His His Gly Pro Val Ile Asn Tyr
            100                 105                 110

Leu Ala Asn Cys Asn Gly Asp Cys Ser Lys Val Asp Lys Lys Thr Leu
        115                 120                 125

Lys Trp Phe Lys Ile Ala Glu Ser Gly Leu Ile Asp Gly Ser Asn Ala
    130                 135                 140

Pro Gly Lys Trp Ala Ser Asp Glu Leu Ile Ala Asn Asn Asn Ser Ala
145                 150                 155                 160

Ser Val Thr Ile Pro Ser Ser Ile Ala Ala Gly Asn Tyr Val Leu Arg
                165                 170                 175

His Glu Ile Ile Ala Leu His Ser Ala Gly Gln Glu Asn Gly Ala Gln
            180                 185                 190

Asn Tyr Pro Gln Cys Leu Asn Leu Lys Val Thr Gly Gly Gly Ser Asp
        195                 200                 205

Val Pro Glu Gly Val Val Gly Thr Glu Leu Tyr Lys Pro Asp Asp Ala
    210                 215                 220

Gly Ile Leu Val Ser Ile Tyr Asn Gln Leu Lys Asp Tyr Thr Ile Pro
225                 230                 235                 240

Gly Pro Ala Leu Tyr Lys Gly Ala Ser Ser Gly Ser Gly Ser Lys Thr
                245                 250                 255

Thr Asp Ala Ala Ser Thr Ala Thr Thr Ala Ser Ala Ser Thr Val Ser
            260                 265                 270

Ala Ser Pro Ile Gln Ser Ser Ser Ser His His Leu Thr Arg Thr Arg
        275                 280                 285

Thr Ala Arg Pro Thr Tyr Ser Pro Ser Gly Thr Pro Thr Pro Ser Ser
    290                 295                 300

Ser Ala Val Ser Ala Ala Ser Thr Ser Thr Pro Ile Gly Ser Val Pro
305                 310                 315                 320

Ala Ser Gly Gly Glu Ala Ser Glu Pro Ser Ala Thr Pro Ala Pro Ser
                325                 330                 335

Ser Ser Gly Ser Ser Ser Gly Ser Ser Ser Ser Ser Gly Ser Ser Ser
            340                 345                 350

Glu Ser Gly Asp Tyr Ala Ser Tyr Leu Ser Ser Leu Ser Ala Glu Lys
        355                 360                 365

Leu Leu Glu Val Ile Arg Ser Thr Leu Lys Trp Leu Val Ser Asp Asn
    370                 375                 380

Lys Val His Ala Arg Ala Leu Ala His
385                 390

<210>  45
<211>  54
<212>  DNA
<213>  Aspergillus niger

<400>  45
atgtcgttcc gatctcttct cgccctgagc ggccttgtct gctcggggtt ggca          54

<210>  46
<211>  18

<212>  PRT
<213>  Aspergillus niger

<400>  46

Met Ser Phe Arg Ser Leu Leu Ala Leu Ser Gly Leu Val Cys Ser Gly
1               5                   10                  15

Leu Ala

<210>  47
<211>  54
<212>  DNA
<213>  Myceliophthora thermophila

<400>  47
atgaagtcct tcaccctcac cactctggcc gccctggctg gcaacgccgc cgct          54

<210>  48
<211>  18
<212>  PRT
<213>  Myceliophthora thermophila

<400>  48

Met Lys Ser Phe Thr Leu Thr Thr Leu Ala Ala Leu Ala Gly Asn Ala
1               5                   10                  15

Ala Ala

<210>  49
<211>  343
<212>  PRT
<213>  Byssochlamys spectabilis

<400>  49

His Gly Tyr Val Gln Gly Ile Val Ala Asp Gly Lys Tyr Tyr Gly Gly
1               5                   10                  15

Tyr Ile Val Thr Gln Tyr Pro Tyr Thr Asn Asp Pro Pro Glu Leu Val
            20                  25                  30

Ala Trp Ser Thr Glu Ala Thr Asp Leu Gly Phe Val Asp Gly Ser Gly
        35                  40                  45

Tyr Ala Ser Gly Asp Ile Ile Cys His Lys Ser Ala Gln Pro Ala Ala
    50                  55                  60

Leu Ala Ala Glu Val Pro Ala Gly Gly Lys Val Glu Leu Gln Trp Thr
65                  70                  75                  80

Glu Trp Pro Glu Ser His His Gly Pro Val Leu Asp Tyr Leu Ala Pro
                85                  90                  95

Cys Asn Gly Asn Cys Ala Asp Val Asp Lys Thr Ala Leu Glu Phe Phe
            100                 105                 110

Lys Ile Asp Gln Gly Gly Leu Val Asp Asp Ser Ser Pro Pro Gly Thr
        115                 120                 125

Trp Ala Ser Asp Gln Leu Ile Ser Asn Asn Asn Ser Trp Thr Val Thr
    130                 135                 140

Ile Pro Ser Thr Ile Ala Pro Gly Asn Tyr Val Leu Arg His Glu Ile
145                 150                 155                 160

Ile Ala Leu His Ser Ala Gly Asn Lys Asp Gly Ala Gln Asn Tyr Pro
                165                 170                 175

Gln Cys Ile Asn Ile Lys Val Thr Gly Ser Gly Thr Asp Ser Pro Ser
            180                 185                 190

Gly Thr Leu Gly Thr Ser Leu Tyr Lys Asp Thr Asp Pro Gly Ile Leu
        195                 200                 205

Val Asn Ile Tyr Gln Thr Leu Ser Asp Tyr Ser Ile Pro Gly Pro Ala
    210                 215                 220

Leu Tyr Glu Gly Gly Ser Ser Ser Gly Thr Ser Ser Gly Ser Gly Thr
225                 230                 235                 240

Ser Thr Ala Thr Ser Thr Ser Val Ala Ser Ser Thr Ser Val Lys Pro
                245                 250                 255

Ser Ser Ile Ser Thr Pro Ser Ser Val Gln Val Ala Ile Pro Ser Ala
            260                 265                 270

Lys Thr Ser Ala Val Pro Ala Thr Thr Gln Ala Pro Thr Thr Ala Thr
        275                 280                 285

Pro Ala Val Thr Ile Pro Thr Glu Ser Ala Leu Thr Asp Tyr Leu Asn
    290                 295                 300

Ser Met Ser Ala Asp Glu Val Leu Asn Leu Val Arg Gly Thr Met Ser
305                 310                 315                 320

Trp Leu Phe Thr Asn Lys Asn His Ala Arg Asp Leu Ser Ala Ser Ser
                325                 330                 335

Glu Leu Thr Asp Arg Pro Met
                340

<210>  50
<211>  228
<212>  PRT
<213>  Penicillium oxalicum

<400>  50

His Gly Phe Val Ser Ser Ile Met Ala Gly Gly Lys Asn Tyr Thr Gly
1               5                   10                  15

Tyr Leu Ala Asn Ser Tyr Pro Tyr Met Ser Asn Pro Pro Gln Ser Val
            20                  25                  30

Gly Trp Ala Thr Thr Ala Thr Asp Leu Gly Phe Glu Asp Gly Thr Glu
        35                  40                  45

Tyr Gln Ser Ala Asn Ile Ile Cys His Arg Asp Gly Lys Asn Ala Ala
    50                  55                  60

Leu Ser Ala Pro Val Thr Ala Gly Ser Lys Val Glu Ile Gln Trp Thr
65                  70                  75                  80

Ser Trp Pro Asp Ser His His Gly Pro Val Ile Thr Tyr Leu Ala Ser
                85                  90                  95

Cys Gly Gly Asp Cys Ser Thr Val Asp Lys Thr Thr Leu Lys Phe Phe
            100                 105                 110

Lys Ile Asp Glu Ala Gly Leu Ile Asp Asp Ser Asn Pro Pro Gly Thr
        115                 120                 125

Trp Ala Thr Asp Gln Leu Ile Ala Ala Gly Asn Arg Trp Thr Val Thr
    130                 135                 140

Ile Pro Lys Ser Ile Ala Ser Gly Asn Tyr Val Met Arg His Glu Ile
145                 150                 155                 160

Ile Ala Leu His Ser Ser Gly Ser Lys Asp Gly Ala Gln Asn Tyr Pro
                165                 170                 175

Gln Cys Leu Asn Leu Gln Val Thr Gly Gly Gly Ser Ala Lys Pro Gln
            180                 185                 190

Gly Thr Leu Gly Glu Ala Leu Tyr Lys Asp Thr Asp Pro Gly Ile Leu
        195                 200                 205

Ile Asn Ile Tyr Gln Ser Leu Lys Ser Tyr Val Ile Pro Gly Pro Ala
    210                 215                 220

Leu Tyr Ser Ala
225

<210>  51
<211>  324
<212>  PRT
<213>  Aspergillus clavatus

<400>  51

His Gly Phe Val Thr Gly Ala Val Val Asp Gly Thr Tyr His Thr Gly
1               5                   10                  15

Tyr Leu Val Asn Asn Tyr Pro Tyr Ser Ser Asn Pro Pro Lys Thr Ile
            20                  25                  30

Gly Trp Ser Glu Thr Ala Thr Asp Leu Gly Phe Val Asp Gly Thr Gly
        35                  40                  45

Tyr Ala Ser Gly Asp Ile Ile Cys His Lys Asn Ala Lys Pro Gly Ala
    50                  55                  60

Leu Ser Ala Asp Ile Lys Ala Gly Gly Lys Val Glu Phe Gln Trp Thr
65                  70                  75                  80

Gln Trp Pro Glu Ser His His Gly Pro Val Ile Thr Tyr Met Ala Asn
                85                  90                  95

Cys Asn Gly Asp Cys Ala Ser Val Asp Lys Ser Gln Leu Glu Phe Phe
            100                 105                 110

Lys Ile Glu Glu Lys Gly Leu Ile Gly Ser Gly His Trp Ala Ser Asp
        115                 120                 125

Asp Leu Ile Ala Asn Asn Asn Ser Trp Thr Val Thr Val Pro Ser Ser
    130                 135                 140

Leu Ala Ala Gly Asn Tyr Val Met Arg His Glu Ile Ile Gly Leu His
145                 150                 155                 160

Ser Ala Gly Asn Ala Asn Gly Ala Gln Asn Tyr Pro Gln Cys Ile Asn
                165                 170                 175

Leu Lys Val Thr Gly Gly Gly Ser Asp Lys Pro Val Gly Thr Leu Gly
            180                 185                 190

Thr Lys Leu Tyr Lys Ala Thr Asp Pro Gly Ile Leu Val Asn Ile Tyr
        195                 200                 205

Gly Ser Leu Thr Ser Tyr Thr Ile Pro Gly Pro Ala Leu Trp Thr Gly
    210                 215                 220

Ala Ser Ser Gly Gly Ser Asp Asp Asn Gly Ser Thr Pro Ala Ser Thr
225                 230                 235                 240

Pro Thr Pro Thr Ala Ala Pro Ser Ser Thr Ala Ala Pro Ser Val Thr
                245                 250                 255

Ile Thr Ser Thr Pro Gly Gln Lys Thr Gln Thr Pro Val Ala Thr Pro
            260                 265                 270

Ala Pro Ser Thr Pro Ser Ser Thr Pro Gly Leu Pro Thr Asp Gly Asn
        275                 280                 285

Leu Thr Asp Tyr Phe Lys Ser Leu Ser Pro Glu Gln Phe Leu Ser Val
    290                 295                 300

Leu Lys Glu Thr Phe Ser Trp Leu Val Thr Glu Lys Val His Ala Arg
305                 310                 315                 320

Ser Leu Ser Ala

<210>  52
<211>  354
<212>  PRT
<213>  Aspergillus fumigatus

<400>  52

His Gly Phe Val Thr Gly Ala Val Val Asp Gly Lys Tyr Tyr Thr Gly
1               5                   10                  15

Tyr Leu Val Asn Gln Tyr Pro Tyr Met Ser Ser Pro Pro Asp Ser Ile
            20                  25                  30

Gly Trp Ser Glu Thr Ala Thr Asp Leu Gly Phe Val Asp Gly Ser Gly
        35                  40                  45

Tyr Ser Ser Gly Asp Ile Ile Cys His Lys Asp Ala Lys Asn Gly Ala
    50                  55                  60

Ile Ser Ala Glu Ile Lys Ala Gly Gly Lys Val Glu Phe Gln Trp Thr
65                  70                  75                  80

Glu Trp Pro Glu Ser His His Gly Pro Val Ile Thr Tyr Met Ala Asn
                85                  90                  95

Cys Asn Gly Asp Cys Ala Ser Val Asp Lys Thr Thr Leu Glu Phe Phe
            100                 105                 110

Lys Ile Asp Glu Ser Gly Leu Ile Ser Asp Ser Asn Val Pro Gly Thr
        115                 120                 125

Trp Ala Ser Asp Asn Leu Ile Ala Asn Asn Asn Ser Trp Thr Val Thr
    130                 135                 140

Val Pro Ser Ser Ile Ala Ala Gly Asn Tyr Val Met Arg His Glu Ile
145                 150                 155                 160

Ile Ala Leu His Ser Ala Gly Asn Gln Asn Gly Ala Gln Asn Tyr Pro
                165                 170                 175

Gln Cys Ile Asn Leu Lys Val Thr Gly Gly Gly Ser Asp Lys Pro Ala
            180                 185                 190

Gly Thr Leu Gly Thr Ala Leu Tyr Lys Asn Thr Asp Ala Gly Ile Leu
        195                 200                 205

Val Asn Ile Tyr Gln Ser Leu Ser Ser Tyr Glu Ile Pro Gly Pro Ala
    210                 215                 220

Leu Tyr Ser Gly Ala Ser Ser Gly Ser Ser Asn Asn Gly Gly Ser Ala
225                 230                 235                 240

Ser Ser Ser Ala Thr Ala Pro Ser Ala Thr Ile Thr Gln Pro Ser Thr
                245                 250                 255

Ala Val Pro Thr Ser Ser Ala Thr Ala Tyr Gln Pro Ser Thr Thr Thr
            260                 265                 270

Glu Ala Val Thr Val Thr Ser Ile Pro Ala Gln Gln Ser Tyr Val Gln
        275                 280                 285

Ala Pro Thr Ala Thr Pro Ser Ser Thr Ala Gly Ser Ser Gly Ser Gly
    290                 295                 300

Ser Gly Ser Ser Ser Ser Gly Thr Leu Pro Ser Ser Ser Asn Leu Thr
305                 310                 315                 320

Glu Tyr Phe Asn Ser Leu Ser Ala Glu Glu Phe Leu Lys Val Leu Lys

325                 330                 335

Gln Thr Phe Ser Trp Leu Val Thr Glu Lys Val His Ala Arg Asp Leu
            340                 345                 350

Ser Ala

<210>  53
<211>  383
<212>  PRT
<213>  Aspergillus niger

<400>  53
   His Gly Tyr Val Ser Gly Ala Val Ile Asp Gly Glu Tyr Tyr Gly Gly
   1               5                   10                  15
   Tyr Ile Val Ser Ser Tyr Pro Tyr Glu Ser Asp Pro Pro Glu Thr Ile
               20                  25                  30
   Ala Trp Ser Thr Thr Ala Thr Asp Leu Gly Phe Val Asp Gly Ser Glu
           35                  40                  45
   Tyr Ser Asp Pro Asp Ile Ile Cys His Lys Ser Ala Lys Pro Gly Ala
       50                  55                  60
   Ile Ser Ala Asp Val Lys Ala Gly Gly Thr Val Glu Leu Gln Trp Thr
   65                  70                  75                  80
   Asp Trp Pro Ser Ser His His Gly Pro Val Leu Thr Tyr Leu Ala Asn
                   85                  90                  95
   Cys Asn Gly Asp Cys Ser Asp Val Thr Lys Thr Asp Leu Glu Phe Phe
               100                 105                 110
   Lys Ile Asp Glu Ser Gly Leu Ile Ser Asp Thr Glu Val Pro Gly Thr
           115                 120                 125
   Trp Ala Thr Asp Asn Leu Ile Ser Asn Asn Asn Ser Trp Thr Val Thr
       130                 135                 140
   Ile Pro Ser Thr Leu Glu Ala Gly Asn Tyr Val Leu Arg His Glu Ile
   145                 150                 155                 160
   Ile Ala Leu His Ser Ala Glu Asn Lys Asp Gly Ala Gln Asn Tyr Pro
                   165                 170                 175
   Gln Cys Leu Asn Leu Lys Val Thr Gly Ser Gly Ser Ser Thr Tyr Ser
   180 185 190
   Gly Thr Lys Gly Glu Ala Leu Tyr Lys Asp Thr Asp Pro Gly Ile Leu
   195 200 205
   Val Asn Ile Tyr Glu Thr Leu Ser Ser Tyr Asp Ile Pro Gly Pro Ala
   210 215 220
   Met Tyr Asn Ala Thr Ser Ser Ser Ser Ser Ser Gly Ser Ser Ser Gly
   225 230 235 240
   Ala Ser Ser Thr Ser Ser Ala Ala Ala Ala Ala Ala Thr Thr Ser Ser
   245 250 255
   Ala Ser Thr Ser Ser Ala Ser Thr Pro Thr Thr Ser Ala Ala Ala Ala
   260 265 270
   Thr Ser Ser Ala Val Ser Ala Thr Thr Thr Ala Ala Ala Ala Val Val
   275 280 285
   Thr Asp Val Glu Thr Thr Val Gln Thr Ala Val Ala Thr Ala Tyr Gln
   290 295 300
   Thr Ser Thr Asp Val Val Gln Val Thr Val Thr Gly Ser Ala Pro Gln
   305 310 315 320
   Gln Thr His Ile Gln Val Ala Ser Ser Ser Ala Ala Ala Ser Thr Pro
   325 330 335
   Ser Ser Ser Ser Val Ala Val Ser Ser Asp Asn Leu Thr Ser Tyr Phe
   340 345 350
   Ser Ser Leu Ser Ala Asp Gln Phe Leu Ser Val Leu Lys Glu Thr Phe
   355 360 365
   Ser Trp Leu Val Thr Glu Lys Lys His Ala Arg Asp Leu Asn Ala
   370 375 380

<210>  54
<211>  334
<212>  PRT
<213>  Aspergillus niger

<400>  54

His Gly Tyr Val Ser Gly Ala Val Ile Asp Gly Glu Tyr Tyr Gly Gly
1               5                   10                  15

Tyr Ile Val Ser Ser Tyr Ala Tyr Glu Ser Asp Pro Pro Glu Thr Ile

20                  25                  30

Ala Trp Ser Thr Thr Ala Thr Asp Leu Gly Phe Val Asp Gly Ser Glu
        35                  40                  45

Tyr Ser Asp Pro Asp Ile Ile Cys His Lys Ser Ala Lys Pro Gly Ala
    50                  55                  60

Ile Ser Ala Asp Val Lys Ala Gly Gly Thr Val Glu Leu Gln Trp Thr
65                  70                  75                  80

Asp Trp Pro Ser Ser His His Gly Pro Val Leu Thr Tyr Leu Ala Asn
                85                  90                  95

Cys Asn Gly Asp Cys Ser Asp Val Thr Lys Thr Asp Leu Glu Phe Phe
            100                 105                 110

Lys Ile Asp Glu Ser Gly Leu Ile Ser Asp Thr Glu Val Pro Gly Thr
        115                 120                 125

Trp Ala Thr Asp Asn Leu Ile Ser Asn Asn Asn Ser Trp Thr Val Thr
    130                 135                 140

Ile Pro Ser Thr Leu Glu Ala Gly Asn Tyr Val Leu Arg His Glu Ile
145                 150                 155                 160

Ile Ala Leu His Ser Ala Glu Asn Lys Asp Gly Ala Gln Asn Tyr Pro
                165                 170                 175

Gln Cys Leu Asn Leu Lys Val Thr Gly Ser Gly Ser Ser Thr Tyr Ser
            180                 185                 190

Gly Thr Lys Gly Glu Ala Leu Tyr Lys Asp Thr Asp Pro Gly Ile Leu
        195                 200                 205

Val Asn Ile Tyr Glu Thr Leu Ser Ser Tyr Asp Ile Pro Gly Pro Ala
    210                 215                 220

Ile Ser Ala Val Ser Ala Thr Thr Thr Ala Ala Ala Ala Val Val Thr
225                 230                 235                 240

Asp Val Glu Thr Thr Val Gln Thr Ala Val Ala Thr Ala Tyr Gln Thr
                245                 250                 255

Ser Thr Asp Val Val Gln Val Thr Val Thr Gly Ser Ala Pro Gln Gln
            260                 265                 270

Thr His Ile Gln Val Ala Ser Ser Ser Ala Ala Ala Ser Thr Pro Ser
        275                 280                 285

Ser Ser Ser Val Ala Val Ser Ser Asp Asn Leu Thr Ser Tyr Phe Ser
    290                 295                 300

Ser Leu Ser Ala Asp Gln Phe Leu Ser Val Leu Lys Glu Thr Phe Ser
305                 310                 315                 320

Trp Leu Val Thr Glu Lys Lys His Ala Arg Asp Leu Asn Ala
                325                 330

<210>  55
<211>  325
<212>  PRT
<213>  Aspergillus ruber

<400>  55

His Gly Tyr Val Ser Gly Ala Val Val Asp Gly Gln Tyr Tyr Gly Gly
1               5                   10                  15

Tyr Ile Val Thr Ser Tyr Ala Tyr Thr Asp Ser Pro Pro Glu Thr Ile
            20                  25                  30

Gly Trp Ser Thr Glu Ala Thr Asp Leu Gly Phe Val Ser Pro Ser Ser
        35                  40                  45

Phe Ser Asp Pro Asp Ile Ile Cys His Lys Ser Ala Gln Pro Gly Ala
    50                  55                  60

Ile Ser Ala Ser Val Ala Ala Gly Lys Asp Val Glu Leu Gln Trp Thr
65                  70                  75                  80

Glu Trp Pro Glu Ser His His Gly Pro Val Ile Thr Tyr Leu Ala Asn
                85                  90                  95

Cys Asn Gly Asp Cys Ser Lys Val Asp Lys Thr Ser Leu Glu Phe Phe
            100                 105                 110

Lys Ile Asp Gln Lys Gly Leu Val Asp Asp Ser Asn Val Pro Gly Thr
        115                 120                 125

Trp Ala Ser Asp Asn Leu Ile Ser Asn Asn Asn Ser Tyr Thr Val Thr
    130                 135                 140

Ile Pro Ser Asp Ile Ala Ala Gly Asn Tyr Val Leu Arg His Glu Ile
145                 150                 155                 160

Ile Ala Leu His Ser Ala Gly Asn Glu Asp Gly Ala Gln Ser Tyr Pro
                165                 170                 175

Gln Cys Val Asn Leu Lys Val Thr Gly Gly Gly Ser Ala Ser Pro Ser
            180                 185                 190

Gly Thr Leu Gly Thr Lys Leu Tyr Ser Glu Asp Asp Pro Gly Ile Leu
        195                 200                 205

Val Asn Ile Tyr Gln Gln Leu Asp Ser Tyr Glu Ile Pro Gly Pro Ala
    210                 215                 220

Leu Tyr Ser Gly Ala Ser Ser Ser Ser Asn Ser Gly Ser Ser Ser Ser
225                 230                 235                 240

Thr Ala Ser Ala Ser Thr Thr Ala Thr Ser Ala Thr Thr Ser Ser Ala
                245                 250                 255

Ser Ser Thr Gln Ala Ser Ala Thr Pro Ala Ser Gln Ala Lys Ala Gln
            260                 265                 270

Val Ser Ser Ser Thr Pro Ser Ala Ser Ser Val Ala Thr Ser Gly Ser
        275                 280                 285

Leu Ser Asp Tyr Phe Ser Ser Leu Ser Ala Glu Glu Phe Leu Asn Val
    290                 295                 300

Ile Ser Glu Thr Leu Ser Trp Leu Val Thr Asp Lys Ile His Ala Arg
305                 310                 315                 320

Asp Ile Ser Thr Ala
                325

<210>  56
<211>  383
<212>  PRT
<213>  Aspergillus terreus

<400>  56

His Gly Tyr Val Thr Gly Ala Val Val Asp Gly Lys Tyr Tyr Ala Gly
1               5                   10                  15

Tyr Asp Val Thr Ser Ala Pro Tyr Ser Ser Asp Pro Pro Ala Thr Ile
            20                  25                  30

Gly Trp Ser Thr Thr Ala Thr Asp Leu Gly Phe Val Asp Gly Thr Glu
        35                  40                  45

Tyr Ser Glu Pro Asp Ile Ile Cys His Lys Asp Ala Lys Pro Gly Ser
    50                  55                  60

Leu Ser Ala Glu Ile Thr Ala Gly Gly Lys Val Glu Leu Gln Trp Thr
65                  70                  75                  80

Glu Trp Pro Glu Ser His His Gly Pro Val Ile Thr Tyr Leu Ala Asn
                85                  90                  95

Cys Asn Gly Asp Cys Ser Ser Val Asp Lys Thr Ser Leu Glu Phe Phe
            100                 105                 110

Lys Ile Asp Gln Lys Gly Leu Ile Glu Gly Asn Thr Trp Ala Ser Asp
        115                 120                 125

Gln Leu Ile Ser Asn Asn Asn Ser Trp Thr Val Thr Ile Pro Ser Ser
    130                 135                 140

Ile Ala Ser Gly Asn Tyr Val Leu Arg His Glu Ile Ile Gly Leu His
145                 150                 155                 160

Ser Ala Gly Asn Lys Asp Gly Ala Gln Asn Tyr Pro Gln Cys Met Asn
                165                 170                 175

Leu Lys Val Thr Gly Gly Gly Ser Asp Lys Pro Ala Gly Thr Leu Gly
            180                 185                 190

Thr Ala Leu Tyr Lys Asp Thr Asp Pro Gly Ile Leu Val Asn Ile Tyr
        195                 200                 205

Gln Thr Leu Ser Ser Tyr Val Ile Pro Gly Pro Ala Leu Tyr Ser Gly
    210                 215                 220

Ser Ser Ser Gly Ser Gly Ser Gly Ser Ala Ser Ser Ser Ala Ala Ala
225                 230                 235                 240

Thr Pro Ser Ser Ser Ser Val Pro Ser Ser Thr Ser Ile Ala Val Pro
                245                 250                 255

Thr Ser Ser Ala Pro Ala Pro Thr Ser Ala Ala Pro Ser Ala Gln Thr
            260                 265                 270

Ser Thr Ala Thr Ala Tyr Gln Thr Ala Thr Thr Ile Glu Ser Val Thr
        275                 280                 285

Val Thr Ala Glu Ala Pro Gln Gln Thr Lys Val Gln Asp Pro Ser Ser
    290                 295                 300

Gly Ser Ser Ser Gly Ser Asn Ser Gly Ser Ser Ser Gly Ser Ser Ser
305                 310                 315                 320

Gly Ser Ser Thr Gly Ser Ser Ser Gly Ser Ser Thr Gly Thr Gly Ser
                325                 330                 335

Ser Ser Val Pro Thr Pro Ser Gly Ser Ser Ser Leu Ser Asp Tyr Phe
            340                 345                 350

Asn Ser Leu Ser Ala Glu Gln Phe Leu Asn Leu Leu Arg Gln Thr Leu
        355                 360                 365

Ser Trp Leu Ile Thr Asp Lys Lys His Ala Arg Asp Leu Ser Ala
    370                 375                 380

<210>  57
<211>  348
<212>  PRT
<213>  Neosartorya fischeri

<400>  57

His Gly Phe Val Ser Gly Ala Val Val Asp Gly Lys Tyr Tyr Thr Gly
1               5                   10                  15

Tyr Leu Val Asn Gln Tyr Pro Tyr Met Ser Ser Pro Pro Glu Ser Ile
            20                  25                  30

Gly Trp Ser Glu Thr Ala Thr Asp Leu Gly Phe Val Asp Gly Ser Gly
        35                  40                  45

Tyr Ser Ser Gly Asp Ile Ile Cys His Lys Ser Ala Lys Asn Gly Ala
    50                  55                  60

Ile Ser Ala Glu Ile Lys Ala Gly Gly Lys Val Glu Phe Gln Trp Thr
65                  70                  75                  80

Glu Trp Pro Glu Ser His His Gly Pro Val Ile Thr Tyr Met Ala Asn
                85                  90                  95

Cys Asn Gly Asp Cys Ala Ser Val Asp Lys Thr Ser Leu Glu Phe Phe
            100                 105                 110

Lys Ile Asp Glu Ser Gly Leu Ile Ser Asp Ser Asn Val Pro Gly Thr
        115                 120                 125

Trp Ala Ser Asp Asn Leu Ile Ala Asn Asn Asn Ser Trp Thr Val Thr
    130                 135                 140

Val Pro Ser Ser Ile Ala Ala Gly Asn Tyr Val Met Arg His Glu Ile
145                 150                 155                 160

Ile Ala Leu His Ser Ala Gly Asn Gln Asn Gly Ala Gln Asn Tyr Pro
                165                 170                 175

Gln Cys Ile Asn Leu Lys Val Thr Gly Gly Gly Ser Asp Lys Pro Ala
            180                 185                 190

Gly Thr Leu Gly Thr Ala Leu Tyr Lys Asn Thr Asp Ala Gly Ile Leu
        195                 200                 205

Val Asn Ile Tyr Gln Ser Leu Ser Ser Tyr Asp Ile Pro Gly Pro Ala
    210                 215                 220

Leu Tyr Ser Gly Ala Ser Ser Gly Ser Ser Asn Ser Gly Gly Ser Ala
225                 230                 235                 240

Ser Ser Ser Ala Ala Ala Pro Ser Ala Thr Ile Thr Gln Thr Ser Thr
                245                 250                 255

Ala Val Gln Thr Ser Ser Ala Thr Val Tyr Gln Pro Ser Thr Thr Thr
            260                 265                 270

Glu Ala Val Thr Val Thr Ser Thr Pro Ala Gln Gln Ser Tyr Val Gln
        275                 280                 285

Ala Pro Thr Ala Thr Pro Ser Ser Thr Ala Gly Ser Ser Ser Ser Ser
    290                 295                 300

Gly Thr Leu Pro Ser Gly Ser Ser Leu Thr Glu Tyr Phe Asn Ser Leu
305                 310                 315                 320

Ser Ala Glu Glu Phe Leu Lys Val Leu Lys Glu Thr Phe Ser Trp Leu
                325                 330                 335

Val Thr Glu Lys Val His Ala Arg Asp Leu Ser Ala
            340                 345

<210>  58
<211>  381
<212>  PRT
<213>  Aspergillus kawachii

<400>  58

His Gly Tyr Val Ser Gly Ala Val Val Asp Gly Gln Tyr Tyr Gly Gly
1               5                   10                  15

Tyr Ile Val Ser Ser Tyr Ala Tyr Glu Ser Asp Pro Pro Glu Thr Ile
            20                  25                  30

Ala Trp Ser Thr Glu Ala Thr Asp Leu Gly Phe Val Asp Gly Ser Glu
        35                  40                  45

Tyr Ala Glu Pro Asp Ile Ile Cys His Lys Ser Ala Lys Pro Gly Ala
    50                  55                  60

Ile Ser Ala Asp Val Lys Ala Gly Gly Thr Val Glu Leu Gln Trp Thr
65                  70                  75                  80

Asp Trp Pro Ser Ser His His Gly Pro Val Leu Thr Tyr Leu Ala Asn
                85                  90                  95

Cys Asn Gly Asp Cys Ser Asp Val Thr Lys Thr Asp Leu Glu Phe Phe
            100                 105                 110

Lys Ile Asp Glu Ser Gly Leu Ile Ser Asp Thr Glu Val Pro Gly Thr
        115                 120                 125

Trp Ala Thr Asp Asn Leu Ile Ser Asn Asn Asn Ser Trp Thr Val Thr
    130                 135                 140

Ile Pro Ser Thr Leu Glu Ser Gly Asn Tyr Val Leu Arg His Glu Ile
145                 150                 155                 160

Ile Ala Leu His Ser Ala Gly Asn Lys Asp Gly Ala Gln Asn Tyr Pro
                165                 170                 175

Gln Cys Leu Asn Leu Lys Val Thr Gly Ser Gly Ser Ser Thr Tyr Ser
            180                 185                 190

Gly Thr Lys Gly Glu Ala Leu Tyr Lys Asp Thr Asp Pro Gly Ile Leu
        195                 200                 205

Val Asn Ile Tyr Gln Ser Leu Ser Ser Tyr Asp Ile Pro Gly Pro Ala
    210                 215                 220

Met Tyr Asn Ala Thr Ser Ser Gly Ser Ser Ser Gly Ser Ser Ser Ser
225                 230                 235                 240

Ala Ser Ser Thr Ser Ser Ala Ala Ala Ala Ala Ala Thr Thr Ser Ser
                245                 250                 255

Ala Ser Ser Ser Thr Thr Ser Ala Ala Ala Ala Thr Ser Ser Ala Val
            260                 265                 270

Ser Ala Thr Thr Thr Ser Ala Ala Ala Ala Ala Val Val Thr Asp Val
        275                 280                 285

Glu Thr Thr Val Gln Thr Ala Val Ala Thr Ala Tyr Gln Thr Ser Thr
    290                 295                 300

Asp Val Val Gln Val Thr Val Thr Gly Ser Ala Pro Gln Gln Thr His
305                 310                 315                 320

Ile Gln Val Ala Ser Ser Ser Ala Ala Ala Val Ala Thr Pro Ser Ser
                325                 330                 335

Ser Ser Val Ala Val Ser Ser Asp Asn Leu Thr Ser Tyr Phe Ser Ser
            340                 345                 350

Leu Ser Ala Asp Gln Phe Leu Ser Val Leu Lys Glu Thr Phe Ser Trp
        355                 360                 365

Leu Val Thr Glu Lys Lys His Ala Arg Asp Leu Asn Ala
    370                 375                 380

<210>  59
<211>  415
<212>  PRT
<213>  Aspergillus nidulans

<400>  59

His Gly Tyr Val Thr Gly Ile Val Ala Asp Gly Thr Tyr Tyr Gly Gly
1               5                   10                  15

Tyr Leu Val Asn Gln Tyr Pro Tyr Ser Asn Asp Pro Pro Ala Val Val
            20                  25                  30

Gly Trp Ala Glu Asp Ala Thr Asp Leu Gly Phe Val Asp Gly Ser Gly
        35                  40                  45

Tyr Thr Ser Gly Asp Ile Ile Cys His Lys Asp Ala Thr Asn Ala Gln
    50                  55                  60

Ala Ser Ala Thr Val Ala Ala Gly Gly Thr Val Glu Leu Gln Trp Thr
65                  70                  75                  80

Glu Trp Pro Glu Ser His His Gly Pro Val Ile Asp Tyr Ile Ala Ser
                85                  90                  95

Cys Asn Gly Asp Cys Thr Thr Val Asp Lys Thr Thr Leu Glu Trp Val
            100                 105                 110

Lys Ile Ser Glu Ser Gly Leu Val Asp Gly Ser Ser Ala Pro Gly Thr
        115                 120                 125

Trp Ala Ser Asp Asn Leu Ile Ser Asn Asn Asn Ser Trp Thr Val Thr
    130                 135                 140

Ile Pro Ser Ser Leu Ala Ala Gly Gly Tyr Val Leu Arg His Glu Ile
145                 150                 155                 160

Ile Ala Leu His Ser Ala Gly Asn Glu Asn Gly Ala Gln Asn Tyr Pro
                165                 170                 175

Gln Cys Val Asn Leu Glu Val Thr Gly Gly Gly Ser Ala Ser Pro Ser
            180                 185                 190

Gly Thr Val Gly Thr Glu Leu Tyr Thr Pro Thr Asp Pro Gly Ile Leu
        195                 200                 205

Val Asn Ile Tyr Thr Ser Leu Asp Ser Tyr Thr Ile Pro Gly Pro Ala
    210                 215                 220

Leu Trp Asp Gly Ala Ser Ser Ser Gly Gly Asn Ser Gly Ser Gly Ser
225                 230                 235                 240

Ala Ser Ser Ser Ala Ala Ala Thr Ser Thr Pro Thr Thr Pro Ser Val
                245                 250                 255

Ser Val Pro Val Ile Pro Thr Ala Ser Ser Gly Ala Ser Ser Thr Pro
            260                 265                 270

Leu Val Pro Thr Pro Ser Ala Pro Ala Val Thr Pro Ser Val Pro Ala
        275                 280                 285

Gly Asn Gln Ala Pro Gln Pro Thr Tyr Thr Ser Thr Tyr Ile Glu Thr
    290                 295                 300

Glu Thr Leu Pro Gly Gln Thr Val Thr Ser Thr Thr Thr Glu Tyr Ala
305                 310                 315                 320

Ser Glu Pro Thr Gln Pro Ala Val Glu Thr Gln Val Ala Gln Pro Ser
                325                 330                 335

Glu Thr Glu Ala Ala Thr Ser Thr Ser Thr Val Thr Glu Thr Ala Ser
            340                 345                 350

Ala Thr Ala Ala Pro Thr Gly Ser Ser Gly Ser Ser Ser Gly Ser Gly

355                 360                 365

Ser Ser Ser Thr Glu Leu Pro Thr Asp Ser Ser Ser Leu Ser Asp Tyr
    370                 375                 380

Phe Ser Ser Leu Ser Ala Glu Glu Phe Leu Asn Leu Leu Lys Glu Thr
385                 390                 395                 400

Leu Lys Trp Leu Val Thr Asp Lys Val His Ala Arg Ser Leu His
                405                 410                 415

<210>  60
<211>  229
<212>  PRT
<213>  Aspergillus oryzae

<400>  60

His Gly Tyr Val Ser Gly Val Val Ile Asp Gly Gln Tyr Tyr Gly Gly
1               5                   10                  15

Tyr Leu Val Asp Lys Tyr Ala Tyr Ser Asp Asn Ala Pro Asp Thr Ile
            20                  25                  30

Gly Trp Thr Thr Ser Ala Thr Asp Leu Gly Phe Val Asp Gly Thr Gly
        35                  40                  45

Tyr Gln Ser Pro Asp Ile Ile Cys His Lys Asp Gly Ala Pro Gly Ala
    50                  55                  60

Leu Thr Ala Glu Val Ala Ala Gly Gly Lys Ile Glu Leu Gln Trp Thr
65                  70                  75                  80

Glu Trp Pro Glu Ser His His Gly Pro Val Leu Asn Tyr Leu Ala Pro
                85                  90                  95

Cys Gly Gly Glu Cys Ser Ala Val Asp Lys Thr Thr Leu Glu Phe Phe
            100                 105                 110

Lys Ile Glu Ala Lys Gly Leu Ile Asp Gly Thr Thr Pro Pro Gly Gln
        115                 120                 125

Trp Ala Thr Asp Asp Leu Ile Ser Asn Asn Asn Ser Tyr Thr Val Thr
    130                 135                 140

Ile Pro Thr Ser Ile Gln Glu Gly Asn Tyr Val Leu Arg His Glu Ile
145                 150                 155                 160

Ile Gly Leu His Ser Ala Gly Gln Lys Asp Gly Ala Gln Asn Tyr Pro

165                 170                 175

Gln Cys Ile Asn Ile Lys Val Thr Gly Gly Gly Asp Ala Thr Pro Ala
            180                 185                 190

Gly Thr Ala Gly Glu Ala Leu Tyr Lys Asp Thr Asp Ala Gly Ile Leu
        195                 200                 205

Phe Asp Ile Tyr Ser Asp Leu Ser Gly Gly Tyr Pro Ile Pro Gly Pro
    210                 215                 220

Glu Val Phe Ser Ala
225

<210>  61
<211>  351
<212>  PRT
<213>  Aspergillus oryzae

<400>  61

His Gly Tyr Val Ser Gly Ala Val Val Asp Gly Gln Tyr Tyr Ser Gly
1               5                   10                  15

Tyr Asp Met Ser Tyr His Tyr Met Ser Asn Pro Pro Gln Val Ile Gly
            20                  25                  30

Trp Ser Thr Asp Ala Thr Asp Leu Gly Phe Val Asp Gly Ser Ser Tyr
        35                  40                  45

Ala Asp Ala Asp Ile Ile Cys His Lys Asn Ala Lys Asn Gly Ala Ile
    50                  55                  60

Ser Ala Glu Ile Ala Ala Gly Lys Gln Val Glu Leu Gln Trp Thr Ala
65                  70                  75                  80

Trp Pro Glu Ser His Lys Gly Pro Val Ile Thr Tyr Leu Ala Asn Cys
                85                  90                  95

Asn Gly Asp Cys Ala Thr Val Asp Lys Thr Gln Leu Glu Phe Phe Lys
            100                 105                 110

Ile Asp Glu Lys Gly Leu Ile Ser Gly Ser Asp Asn Thr Trp Ala Ser
        115                 120                 125

Asp Asn Leu Ile Ser Ser Asn Asn Ser Trp Thr Val Thr Ile Pro Ser
    130                 135                 140

Ser Ile Ala Ala Gly Asn Tyr Val Met Arg His Glu Ile Ile Ala Leu

145                 150                 155                 160

His Ser Ala Gly Asn Lys Asp Gly Ala Gln Asn Tyr Pro Gln Cys Leu
                165                 170                 175

Asn Phe Lys Val Thr Gly Gly Gly Ser Asp Lys Pro Glu Gly Thr Leu
            180                 185                 190

Gly Thr Ala Leu Tyr Lys Asp Thr Asp Pro Gly Ile Glu Ile Asn Ile
        195                 200                 205

Tyr Gln Thr Leu Ser Ser Tyr Thr Ile Pro Gly Pro Ala Leu Tyr Thr
    210                 215                 220

Gly Ser Ser Ser Ser Gly Ser Ser Gly Ser Gly Ser Ser Ser Ala Ala
225                 230                 235                 240

Pro Ser Pro Thr Ala Ser Ala Ser Ala Ser Ala Thr Ala Ala Pro Val
                245                 250                 255

Gln Thr Ser Thr Ala Thr Ala Tyr Gln Thr Ser Thr Ala Val Ala Ser
            260                 265                 270

Val Thr Val Thr Gly Ser Ala Pro Ala Gln Thr His Val Gln Ala Thr
        275                 280                 285

Ser Ser Ser Ala Ala Ala Ser Thr Pro Thr Ala Ser Ser Gly Ala Ser
    290                 295                 300

Thr Gly Ser Gly Ser Gly Ser Ser Ser Ser Asp Leu Thr Ser Tyr Phe
305                 310                 315                 320

Asn Ser Leu Ser Ala Asp Glu Leu Leu Asn Val Ile Lys Gln Thr Leu
                325                 330                 335

Ser Trp Leu Val Thr Asp Lys Ile His Ala Arg Asp Ile Ser Ala
            340                 345                 350

<210>  62
<211>  359
<212>  PRT
<213>  Baudoinia compniacensis

<400>  62

His Gly His Val Gln Gly Ile Val Ala Gly Gly Thr Tyr Tyr Thr Gly
1               5                   10                  15

Tyr Asp Pro Ser Phe Gln Tyr Ser Pro Ile Pro Pro Ile Val Ile Gly

20                  25                  30

Trp Ser Asp Pro Gln Asp Leu Asp Asn Gly Phe Ile Ala Pro Ser Asn
        35                  40                  45

Tyr Thr Asn Pro Asp Ile Ile Cys His Arg Asn Ala Thr Ala Ala Gly
    50                  55                  60

Thr Ser Ala Arg Ile Ala Ala Gly Asp Val Val Glu Pro Gln Trp Thr
65                  70                  75                  80

Pro Trp Pro Ser Ser His His Gly Pro Val Ile Asp Tyr Leu Ala Lys
                85                  90                  95

Cys Pro Gly Ser Cys Ser Asp Val Asp Lys Thr Thr Leu Glu Phe Phe
            100                 105                 110

Lys Ile Asp Gln Val Gly Leu Ile Asp Asp Thr Thr Glu Pro Gly Thr
        115                 120                 125

Trp Gly Ser Asp Gln Leu Leu Ala Asn Asn Asn Ser Trp Thr Val Thr
    130                 135                 140

Ile Pro Pro Ser Ile Ala Pro Gly Asn Tyr Val Leu Arg His Glu Ile
145                 150                 155                 160

Ile Ala Leu His Ser Ala Gly Thr Ala Asp Gly Ala Gln Asn Tyr Pro
                165                 170                 175

Gln Cys Val Asn Leu Glu Ile Thr Gly Ser Gly Thr Asp Thr Pro Ala
            180                 185                 190

Gly Thr Leu Gly Gln Lys Leu Tyr Gly Ala Asn Asp Ser Gly Ile Leu
        195                 200                 205

Ile Asn Ile Tyr Gln Ser Leu Ser Thr Tyr Glu Ile Pro Gly Pro Ser
    210                 215                 220

Leu Tyr Ser Gly Ala Val Ala Met Ser Gln Thr Gln Val Ala Ile Thr
225                 230                 235                 240

Gly Tyr Ala Pro Leu Ser Ser Gly Gly Val Ala Val Ser Ile Ala Tyr
                245                 250                 255

Ser Ser Ala Thr Thr Thr Thr Ala Ala Ala Gly Ser Ser Ser Val Ala
            260                 265                 270

Arg Ser Thr Thr Ala Ala Ser Ala Ser Ala Ser Thr Ala Ile Val Ser
        275                 280                 285

Ala Leu Pro Ser Thr Ala Leu Thr Gln Thr Leu Pro Thr Val Leu Pro
    290                 295                 300

Thr Gly Thr Ala Val Ser Ser Asn Asp Thr Ile Ala Gly Leu Pro Pro
305                 310                 315                 320

Ala Glu Ala Pro Pro Ala Gly Thr Thr Leu Gln Asp Leu Phe Ala Trp
                325                 330                 335

Leu Glu Tyr Met Leu Glu Gly Leu Ala Ser Thr Gly Gly Arg His Gln
            340                 345                 350

Arg Asp Phe Lys Glu Arg Lys
        355

<210>  63
<211>  227
<212>  PRT
<213>  Penicillium roqueforti

<400>  63

His Gly Tyr Val Ser Ser Ile Val Ala Asp Gly Lys Asn Tyr Thr Gly
1               5                   10                  15

Tyr Ile Val Asp Lys Tyr Pro Tyr Met Ala Asp Pro Pro Asp Ser Val
            20                  25                  30

Gly Trp Ala Thr Thr Ala Thr Asp Leu Gly Phe Glu Asp Gly Thr Glu
        35                  40                  45

Tyr Gln Asp Pro Asn Ile Ile Cys His Arg Asn Gly Val Asn Ala Ala
    50                  55                  60

Leu Ser Ala Thr Val Thr Ala Gly Ser Ser Ile Asp Leu Gln Trp Thr
65                  70                  75                  80

Thr Trp Pro Asp Ser His His Gly Pro Leu Ile Thr Tyr Leu Ala Ser
                85                  90                  95

Cys Asn Gly Asp Cys Ser Thr Val Asp Lys Thr Thr Leu Lys Phe Phe
            100                 105                 110

Lys Ile Glu Glu Asp Gly Leu Ile Asp Gly Ala Thr Pro Pro Gly Thr
        115                 120                 125

Trp Ala Thr Asp Asp Leu Ile Ala Ala Gly Gly Gln Trp Thr Val Lys
    130                 135                 140

Ile Pro Pro Thr Leu Ala Ala Gly Asn Tyr Val Met Arg His Glu Ile
145                 150                 155                 160

Ile Ala Leu His Ser Ala Gly Gln Leu Asn Gly Ala Gln Asn Tyr Pro
                165                 170                 175

Gln Cys Phe Asn Leu Glu Ile Thr Gly Gly Gly Ser Asp Val Pro Asp
            180                 185                 190

Gly Thr Phe Gly Gly Glu Leu Tyr Ile Asn Thr Asp Pro Gly Ile Leu
        195                 200                 205

Phe Asp Ile Tyr Ser Thr Leu Thr Ser Tyr Ile Ile Pro Gly Pro Ala
    210                 215                 220

Leu Tyr Ala
225

<210>  64
<211>  300
<212>  PRT
<213>  Sclerotinia sclerotiorum

<400>  64

His Gly Tyr Val Thr Gly Ile Val Ala Gly Gly Thr Tyr Thr Lys Gly
1               5                   10                  15

Trp Leu Val Asn Tyr Ala Tyr Gln Asn Pro Met Pro Glu Ser Ile Gly
            20                  25                  30

Trp Ser Asp Gln Asn Leu Asp Leu Gly Phe Thr Ala Pro Ser Ala Tyr
        35                  40                  45

Ala Thr Gly Asp Ile Ile Cys Ala Lys Asn Gly Thr Asn Ala Ala Leu
    50                  55                  60

Ser Ala Lys Val Ala Ala Gly Asp Thr Val Asp Phe Gln Trp Thr Val
65                  70                  75                  80

Trp Pro Asp Ser His Lys Gly Pro Val Ile Thr Tyr Leu Ala Asn Cys
                85                  90                  95

Asn Gly Asp Cys Ser Thr Val Asp Lys Thr Lys Leu Glu Phe Phe Lys
            100                 105                 110

Ile Asp Glu Gly Gly Leu Ile Asp Asp Thr Thr Val Pro Gly Thr Trp
        115                 120                 125

Ala Ser Asp Asn Leu Ile Thr Asn Asn Asn Thr Trp Thr Ser Thr Ile
    130                 135                 140

Pro Ala Asp Leu Ala Pro Gly Asn Tyr Val Ala Arg His Glu Ile Ile
145                 150                 155                 160

Ala Leu His Gly Ala Gly Ser Asn Asp Gly Ala Gln Asn Tyr Pro Gln
                165                 170                 175

Cys Ile Asn Leu Glu Val Thr Gly Ser Gly Thr Leu Ala Pro Ser Gly
            180                 185                 190

Thr Leu Gly Glu Lys Leu Tyr Thr Pro Thr Asp Pro Gly Ile Leu Val
        195                 200                 205

Asn Ile Tyr Ala Lys Leu Thr Tyr Thr Ile Pro Gly Pro Pro Met Tyr
    210                 215                 220

Asn Ala Thr Gly Ala Ala Thr Gly Ala Ala Thr Gly Ala Thr Thr Pro
225                 230                 235                 240

Ala Thr Gly Lys Thr Ala Ser Ala Thr Ser Ala Ala Ala Ala Leu Ser
                245                 250                 255

Thr Pro Ala Val Val Ser Thr Pro Pro Thr Pro Thr Thr Met Ile Thr
            260                 265                 270

Ser Val Ala Pro Ala Ala Thr Pro Ala Thr Ser Ala Ala Ala Ser Ser
        275                 280                 285

Gly Ala Ser Gly Asp Asp Asp Asp Thr Cys Glu Ala
    290                 295                 300

<210>  65
<211>  385
<212>  PRT
<213>  Penicillium roqueforti

<400>  65

His Gly Phe Val Ser Gly Ala Val Val Asp Gly Thr Tyr His Gly Gly
1               5                   10                  15

Phe Ile Val Asn Thr Tyr Asn Tyr Met Thr Glu Val Pro Ala Asn Ile
            20                  25                  30

Gly Trp Ser Glu Lys Ala Thr Asp Leu Gly Tyr Ile Asp Gly Ser Gly
        35                  40                  45

Tyr Thr Gly Ser Asp Ile Ile Cys His Lys Glu Ala Thr Pro Gly Ala
    50                  55                  60

Ile Ser Ala Glu Val Lys Ala Gly Gly Ser Val Glu Leu Gln Trp Thr
65                  70                  75                  80

Asp Trp Pro Glu Ser His His Gly Pro Val Ile Thr Tyr Met Ala Asn
                85                  90                  95

Cys Asn Gly Asp Cys Ser Val Val Asp Lys Thr Thr Leu Lys Phe Phe
            100                 105                 110

Lys Ile Ala Glu Ala Gly Leu Ile Asp Asp Thr Asn Val Pro Gly Thr
        115                 120                 125

Trp Ala Ser Asp Gln Leu Ile Ala Ala Asn Asn Ser Ala Thr Val Thr
    130                 135                 140

Ile Pro Ser Thr Ile Ala Ala Gly Asn Tyr Val Leu Arg His Glu Ile
145                 150                 155                 160

Ile Ala Leu His Ser Ala Gly Asp Ala Asn Gly Ala Gln Asn Tyr Pro
                165                 170                 175

Gln Cys Ile Asn Leu Lys Val Thr Gly Gly Gly Ser Asp Ser Pro Asp
            180                 185                 190

Gly Val Leu Gly Thr Ala Leu Tyr Thr Pro Thr Asp Pro Gly Ile Leu
        195                 200                 205

Ile Ser Ile Tyr Ser Ala Leu Glu Ser Tyr Val Ile Pro Gly Pro Ala
    210                 215                 220

Leu Tyr Thr Gly Ala Ser Ser Gly Ser Ser Ser Thr Ser Ser Thr Thr
225                 230                 235                 240

Ala Ala Ala Thr Thr Glu Ala Thr Thr Ala Ser Ala Thr Val Ser Thr
                245                 250                 255

Thr Ala Ala Pro Thr Val Ala Thr Thr Ser Ala Ser Thr Thr Ala Ser
            260                 265                 270

Thr Ala Asp Ser Ile Thr Ile Thr Thr Thr Ala Gly Gly Leu Ser Ala
        275                 280                 285

Gln Ser Ser Ala Ala Val Leu Glu Ser Ser Ala Thr Ser Thr Val Thr
    290                 295                 300

Ala Glu Ala Thr Ala Thr Ala Ala Pro Thr Ala Ala Ala Thr Thr Ser
305                 310                 315                 320

Thr Ser Ser Thr Ser Ser Gly Ser Ser Ser Ser Ser Ser Thr Ser Ser
                325                 330                 335

Ser Gly Ser Ser Ser Thr Ser Ser Thr Ser Thr Ser Asn Ser Ser Tyr
            340                 345                 350

Leu Ser Ser Leu Ser Ala Glu Lys Leu Leu Glu Val Ile Arg Ser Thr
        355                 360                 365

Leu Lys Trp Leu Val Ser Asp Lys Lys Val His Ala Arg Ala Leu Ala
    370                 375                 380

Tyr
385

<210>  66
<211>  227
<212>  PRT
<213>  Penicillium italicum

<400>  66

His Gly Tyr Val Ser Ser Ile Val Ala Asn Gly Lys Asn Tyr Thr Gly
1               5                   10                  15

Tyr Ile Val Asp Lys Tyr Ala Tyr Met Ser Asp Pro Pro Asp Ser Val
            20                  25                  30

Gly Trp Ala Thr Thr Ala Thr Asp Leu Gly Phe Glu Asp Gly Thr Glu
        35                  40                  45

Tyr Gln Asn Pro Asn Ile Ile Cys His Arg Gly Gly Val Asn Ala Ala
    50                  55                  60

Leu Ser Ala Thr Val Ala Ala Gly Ser Ser Ile Asp Ile Gln Trp Thr
65                  70                  75                  80

Thr Trp Pro Asp Ser His His Gly Pro Leu Ile Thr Tyr Leu Ala Ser
                85                  90                  95

Cys Asn Gly Asp Cys Ser Thr Val Asp Lys Thr Thr Leu Lys Phe Phe
            100                 105                 110

Lys Ile Glu Glu Asp Gly Leu Ile His Gly Ala Thr Pro Pro Gly Thr
        115                 120                 125

Trp Ala Thr Asp Asp Leu Ile Ala Ser Gly Gly Gln Trp Thr Val Arg
    130                 135                 140

Ile Pro Ser Thr Ile Ala Thr Gly Asn Tyr Val Met Arg His Glu Ile
145                 150                 155                 160

Ile Ala Leu His Ser Ala Gly Gln Leu Asn Gly Ala Gln Asn Tyr Pro
                165                 170                 175

Gln Cys Phe Asn Leu Gln Val Thr Gly Gly Gly Gly Asn Ala Pro Asn
            180                 185                 190

Gly Thr Leu Gly Glu Lys Leu Tyr Thr Asn Thr Asp Pro Gly Ile Leu
        195                 200                 205

Phe Asn Ile Tyr Ser Thr Leu Thr Ser Tyr Val Ile Pro Gly Pro Ala
    210                 215                 220

Leu Tyr Ala
225

<210>  67
<211>  352
<212>  PRT
<213>  Aspergillus flavus

<400>  67

Gly His Val Ser Gly Ala Val Val Asp Gly Gln Tyr Tyr Pro Gly Tyr
1               5                   10                  15

Asp Ile Ser Tyr His Tyr Met Pro Asp Pro Pro Lys Val Ile Gly Trp
            20                  25                  30

Ser Thr Asp Ala Thr Asp Asn Gly Phe Val Asp Gly Ser Ser Tyr Ala
        35                  40                  45

Asp Ala Asp Ile Ile Cys His Lys Asn Ala Lys Asn Gly Ala Ile Ser
    50                  55                  60

Ala Glu Ile Ala Ala Gly Lys Gln Val Glu Leu Gln Trp Thr Ala Trp
65                  70                  75                  80

Pro Glu Ser His Lys Gly Pro Val Ile Thr Tyr Leu Ala Asn Cys Asn
                85                  90                  95

Gly Asp Cys Ala Thr Val Asp Lys Thr Gln Leu Glu Phe Phe Lys Ile
            100                 105                 110

Asp Glu Lys Gly Leu Ile Ser Gly Ser Asp Asn Thr Trp Ala Thr Asp
        115                 120                 125

Asn Leu Ile Ala Ser Asn Asn Ser Trp Thr Val Thr Ile Pro Ser Ser
    130                 135                 140

Ile Ala Ala Gly Asn Tyr Val Met Arg His Glu Ile Ile Gly Leu His
145                 150                 155                 160

Ser Ala Gly Gln Lys Asp Gly Ala Gln Asn Tyr Pro Gln Cys Leu Asn
                165                 170                 175

Phe Lys Val Thr Gly Gly Gly Ser Asp Lys Pro Glu Gly Thr Leu Gly
            180                 185                 190

Thr Ala Leu Tyr Lys Asp Thr Asp Pro Gly Ile Gln Ile Asn Ile Tyr
        195                 200                 205

Gln Thr Leu Thr Ser Tyr Thr Ile Pro Gly Pro Ala Leu Tyr Ser Gly
    210                 215                 220

Ser Ser Ser Ser Gly Ser Ser Gly Ser Gly Ser Ser Ser Ala Ala Pro
225                 230                 235                 240

Ser Ala Thr Ala Ser Ala Ser Ala Ser Ala Ser Ala Thr Ala Ala Pro
                245                 250                 255

Val Gln Thr Ser Thr Ala Thr Ala Tyr Gln Thr Ser Thr Ala Val Ala
            260                 265                 270

Ser Val Thr Val Thr Gly Ser Ala Pro Ala Gln Thr His Val Gln Ala
        275                 280                 285

Thr Ser Ser Ser Ala Ala Ala Ser Thr Pro Thr Ala Ser Ser Gly Ala
    290                 295                 300

Ser Thr Gly Ser Gly Ser Gly Ser Ser Ser Ser Asp Leu Thr Gly Tyr
305                 310                 315                 320

Phe Asn Ser Leu Ser Ala Asp Glu Leu Leu Asn Val Ile Lys Gln Thr
                325                 330                 335

Leu Ser Trp Leu Val Thr Asp Lys Ile His Ala Arg Asp Ile Ser Ala
            340                 345                 350

<210>  68
<211>  227
<212>  PRT
<213>  Penicillium crysogenum

<400>  68
   His Gly Tyr Val Ser Ser Ile Val Ala Asp Gly Lys Ser Tyr Thr Gly
   1 5 10 15
   Tyr Leu Val Asp Lys Tyr Ala Tyr Met Ala Asp Pro Pro Asp Ser Val
   20 25 30
   Gly Trp Thr Thr Thr Ala Thr Asp Leu Gly Phe Glu Asp Gly Thr Glu
   35 40 45
   Tyr Gln Asp Pro Asn Ile Ile Cys His Arg Asp Gly Ala Asn Ala Ala
   50 55 60
   Leu Ser Ala Thr Val Lys Ala Gly Ser Ser Ile Asp Leu Gln Trp Thr
   65 70 75 80
   Thr Trp Pro Asp Ser His His Gly Pro Val Ile Thr Tyr Leu Ala Ser
   85 90 95
   Cys Asn Gly Asp Cys Thr Thr Val Asp Lys Thr Thr Leu Glu Phe Phe
   100 105 110
   Lys Ile Glu Glu Glu Gly Leu Ile Asp Gly Ala Asn Pro Pro Gly Thr
   115 120 125
   Trp Ala Thr Asp Asp Leu Ile Ala Ala Gly Gly Lys Trp Thr Val Lys
   130 135 140
   Ile Pro Ser Thr Ile Ala Pro Gly Asn Tyr Val Met Arg His Glu Ile
   145 150 155 160
   Ile Ala Leu His Ser Ala Gly Gln Thr Asn Gly Ala Gln Asn Tyr Pro
   165 170 175
   Gln Cys Phe Asn Leu Glu Val Thr Gly Gly Gly Ser Asp Thr Pro Asp
   180 185 190
   Gly Thr Pro Gly Met Glu Leu Tyr Thr Asn Thr Asp Pro Gly Ile Leu
   195 200 205
   Phe Asp Ile Tyr Ser Ser Leu Thr Ser Tyr Asp Ile Pro Gly Pro Ala
   210 215 220
   Leu Tyr Ala
   225

<210> 69
<211> 227
<212> PRT
<213> Penicillium expansum

<400> 69

His Gly Tyr Val Ser Ser Ile Val Ala Asn Gly Lys Asn Tyr Thr Gly
1               5                   10                  15

Tyr Ile Val Asp Lys Tyr Ala Tyr Met Ser Asp Pro Pro Asp Ser Val
            20                  25                  30

Gly Trp Ala Thr Thr Ala Thr Asp Leu Gly Phe Glu Asp Gly Thr Glu
        35                  40                  45

Tyr Gln Asp Pro Asn Ile Ile Cys His Arg Gly Gly Ile Asn Ala Ala
    50                  55                  60

Leu Ser Ala Pro Val Thr Ala Gly Ser Ser Ile Asp Ile Gln Trp Thr
65                  70                  75                  80

Thr Trp Pro Asp Ser His His Gly Pro Leu Val Thr Tyr Leu Ala Ser
                85                  90                  95

Cys Asn Gly Asp Cys Ser Thr Val Asp Lys Thr Thr Leu Lys Phe Phe
            100                 105                 110

Lys Ile Glu Glu Asp Gly Leu Ile Asp Gly Thr Thr Pro Pro Gly Thr
        115                 120                 125

Trp Ala Thr Asp Asp Leu Ile Ala Ala Gly Gly Gln Trp Thr Val Arg
    130                 135                 140

Ile Pro Ser Thr Ile Ala Ala Gly Asn Tyr Val Met Arg His Glu Ile
145                 150                 155                 160

Ile Ala Leu His Ser Ala Gly Gln Ser Asn Gly Ala Gln Asn Tyr Pro
                165                 170                 175

Gln Cys Phe Asn Leu Gln Val Thr Gly Gly Gly Ser Asp Val Pro Asp
            180                 185                 190

Gly Thr Leu Gly Glu Lys Leu Tyr Thr Asn Thr Asp Pro Gly Ile Leu
        195                 200                 205

Phe Asn Leu Tyr Ser Thr Leu Thr Ser Tyr Ile Ile Pro Gly Pro Ala
    210                 215                 220

Leu Tyr Ala
225

<210>  70
<211>  374
<212>  PRT
<213>  Penicillium rubens

<400>  70

His Gly Phe Val Ser Gly Ala Val Val Asp Gly Thr Tyr His Gly Gly
1               5                   10                  15

Tyr Leu Val Asn Asn Tyr Pro Tyr Asn Asp Asn His Pro Glu Thr Ile
            20                  25                  30

Gly Trp Ala Glu Lys Ala Thr Asp Leu Gly Phe Val Asp Gly Ser Gly
        35                  40                  45

Tyr Ser Gly Pro Asp Ile Ile Cys His Lys Glu Ala Thr Pro Gly Ala
    50                  55                  60

Ile Ser Ala Glu Val Lys Ala Gly Gly Asp Val Glu Leu Gln Trp Thr
65                  70                  75                  80

Glu Trp Pro Glu Ser His His Gly Pro Val Ile Asn Tyr Leu Ala Asn
                85                  90                  95

Cys Asn Gly Asp Cys Ser Lys Val Asp Lys Lys Thr Leu Lys Trp Phe
            100                 105                 110

Lys Ile Ala Glu Ser Gly Leu Ile Asp Gly Ser Asn Ala Pro Gly Lys
        115                 120                 125

Trp Ala Ser Asp Glu Leu Ile Ala Asn Asn Asn Ser Ala Ser Val Thr
    130                 135                 140

Ile Pro Ser Ser Ile Ala Ala Gly Asn Tyr Val Leu Arg His Glu Ile
145                 150                 155                 160

Ile Ala Leu His Ser Ala Gly Gln Glu Asn Gly Ala Gln Asn Tyr Pro
                165                 170                 175

Gln Cys Leu Asn Leu Lys Val Thr Gly Gly Gly Ser Asp Val Pro Glu
            180                 185                 190

Gly Val Val Gly Thr Glu Leu Tyr Lys Pro Asp Asp Ala Gly Ile Leu
        195                 200                 205

Val Ser Ile Tyr Asn Gln Leu Lys Asp Tyr Thr Ile Pro Gly Pro Ala
    210                 215                 220

Leu Tyr Lys Gly Ala Ser Ser Gly Ser Gly Ser Lys Thr Thr Asp Ala
225                 230                 235                 240

Ala Ser Thr Ala Thr Thr Ala Ser Ala Ser Thr Val Ser Ala Ser Pro
                245                 250                 255

Ile Gln Ser Ser Ser Ser His His Leu Thr Arg Thr Arg Thr Ala Arg
            260                 265                 270

Pro Thr Tyr Ser Pro Ser Gly Thr Pro Thr Pro Ser Ser Ser Ala Val
        275                 280                 285

Ser Ala Ala Ser Thr Ser Thr Pro Ile Gly Ser Val Pro Ala Ser Gly
    290                 295                 300

Gly Glu Ala Ser Glu Pro Ser Ala Thr Pro Ala Pro Ser Ser Ser Gly
305                 310                 315                 320

Ser Ser Ser Gly Ser Ser Ser Ser Ser Gly Ser Ser Ser Glu Ser Gly
                325                 330                 335

Asp Tyr Ala Ser Tyr Leu Ser Ser Leu Ser Ala Glu Lys Leu Leu Glu
            340                 345                 350

Val Ile Arg Ser Thr Leu Lys Trp Leu Val Ser Asp Asn Lys Val His
        355                 360                 365

Ala Arg Ala Leu Ala His
    370

## Description

The invention relates to the field of bioproducts, more particularly to the biotechnological processes for biofuel production. Specifically, the invention relates to enzymes with polysaccharide monooxygenase activity and their use, as part of enzymatic cocktails, for the production of fermentable sugars by hydrolysis of cellulosic biomass during processes for the production of bioproducts such as bioethanol.

### PRIOR STATE OF THE ART

Biofuels are an attractive alternative to fossil fuels and can be obtained by fermenting monomeric sugars derived from starch or cellulose and hemicellulose.

Plant biomass provides an abundant source of potential energy in the form of carbohydrates that can be used for numerous industrial and agricultural processes and, therefore, is an important and renewable source for generating fermentable sugars. Fermentation of these sugars can produce valuable commercial end products, such as ethanol also known as bioethanol.

Although fermentation of sugars to ethanol is relatively straightforward, efficient conversion of cellulosic biomass to fermentable sugars, such as glucose, is more challenging. The huge potential energy of carbohydrates in plant biomass is not sufficiently used because the sugars form part of complex polymers (polysaccharides such as cellulose and hemicellulose) and, therefore, are not easily accessible for fermentation. Thus, cellulose can be pre-treated, mechanically, chemically, enzymatically or in other ways, to increase its susceptibility to hydrolysis. After this pre-treatment process, occurs a saccharification or hydrolysis stage consisting of an enzymatic process in which complex carbohydrates (such as starch or cellulose) are hydrolysed into their monosaccharide components. The goal of any saccharification technology therefore is to alter or eliminate structural and compositional impediments in order to improve the rate of enzymatic hydrolysis and increase the yields of fermentable sugars obtained from cellulose and hemicellulose (N. Mosier et al., 2005, Bioresource Technology 96, 673-686). After this stage of saccharification, a fermentation process takes place. Therefore, the higher the amount of complex sugars that remain at the end of the hydrolytic process, the lower the yield in ethanol production at the end of the fermentation process. Thus, an area of research directed at reducing costs and improving the yield of biofuel production processes is focused on improving the technical efficiency of hydrolytic enzymes, or generally on improving the efficiency of enzymatic cocktails used to generate, by hydrolysis, fermentable sugars from biomass.

It has been shown that individual enzymes are only able to partially digesting cellulose and hemicellulose and, therefore, the combined action of different classes of enzymes is required to complete their conversion into monomeric sugars. Many more enzymes are required for digesting hemicellulose to monomeric sugars than cellulose, including enzymes with xylanase, beta-xylosidase, arabinofuranosidase, mannanase, galactosidase, and glucuronidase activity. Other enzymes without glycosyl hydrolase activity, such as acetyl xylan esterase and ferulic acid esterase, may also be involved. Therefore, the enzymatic hydrolysis of polysaccharides for conversion into soluble sugars and, finally, into monomers such as xylose, glucose and other pentoses and hexoses, is catalysed by several enzymes which are collectively called "cellulases". Cellulases comprise at least three main activities, endo-β-glucanase (EC 3.2.1.4), exo-β-glucanase or cellobiohydrolase (EC 3.2.1.9.1) and β-glucosidase (EC 3.2.1.21), and it has been shown that they act synergistically in cellulose hydrolysis (Woodward, J. 1991, Bioresource Technology Vol. 36, pg. 67-75).

Microbial cellulases, particularly those of fungal origin, have become important biocatalysts due to their extensive industrial applications (Kuhad R. C. et al., 2011, Enzyme Research, Article ID 280696). Nowadays, considerable attention has been paid to research on cellulases and the challenges in their production, especially for the improvement of the economy of the process for their industrial application, in order to obtain cells with greater activity and better properties.

On the other hand, the glycosyl-hydrolase proteins of the family 61 (GH61) have been known for more than 20 years. The first GH61 described, called CEL1, was isolated from *Agaricus bisporus* in 1992 (Raguz et al., 1992, Gene 119: 183-190). These GH61 proteins are accessory proteins that contribute to the cellulose degradation. The fact that these enzymes act by direct oxidation of cellulose, rather than by hydrolysis, has led to their current name: Cu dependent polysaccharide monooxygenases (polysaccharide monooxygenases; PMOs). Compared to other cellulolytic enzymes, PMOs are relatively small proteins with typical molecular masses between 20 and 50 kDa (Baldrian and Valaskova 2008, FEMS Microbiology Reviews 32: 501-521; Harris et al, 2010, Biochemistry 49: 3305-3316). These proteins require an oxygen molecule to break the substrate by oxidation. One of the two atoms ends up forming a water molecule, and with the other one the direct oxidation of the substrate is performed. Therefore, the members of this enzyme family act as Cu monooxygenases that catalyse cellulose breakdown by an oxidative mechanism, releasing cellodextrins (Langston et al., 2011, Applied and Environmental Microbiology 77: 7007-7015). The action of PMOs is also described, for example, in Glyn R. Hemsworth, et al., 2014, Nat Chem Biol. 10(2): 122-126, in Lucia Zifcakova, Petr Baldrian, 2012, Fungal ecology 5: 481-189 and in many patent documents where new PMOs have been identified in filamentous fungi, as in P201430155.

The hydrolytic efficiency of a multi-enzymatic complex in the saccharification process of cellulosic material depends on the properties of the individual enzymes present in the complex. Therefore, in the context of biofuel production processes, it is necessary to design enzymatic cocktails with improved individual activities whose use during the saccharification or hydrolysis stage of cellulosic biomass leads to an improvement in the yield of this stage through an increase in the amount of fermentable sugars obtained. Then, these sugars can be fermented to produce biofuels, such as bioethanol, so the use of these improved enzymatic cocktails would ultimately increase the efficiency and profitability of the whole biofuel production process.

### DESCRIPTION OF THE INVENTION

The present invention relates polypeptides with polysaccharide monooxygenase activity (PMO, also known as glycosyl-hydrolase of the family 61 or GH61) which have been identified, isolated and characterised from different microorganisms. As the examples of the present invention show, these polypeptides have the advantage that they are able to increasing the saccharification yield of cellulosic biomass, by increasing the amount of fermentable sugars (mainly glucose) produced at the end of this hydrolytic process, when they are added to the enzymatic cocktails normally used in biofuel production processes.

These polypeptides are polysaccharide monooxygenase enzymes that intervene in the initial stages of the decomposition process from cellulosic biomass to fermentable sugars, these enzymes being responsible for improving the accessibility of the rest of the enzyme machinery to the substrate. In this way, they increase the yield of the hydrolysis process by increasing the amount of simple sugars obtained (mainly glucose) and thus, ultimately, the yield of ethanol production from biomass.

Specifically, the inventors of the present invention have identified, in the genome of different fungi, 22 genes that encode enzymes with PMO activity. The amino acid sequences of these 22 mature enzymes are shown in the SEQ ID NO: 49 to SEQ ID NO:SEQ ID NO: 70. These polypeptides have been isolated from *Byssochlamys spectabilis, Penicillium oxalicum* 114-2, *Aspergillus clavatus, Aspergillus fumigatus* Af293, *Aspergillus niger* CBS 513.88, *Aspergillus niger* ATCC 1015, *Aspergillus ruber* CBS 135680, *Aspergillus terreus* NIH2624, *Neosartorya fischeri* NRRL181, *Aspergillus kawachii* IFO 4308, *Aspergillus nidulans* FGSC A4, *Aspergillus oryzae* RIB40, *Baudoinia compniacensis* UAMH 10762, *Penicillium roqueforti* FM164, *Sclerotinia sclerotiorum, Penicillium italicum, Aspergillus flavus, Penicillium crysogenum Wisconsin, Penicillium expansum* and *Penicillium rubens Wisconsin.* These polypeptides have been characterised and expressed recombinantly in a host cell, preferably in the C1 strain of *Myceliophthora thermophila,* which secretes them to the extracellular medium together with an enzymatic mixture comprising the main cellulolytic enzymes. Thus, the effect of these PMOs of the invention on the yield of these cellulolytic enzymatic cocktails in the saccharification of cellulosic biomass, preferably pre-treated and more preferably on PCS or *pre-treated corn stover,* has been evaluated. The results have shown an increase in the concentration of fermentable sugars (mainly glucose) released in the hydrolytic process compared to an enzymatic cocktail that did not comprise the polypeptides with PMO active of the invention.

Thus, this invention demonstrates the improvement in the enzymatic hydrolysis of cellulosic biomass with a cellulolytic cocktail produced by a host cell, preferably *M.* *thermophila,* modified to recombinantly express at least one of the polypeptides with PMO activity of the invention.

Improving the yield of enzymatic mixtures used to produce fermentable sugars from cellulosic material in biofuel production processes, preferably ethanol, is essential to ensure the profitability of these processes. For this reason, it is important to find new and improved enzymes capable of increasing the efficiency (production yield of fermentable sugars during hydrolysis) of the enzymatic cocktails in which they are included. The supplementation of an enzymatic cocktail comprising cellulolytic enzymes with these PMOs provided by the invention contributes, therefore, to improving the yield of the hydrolytic process where these cocktails are used, in particular by increasing the yield of glucose release from biomass.

Therefore, a first aspect of the present invention relates to an isolated polypeptide, preferably with polysaccharide monooxygenase activity, hereinafter referred to as the "polypeptide of the invention" or the "PMO of the invention", comprising an amino acid sequence having at least 80% identity with an amino acid sequence selected from SEQ ID NO:SEQ ID NO: 49 to SEQ ID NO:SEQ ID NO: 70, preferably selected from SEQ ID NO: 52, SEQ ID NO: 53, SEQ ID NO: 59, SEQ ID NO: 60 or SEQ ID NO: 65; more preferably selected from SEQ ID NO: 52, SEQ ID NO: 59 or SEQ ID NO: 65; even more preferably with SEQ ID NO: 65.

The polypeptide of the invention may be isolated from its natural source (microorganism producer) or environment (extracellular medium), preferably after its natural expression in the fungi indicated later in this description or from a natural or growing environment where such fungi are present, or it may be recombinantly produced. The polypeptide of the present invention and its variants or derivatives can be synthesized, for example, but not be limited to, *in vitro.* For example, by solid-phase peptide synthesis or recombinant DNA approximations. The polypeptide of the invention can be produced recombinantly, not only directly but also as a fusion polypeptide linked to another peptide or polypeptide which can be, but not be limited to, heterologous (with different origin than the polypeptide of the invention). This other peptide or polypeptide fused to the polypeptide of the invention may comprise or consist of, for example but not be limited to, a signal sequence (signal peptide) or another polypeptide which has for example a sequence to facilitate its expression and/or purification or a protease cleavage site, for example but not be limited to, at the N-terminal end of the mature protein.

The polypeptide of the invention may have variants. These variants refer to limited variations in the amino acid sequence, which allow the maintenance of the functionality of the polypeptide. This means that the reference sequence and the variant sequence are similar as a whole, and identical in many regions. These variations are generated by substitutions, deletions or additions. Such substitutions are preferably by conserved amino acids. Conserved amino acids are amino acids that have side chains and similar properties in terms of, for example, hydrophobicity or aromaticity. These substitutions include, but are not limited to, substitutions between glutamic acid (Glu) and aspartic acid (Asp), between lysine (Lys) and arginine (Arg), between asparagine (Asn) and glutamine (Gin), between serine (Ser) and threonine (Thr), and/or between the amino acids that compose the group alanine (Ala), leucine (Leu), valine (Val) and isoleucine (Ile). Variations can be artificially generated variations such as mutagenesis or direct synthesis. These variations do not lead to essential changes in the essential characteristics or properties of the polypeptide. Therefore, within the extent of this invention are also included peptides or polypeptides whose sequence of amino acids is identical or homologous to the sequences described in this invention.

The terms "polysaccharide monooxygenase", "PMO", "Glycosylhydrolase 61 family" or "GH61" refer to an enzyme with GH61 or PMO activity, which when included in a saccharification reaction (for example, one in which endoglucanase, beta-glucosidases and celobiohydrolases are used), results in a higher amount (higher yield) of one or more soluble sugars (e.g. glucose) compared to the saccharification reaction carried out under the same conditions but in the absence of the GH61 protein. PMO activity can be determined by, for example, indirect oxidative assays that colorimetrically evidence the electron transfer phenomenon using different electron donor and acceptor compounds (Kitt et al., 2012, Biotechnology for Biofuels Vol. 5:79, pg. 1-13). On the other hand, PMO activity on biomass could be measured, for example, by combining the polypeptide to be tested with cellulose or cellulolytic enzymes in a saccharification reaction and determining whether there is an increase in glucose yield compared to the same saccharification reaction performed in the presence of the same enzymes and the same conditions but in the absence of the polypeptide to be tested.

In this invention, the PMOs comprising the SEQ ID NO: 49 to SEQ ID NO: 70 have been obtained from the following fungi:
- SEQ ID NO: 49, *Byssochlamys spectabilis.*
- SEQ ID NO: 50, *Penicillium oxalicum* 114-2.
- SEQ ID NO: 51, *Aspergillus clavatus.*
- SEQ ID NO: 52, *Aspergillus fumigatus* Af293.
- SEQ ID NO: 53, *Aspergillus niger* CBS 513.88.
- SEQ ID NO: 54, *Aspergillus niger* ATCC 1015.
- SEQ ID NO: 55, *Aspergillus ruber* CBS135680.
- SEQ ID NO: 56, *Aspergillus terreus* NIH2624.
- SEQ ID NO: 57, *Neosartorya fischeri* NRRL181.
- SEQ ID NO: 58, *Aspergillus kawachii* IFO 4308.
- SEQ ID NO: 59, *Aspergillus nidulans* FGSC A4.
- SEQ ID NO: 60, *Aspergillus oryzae* RIB40.
- SEQ ID NO: 61, *Aspergillus oryzae* RIB40.
- SEQ ID NO: 62, *Baudoinia compniacensis* UAMH 10762.
- SEQ ID NO: 63, *Penicillium roqueforti* FM164.
- SEQ ID NO: 64, *Sclerotinia sclerotiorum.*
- SEQ ID NO: 65, *Penicillium roqueforti* FM164.
- SEQ ID NO: 66, *Penicillium italicum.*
- SEQ ID NO: 67, *Aspergillus flavus.*
- SEQ ID NO: 68, *Penicillium crysogenum Wisconsin.*
- SEQ ID NO: 69, *Penicillium expansum.*
- SEQ ID NO: 70, *Penicillium rubens Wisconsin.*

In a more preferred embodiment, the polypeptide of the invention comprises an amino acid sequence having at least 81%, 82%, 83%, 84%, 85%, 86%, 87%, 88%, 89%, 90%, 91%, 92%, 93%, 94%, 95%, 96%, 97%, 98% or 99% identity with an amino acid sequence selected from SEQ ID NO: 49 to SEQ ID NO: 70, preferably selected from SEQ ID NO: 52, SEQ ID NO: 53, SEQ ID NO: 59, SEQ ID NO: 60 or SEQ ID NO: 65; more preferably selected from SEQ ID NO: 52, SEQ ID NO: 59 or SEQ ID NO: 65; even more preferably with SEQ ID NO: 65. In an even more preferred embodiment, the polypeptide of the invention comprises an amino acid sequence that has at least 90% identity with an amino acid sequence selected from SEQ ID NO: 49 to SEQ ID NO: 70, preferably selected from SEQ ID NO: 52, SEQ ID NO: 53, SEQ ID NO: 59, SEQ ID NO: 60 or SEQ ID NO: 65; more preferably selected from SEQ ID NO: 52, SEQ ID NO: 59 or SEQ ID NO: 65; even more preferably with SEQ ID NO: 65.

The term "identity", "homology" or "percentage of identity" refers to the ratio of residues of nucleic acids or amino acids that are identical between two sequences of nucleic acids or amino acids being compared. Preferably, "identity" refers to the ratio of residues of nucleic acids or amino acids that are identical between two nucleic acid or amino acid sequences, with respect to a length of at least 100 residues, preferably with respect to the full length of the reference sequence. The degree of identity can be determined by the Clustal method, the Wilbur-Lipman method, the GAG program, which includes GAP, BLAST or BLASTN, EMBOSS Needle and FASTA. In addition, the Smith Waterman algorithm can be used to determine the degree of identity between two sequences.

For sequence comparison, typically one of the sequences acts as a reference sequence with which the "problem" sequences are compared. When a sequence comparison algorithm is used to determine its identity, the reference sequence and the problem sequence(s) are entered into the program, and the program parameters are configured. The program parameters that appear by default can be used or configured; preferably these parameters will be those that appear by default. Thus, the sequence comparison algorithm calculates the percentage of identity between the problem sequence(s) and the reference sequence based on program parameters. Two examples of algorithms that are useful for determining percentage of sequence identity are BLAST and BLAST 2.0, described in Altschul et al. (1997) Nucleic Acids Res 25(17):3389-3402 and Altschul et al. (1990) J. Mol Biol 215(3)-403-410, respectively. Preferably, the degree of identity to which this invention refers is calculated by BLAST. The software for conducting the BLAST analysis is publicly available at the *National Center for Biotechnology Information* (NCBI).

In a more preferred embodiment, the polypeptide of the invention comprises a sequence of amino acids selected from SEQ ID NO: 49 to SEQ ID NO: 70, preferably selected from SEQ ID NO: 52, SEQ ID NO: 53, SEQ ID NO: 59, SEQ ID NO: 60 or SEQ ID NO: 65; more preferably selected from SEQ ID NO: 52, SEQ ID NO: 59 or SEQ ID NO: 65; even more preferably comprising the SEQ ID NO: 65.

The polypeptides of the invention include those that may comprise a signal peptide linked to its N-terminal end. This signal peptide can be the one that is naturally present in a PMO or another from another protein (heterologous). Examples of heterologous signal peptides that could be linked to the PMO polypeptides of the invention are, but not be limited to, the signal peptide of *Aspergillus niger* glucoamylase, preferably of sequence SEQ ID NO: 46, and the PMO-06230 peptide signal from *M. thermophila,* preferably from sequence SEQ ID NO: 48. More preferably, the signal peptide with SEQ ID NO: 46 is encoded by the nucleotide sequence SEQ ID NO: 45 and the signal peptide with SEQ ID NO: 48 is encoded by the nucleotide sequence SEQ ID NO: 47.

When the polypeptide of the invention is linked to a signal peptide, said polypeptide corresponds to the pre-protein or immature polypeptide of the mature PMO enzyme. Thus, both mature PMO polypeptides (where there is no signal peptide) and these immature PMO pre-proteins or polypeptides that comprising a signal peptide located at the N-terminal end of the amino acid sequence of the mature enzyme, are within the extent of this invention. These pre-proteins are preferably the SEQ ID NO: 23 to SEQ ID NO: 44. The signal peptide of each of these sequences SEQ ID NO: 23 to SEQ ID NO: 44 is shown in the respective polypeptide sequences in the sequence listing.

In an even more preferred embodiment, the polypeptide of the invention consists of an amino acid sequence selected from SEQ ID NO: 49 to SEQ ID NO: 70, preferably selected from SEQ ID NO: 52, SEQ ID NO: 53, SEQ ID NO: 59, SEQ ID NO: 60 or SEQ ID NO: 65; more preferably selected from SEQ ID NO: 52, SEQ ID NO: 59 or SEQ ID NO: 65; even more preferably it consists of the SEQ ID NO: 65. These sequences correspond to the mature polypeptides of the invention.

In another preferred embodiment, the polypeptide of the invention consists of an amino acid sequence selected from SEQ ID NO: 23 to SEQ ID NO: 44, preferably selected from SEQ ID NO: 26, SEQ ID NO: 27, SEQ ID NO: 33, SEQ ID NO: 34 or SEQ ID NO: 39; more preferably selected from SEQ ID NO: 26, SEQ ID NO: 33 or SEQ ID NO: 39; even more preferably it consists of the SEQ ID NO: 39. These sequences correspond to the immature polypeptides of the invention (comprising the native or naturally present signal peptide in the enzyme).

In another preferred embodiment, the polypeptide of the invention further comprises, linked to its N-terminal end, a signal peptide. Preferably such a signal peptide is selected from the list consisting of: the native signal peptide or that originally present in the enzyme naturally (SPWT, whose amino acid sequence is indicated in the listing of sequences in each polypeptide of the invention of SEQ ID NO: 23 to SEQ ID NO: 44), the glucoamylase A signal peptide of *A. niger* (SPGA, preferably of SEQ ID NO: 46) or the PMO-06230 peptide signal from *M. thermophila* (SPPMO, preferably from SEQ ID NO: 48). In a more preferred embodiment, the polypeptide of the invention is linked, to its N-terminal end, to the SPGA signal peptide, more preferably with SEQ ID NO: 46.

As explained above, the term "pre-protein" refers to a polypeptide that includes a signal peptide (or leading sequence) at its terminal amino end. This signal peptide is cleaved from the pre-protein by a peptidase, thus secreting the mature protein. The secreted portion of the polypeptide is called "mature protein" or "secreted protein". The "signal peptide" is the one that directs the polypeptide inside the cell towards its secretion pathway.

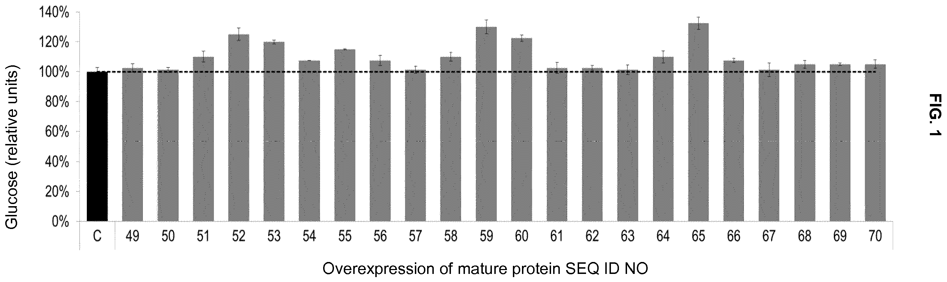

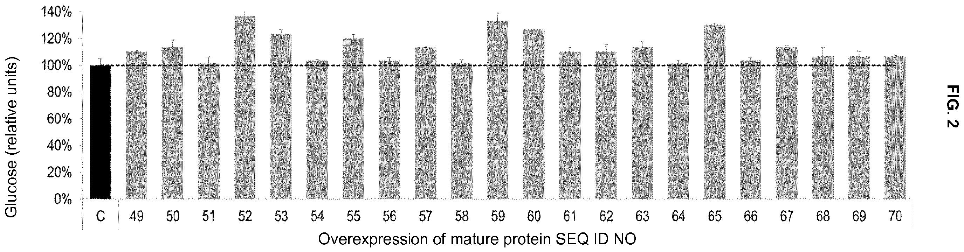

As the examples of this invention show, the polypeptides referred to here as SEQ ID NO: 52, SEQ ID NO: 53, SEQ ID NO: 59, SEQ ID NO: 60 and SEQ ID NO: 65 were those which were provided the largest increases in saccharification capacity when were added as a supplement to enzymatic mixtures or cocktails produced by *M. thermophila* (see Figures 1 and 2). Therefore, in a more preferred embodiment, the polypeptide of the invention consists of an amino acid sequence selected from SEQ ID NO: 52, SEQ ID NO: 53, SEQ ID NO: 59, SEQ ID NO: 60 or SEQ ID NO: 65; more preferably selected from SEQ ID NO: 52, SEQ ID NO: 59 or SEQ ID NO: 65; even more preferably it consists of the SEQ ID NO: 65.

According to the examples shown below, the polypeptides of the invention provided increases in saccharification capacity (measured as relative units of released glucose) following the order set out below from highest to lowest:
SEQ ID NO: 65
SEQ ID NO: 59
SEQ ID NO: 52
SEQ ID NO: 60
SEQ ID NO: 53
SEQ ID NO: 55
SEQ ID NO: 51
SEQ ID NO: 58
SEQ ID NO: 64
SEQ ID NO: 54
SEQ ID NO: 56
SEQ ID NO: 66
SEQ ID NO: 68
SEQ ID NO: 69
SEQ ID NO: 70
SEQ ID NO: 49
SEQ ID NO: 61
SEQ ID NO: 62
SEQ ID NO: 50
SEQ ID NO: 57
SEQ ID NO: 63
SEQ ID NO: 67

Therefore, the above list shows the order of preference of the polypeptides of the invention.

In a particular embodiment, the polypeptide of the invention comprises an amino acid sequence having at least 80% identity, preferably at least 90% identity, with the SEQ ID NO: 65 and is linked, to its N-terminal end, to the SPGA signal peptide, more preferably to SEQ ID NO: 46.

The term "increase" as used in the present invention refers to an increase in the yield of a reaction product, for example, from a fermentable sugar, preferably glucose, produced when a particular component present during the reaction (such as a PMO polypeptide of the invention) causes a greater production of the product compared to a reaction performed under the same conditions and with the same substrate but in the absence of the component in question.

Due to the degeneration of the genetic code, in which several nucleotide triplets give rise to the same amino acid, there are several nucleotide sequences that give rise to the same amino acid sequence. For this reason, another aspect of the invention refers to an isolated polynucleotide, hereinafter "polynucleotide of the invention", which encodes any of the polypeptides of the invention. Another aspect of the invention refers to an isolated polynucleotide comprising a nucleotide sequence complementary to the polynucleotide of the invention, or hybridizing under astringent conditions, preferably under conditions of high astringency, with the polynucleotide of the invention.

The terms "nucleotide sequence", "nucleic acid", "oligonucleotide" and "polynucleotide" are used interchangeably herein and refer to a polymeric form of nucleotides of any length that may or may not be, chemically or biochemically modified. Therefore, they refer to any polyribonucleotide or polydesoxyribonucleotide, whether single-chain or double-stranded. The polynucleotide of the invention, therefore, can be DNA, RNA, or derivatives of both DNA and RNA, including cDNA. The polynucleotide of the invention can be obtained artificially by conventional cloning and selection methods, or by sequencing. The polynucleotide, in addition to the coding sequence, may comprise other elements, for example but not limited to, one or more introns, non-coding sequences at the 5' and/or 3' ends, ribosome binding sites, coding sequences for a signal peptide, or stabilising sequences. These polynucleotides may additionally include coding sequences for additional amino acids that may be useful, for example, but not limited, to increase the stability of the peptide generated from it or to allow better purification of the peptide.

The nucleic acid sequences encoding the polypeptides of the invention, for example, can be designed on the basis of the amino acid sequences provided in the present invention. The nucleotide coding sequences for the immature PMO polypeptides described in the present invention (SEQ ID NO: 23 to SEQ ID NO: 44) consist, preferably, of SEQ ID NO: 1 to SEQ ID NO: 22. The nucleotide sequence coding for the signal peptide in each of these immature PMOs is shown in the respective sequences SEQ ID NO: 1 to SEQ ID NO: 22 of the sequence listing.

The polynucleotide of the invention can be introduced into a gene construct, for example, into a cloning vector or expression vector, to allow its replication or expression. Preferably, said vector is an appropriate vector for the expression and purification of the polypeptide of the invention. For this reason, another aspect of the invention refers to a gene construct that comprises:
- the polynucleotide of the invention that encodes any of the polypeptides of the invention, or
- a combination of those polynucleotides of the invention.

Hereinafter, this gene construct will be referred to as "gene construct of the invention" or "construct of the invention".

The term "gene construct", as used herein, refers to a nucleic acid molecule, both monocatenary and bicatenary, that is isolated and modified to contain nucleic acid segments in a way that could not exist in nature. The term "nucleic acid construct" or "gene construct" is synonymous with the term "expression cassette" when the nucleic acid construct contains the control sequences required for the expression of the polynucleotide of the invention. Therefore, the genetic construct of the invention may also comprise one or more control or regulatory sequences of gene expression, such as, but not limited to, promoter sequences, leader sequences, terminator sequences of transcription, polyadenylation sequences, signal sequences, regulators, enhancers, etc.
The term "control sequences" includes all components that are necessary or advantageous for the expression of the polynucleotide of this invention in a cell. Each control sequence may be of the same or different origin as the polynucleotide of the invention. Such control sequences include, but are not limited to, a leader sequence, a booster or regulator sequence, a polyadenylation sequence, a pro-peptide sequence, a promoter, a coding sequence for a signal peptide, and a transcription terminator sequence. At a minimum, control sequences include a sequence of the signal peptide, and more preferably also a promoter, and termination signals from the transcription and translation. Control sequences with linkers may also be provided in order to introduce specific restriction sites that facilitate the binding of the control sequences to the coding region of the polypeptide of the invention. Appropriate control sequences for the expression of a polynucleotide in eukaryotic cells are known in the state of the art.

As used herein, the term "promoter" refers to a nucleotide sequence, usually "upstream" of the transcription starting point, that is capable of initiating transcription in a cell. This term includes, but is not limited to, constitutive promoters, specific cellular promoters, ubiquitous promoters, and inducible or repressible promoters. In general, control sequences depend on the origin of the host cell into which the gene construct is to be inserted.

In a preferred embodiment, the gene construct of the invention is an expression vector. An "expression vector" is a linear or circular DNA molecule comprising at least one polynucleotide of the invention operationally linked to additional nucleotides provided for its expression. This vector that comprising the polynucleotide of the invention can be introduced into a host cell in such a way that the vector is maintained as a chromosomal component or as an autoreplicating extracromosomal vector.

The term "operatively linked" refers to a configuration in which a control sequence is located in an appropriate position with respect to the coding sequence of the polynucleotide of the invention, such that the control sequence directs the expression of said polynucleotide. When creating the expression vector, the coding sequence is located in the vector in such a way that it is operatively linked to the appropriate control sequences for its expression. Therefore, the expression vectors referred to in the present invention comprise the polynucleotide of the invention, a promoter, and transcription and translation termination signals. The various nucleic acids and control sequences described herein may link together to produce a recombinant expression vector which may also include one or more suitable restriction sites to allow insertion or substitution of the polynucleotide encoding for the polypeptide of the invention at such sites.

The expression vector referred to in the present invention may be any vector (e.g. plasmid or virus) that can be conveniently subjected to a recombinant DNA procedure and can produce the expression of the polynucleotide of the invention. The choice of vector will normally depend on the compatibility of the vector with the host cell into which the vector is to be introduced. The expression vector can be, for example but not be limited to, a plasmid, a cosmid, a phage, a virus or viral vector, an artificial bacterial chromosome (BAC), an artificial yeast chromosome (YAC), or similar.

Vectors can be linear or closed circular. The vector can be an autonomous replicating vector, in other words, a vector that exists as an extracromosomal entity, whose replication is independent of chromosomal replication, for example, a plasmid, an extracromosomal element, a mini chromosome, or an artificial chromosome. The vector can contain any means to ensure self-replication. Alternatively, the vector may be of the type that, when introduced into the host cell, it integrates into the genome and replicates along with the chromosome(s) in which it has been integrated. In addition, a single vector or plasmid or two or more vectors or plasmids together containing the total DNA to be introduced into the host cell genome, or a transposon, may be used.

The vectors used in this invention contain, preferably, one or more selectable markers that allow an easy selection of transformed, transfected, transduced, or similar cells. A selectable marker is a gene whose product provides a distinguishable signal or effect, such as but not limited to, luminescence, resistance to biocides or virus, resistance to heavy metals, prototrophy to auxotrophs and similar. Markers selectable for use in a host filamentous fungus cell include, but are not limited to, amdS (acetamidase), *argB* (ornithine carbamoyltransferase), bar (phosphinothricin acetyltransferase), *hph* (higromicin phosphotransferase), *niaD* (nitrate reductase), *pyrG* (orotidine-5'-phosphate decarboxylase), *pyr5, pyr4, cysC* (sulphate adenyltransferase), and *trpC* (anthranylate synthase), as well as their equivalents.

The vectors referred to in the present invention preferably contain an element(s) allowing the integration of the vector into the genome of the host cell or the autonomous replication of the vector into the cell independently of the genome. For integration into the host cell genome, the vector may be based on the polynucleotide sequence of the invention or on any other vector element for integration into the genome by homologous or non-homologous recombination. Alternatively, the vector may contain additional nucleotide sequences to direct integration by homologous recombination into the host cell genome at a precise location(s) of the chromosome(s).

For autonomous replication, the vector may comprise a replication origin that allows it to replicate autonomously in the host cell in question. The replication origin can be any plasmid replicator that mediates autonomous replication that works in a cell. The term "replication origin" or "plasmid replicator" is defined herein as a nucleotide sequence that allows a plasmid or vector to replicate *in vivo.* Examples of useful replication origins in a filamentous fungus cell are AMA1 and ANS1.

A single polynucleotide of the invention or several polynucleotides of the invention may be inserted jointly into the host cell by introducing one or more expression vectors. Similarly, both one and more than one copy of the same polynucleotide of this invention can be inserted into the host cell to increase the production of the gene product(s). An increase in the number of copies of the polynucleotide can be obtained by integrating at least one additional copy of the sequence into the host cell genome and by including with the polynucleotide an amplifiable selectable marker gene, where cells containing amplified copies of the selectable marker gene, and thus additional copies of the polynucleotide, can be selected by culturing the cells in the presence of the appropriate selection agent.

The procedures used to link the elements described above to construct the recombinant expression vectors referred to in this invention are well known to technical experts.

The gene construct of the invention may be introduced into a host cell competent to carry out the expression of one or more of the polypeptides of the invention. Thus, another aspect of the invention refers a host cell which comprises, in a recombinant manner (i.e. unnatural or by human intervention), one or more of the polynucleotides of the invention or the gene construct of the invention, hereinafter referred to as the "host cell of the invention". In other words, the host cell of the invention has been transformed, transfected, transduced or similar, with at least one polynucleotide of the invention or with the gene construct of the invention. Preferably, the host cell of the invention is not a *Penicillium roqueforti* cell, and more preferably not a *Byssochlamys spectabilis, Penicillium oxalicum, Aspergillus clavatus, Aspergillus fumigatus, Aspergillus niger, Aspergillus ruber, Aspergillus terreus, Neosartorya fischeri, Aspergillus kawachii, Aspergillus nidulans, Aspergillus oryzae, Baudoinia compniacensis, Penicillium roqueforti, Sclerotinia sclerotiorum, Penicillium italicum, Aspergillus flavus, Penicillium crysogenum, Penicillium expansum* or *Penicillium rubens* cell. Preferably, the host cell of the invention comprises, in a recombinant manner (i.e. introduced by human intervention), one or more of the polynucleotides of the invention selected from the list consisting of the SEQ ID NO: 1 to SEQ ID NO: 22, preferably one or more of the polynucleotides of SEQ ID NO: 4, SEQ ID NO: 5, SEQ ID NO: 11, SEQ ID NO: 12 or SEQ ID NO: 17; more preferably from SEQ ID NO: 4, SEQ ID NO: 11 or SEQ ID NO: 17; even more preferably from SEQ ID NO: 17.

In another preferred embodiment, the polynucleotide of the invention encodes for a polypeptide of the invention comprising a sequence of amino acids presenting at least 80% identity, more preferably at least 90% identity, with the SEQ ID NO: 52, SEQ ID NO: 53, SEQ ID NO: 59, SEQ ID NO: 60 or SEQ ID NO: 65; more preferably with SEQ ID NO: 52, SEQ ID NO: 59 or SEQ ID NO: 65; even more preferably with SEQ ID NO: 65.

In another preferred embodiment, the gene construct of the invention comprises at least one polynucleotide of the invention coding for a polypeptide of the invention comprising a sequence of amino acids presenting at least 80% identity, more preferably at least 90% identity, with the SEQ ID NO: 52, SEQ ID NO: 53, SEQ ID NO: 59, SEQ ID NO: 60 or SEQ ID NO: 65; more preferably with SEQ ID NO: 52, SEQ ID NO: 59 or SEQ ID NO: 65; even more preferably with SEQ ID NO: 65.

In another preferred embodiment, the cell of the invention comprises at least one polynucleotide of the invention, or a gene construct of the invention comprising it, coding for a polypeptide of the invention comprising a sequence of amino acids presenting at least 80% identity, more preferably at least 90% identity, with the SEQ ID NO: 52, SEQ ID NO: 53, SEQ ID NO: 59, SEQ ID NO: 60 or SEQ ID NO: 65; more preferably with SEQ ID NO: 52, SEQ ID NO: 59 or SEQ ID NO: 65; even more preferably with SEQ ID NO: 65.

The "host cell", as used here, includes any type of cell that is susceptible to transformation, transfection, transduction, and the like, with the polynucleotide of the invention or with the gene construct of the invention. The host cell may be eukaryotic, such as a mammalian cell, insect, plant, or fungus. In a preferred embodiment, the host cell is a filamentous fungus cell. Filamentous fungi are generally characterised by a mycelial wall composed of chitin, cellulose, glucan, chitosan, mannan, and other complex polysaccharides. In a more preferred embodiment, the host cell of the filamentous fungus is a *Acremonium, Aspergillus, Aureobasidium, Bjerkandera, Ceriporiopsis, Coprinus, Coriolus, Cryptococcus, Filibasidium, Fusarium, Gibberella, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes,* or *Trichoderma* cell. In a more preferred embodiment, the host cell of the filamentous fungus is an *Aspergillus awamori, Aspergillus foetidus* or *Aspergillus japonicus* cell. In another more preferred embodiment, the host cell of the filamentous fungus is a *Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium pseudograminearum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides,* or *Fusarium venenatum* cell. In another more preferred embodiment, the host cell of the filamentous fungus is a *Bjerkandera adusta, Ceriporiopsis aneirina, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Coprinus cinereus, Coriolus hirsutus, Gibberella zeae, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Thielavia terrestris, Trametes villosa, Trametes versicolor, Trichoderma harzianum, Trichoderma koningii, Trichoderma longibrachiatum, Trichoderma reesei,* or *Trichoderma viride* cell. In an even more preferred embodiment, the host cell of the invention is any strain of the species *Myceliophthora thermophila.* In an even more preferred embodiment, the host cell of the invention is the C1 strain of *Myceliophthora thermophila.*

It shall be understood that for the above species, the invention covers the perfect and imperfect states, and other taxonomic equivalents, e.g. anamorphs, with respect to the name of the species through which they are known. Technical experts will easily recognise the identity of suitable equivalents. For example, *Myceliophthora thermophila* is equivalent to *Chrysosporium lucknowense.*

The host cell of the invention comprises, therefore, at least one polynucleotide of the recombinantly introduced invention, preferably by means of the gene construct of the invention. The term "recombinantly introduced" refers to the fact that the polynucleotide of the invention or the gene construct of the invention is not naturally present in that cell, but has been intentionally introduced through genetic engineering procedures. These polynucleotides can encode the mature polypeptide or a pre-protein consisting of a signal peptide linked to the mature enzyme that will have to be processed later in order to produce the mature PMO enzyme.

The host cell of the invention expresses, and preferably also secretes to the extracellular medium, at least one of the PMO polypeptides of the invention, or any combination thereof, so that they are functional. The term "functional" refers that the expressed enzyme(s) retains its ability to oxidise cellulose, i.e., retains its polysaccharide monooxygenase activity. This activity can be measured by any appropriate procedure known in the state of the art to evaluate the activity of PMO.

The term "expression" includes any stage involved in the production of the polypeptide of the invention for example, but not limited to, transcription, post-transcriptional modification, translation, post-translational modification, and secretion.

The expression of the polypeptide of the invention in the host cell of the invention may be induced by any procedure known in the technique, such as the transformation of a suitable host cell with at least one polynucleotide of the invention or with the gene construct of the invention, and the culture of the transformed host cell under conditions that induce the expression of that polynucleotide in order to obtain the secreted and functional enzyme.

The host cell of the invention can be cultivated in a suitable nutrient medium, whether solid or liquid, for the production of the PMO polypeptides of the invention, using well known procedures in the technique. For example, the cell can be cultured by flask culture with agitation, and small-scale or large-scale fermentation (including continuous, batch or discontinuous, fed-batch, or solid-state fermentation) carried out in a laboratory or industrial bioreactor in a suitable medium and under conditions that allow PMO to be expressed and/or isolated. Cultivation takes place in a suitable nutrient medium comprising, for example, sources of carbon and nitrogen and inorganic salts, using the procedures known in the technique. Once the PMO has been secreted into the nutrient medium, it can be recovered directly from the medium.

Expressed PMO can be detected using procedures known in the technique specific to polypeptides. These detection procedures may include the use of specific antibodies, monitoring the formation of an enzyme product, or monitoring the disappearance of an enzyme substrate.

The resulting PMO can be retrieved from the medium using procedures known in the technique. For example, PMO can be recovered from the nutrient medium by conventional procedures including, but not limited to, centrifugation, filtration, extraction, spray drying, evaporation, or precipitation.

The PMOs produced in this invention can be purified by a variety of procedures known in the technique including, but not limited to, chromatography (e.g., ion exchange, affinity, hydrophobia, chromatofocus, and size exclusion), electrophoretic procedures (e.g. preparative isoelectric focusing), differential solubility (e.g. ammonium sulphate precipitation), SDS-PAGE, or extraction, in order to obtain a substantially pure PMO that can be included in an enzymatic composition together with other cellulolytic enzymes.

The host cell of the invention expresses at least one of the polypeptides of the invention, preferably one or more polypeptides comprising or consisting of the SEQ ID NO: 52, SEQ ID NO: 53, SEQ ID NO: 59, SEQ ID NO: 60 or SEQ ID NO: 65; more preferably one or more polypeptides comprising or consisting of the SEQ ID NO: 52, SEQ ID NO: 59 or SEQ ID NO: 65; even more preferably a polypeptide comprising or consisting of the SEQ ID NO: 65, or any combination of them, or all of them. The cell may also express one or more other cellulolytic, native or recombinant enzymes.

Another aspect of the invention concerns an enzymatic composition or enzymatic cocktail comprising at least one of the polypeptides of the invention, hereinafter referred to as the "composition of the invention". Preferably, the composition of the invention comprises one or more polypeptides which comprise or consist of a polypeptide selected from the list consisting of the SEQ ID NO: 49 to SEQ ID NO: 70, preferably selected from SEQ ID NO: 52, SEQ ID NO: 53, SEQ ID NO: 59, SEQ ID NO: 60 or SEQ ID NO: 65; more preferably selected from SEQ ID NO: 52, SEQ ID NO: 59 or SEQ ID NO: 65; even more preferably the polypeptide from SEQ ID NO: 65.

"Enzymatic composition" or "enzymatic cocktail" means a mixture comprising at least two enzymes capable of hydrolysing, oxidising, degrading or similar, sugars.

This composition of the invention may further comprises other enzymatic activities, such as aminopeptidase, amylase, carbohydrase, carboxypeptidase, catalase, cellulase, such as endoglucanase, beta-glucosidase and/or celobiohydrolase; chitinase, cutinase, cyclodextrin glucosyltransferase, deoxyribonuclease, esterase, alpha-galactosidase, beta-galactosidase, glucoamylase, alpha-glucosidase, haloperoxidase, invertase, lacase, lipase, manosidase, oxidase, reductase, pectinolytic enzyme, peptidoglutaminase, peroxidase, phytase, polyphenoloxidase, protease, ribonuclease, transglutaminase, or xylanase, or any combination thereof. The additional enzyme(s) may be produced recombinantly or naturally, for example, by a microorganism belonging to the genus *Aspergillus,* such as *Aspergillus aculeatus, Aspergillus awamori, Aspergillus fumigatus, Aspergillus foetidus, Aspergillus japonicus, Aspergillus nidulans, Aspergillus niger,* or *Aspergillus oryzae; Fusarium,* such as *Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium pseudograminearum, Fusarium reticulatum, Fusarium roseum, Fusarium sambucinum, Fusarium sarcochroum, Fusarium sulphureum, Fusarium toruloseum, Fusarium trichothecioides,* or *Fusarium venenatum; Gibberella,* such as *Gibberella zeae; Humicola,* such as *Humicola insolens* or *Humicola lanuginosa; Trichoderma,* such as *Trichoderma harzianum, Trichoderma koningii, Trichoderma longibrachiatum, Trichoderma reesei,* or *Trichoderma viride;* or *Myceliophthora,* such as *Myceliophthora thermophila.*

In a preferred embodiment, the composition of the invention also comprises another (one or more) cellulolytic enzyme. The term "cellulolytic enzymes" also known as "cellulases" refers to a class of enzymes capable of hydrolysing cellulose (links from β-1,4-glucan or β-D-glucosidics) or hemicellulose to shorter oligosaccharides, cellobiose and/or glucose. Examples of cellulolytic enzymes include, but are not limited to, endoglucanases, beta-glucosidases, celobiohydrolases, polysaccharide monooxygenases (other than those described in the present invention), beta-xylosidases, endoxyloglucanases or endoxylanases. Thus, in a more preferred embodiment, these cellulolytic enzymes are selected from the list consisting of endoglucanases, beta-glucosidases, celobiohydrolases, celobiose dehydrogenases, polysaccharide monooxygenases, beta-xylosidases, alpha-xylosidases, endoxylanases, endoxyl glucanases, or any of their combinations. These cellulolytic enzymes may derive from the host cell of the invention or from other microorganisms producing cellulolytic enzymes other than the host cell of the invention, such as those indicated above. They can also be produced naturally or recombinantly.

In a preferred embodiment, the composition of the invention comprises all the PMO polypeptides of the invention, more preferably mature (without signal peptides), even more preferably of sequences SEQ ID NO: 49 to SEQ ID NO: 70.

Preferably, the composition of the invention comprises at least one polypeptide of the invention and other cellulolytic enzymes derived from the host cell of the invention. In an even more preferred embodiment, the composition of the invention is an enzymatic mixture expressed (secreted or obtained) by the host cell of the invention. In an even more preferred embodiment, the composition of the invention is an enzymatic mixture obtained by culture of the host cell of the invention, more preferably of the strain *M. thermophila* C1. This enzyme mixture expressed by the host cell of the invention shall comprise all or part of the cellulolytic enzymes produced naturally or recombinantly and secreted by that cell. These cellulolytic enzymes include, for example but not limited to, endoglucanases, beta-glucosidases, arabinofuranosidases, celobiohydrolases, celobiose dehydrogenases, xylidases, xylanases, etc.

The term "endoglucanase" or "EG" refers to a group of cellulase enzymes classified as E.C. 3.2.1.4. These enzymes hydrolyse the β-1,4 internal glycosidic links of cellulose.

The term "cellobiohydrolase" refers to a protein that catalyses the hydrolysis of cellulose to cellobiose by exoglucanase activity, sequentially releasing cellobiose molecules from the reducing or non-reducing ends of cellulose or celooligosaccharides.

The term "beta-glucosidase", as used herein, refers to an enzyme that catalyses the hydrolysis of a sugar dimer, including, but not limited to cellobiose, with the release of a corresponding sugar monomer, used, but not limited, for ethanol synthesis. The enzyme beta-glucosidase acts on the bridges β1->4 that bind two glucose molecules or substituted glucose (i.e. The disaccharide, cellobiose). It is an exocellulase with specificity for a variety of beta-D-glucoside substrates. It catalyses the hydrolysis of non-terminal reducing residues in beta-D-glucosides with glucose release.

The term "endoxylanase" refers to an enzyme that catalyses the endohydrolysis of 1.4-beta-D-xylosidic bonds in xylans.

The term "beta-xylosidase" refers to a protein that hydrolyses short 1.4-β-D-xyloligomers to xylose.

The term "endoxyloglucanase" refers to an enzyme specific to xyloglucan, capable of catalysing the solubilisation of xyloglucan in oligosaccharides but not showing substantial cellulolytic activity.

The term "cellobiose dehydrogenase" refers to an enzyme that catalyses a chemical reaction in which the substrates are cellobiose and the acceptor and the products are celobion-1.5-lactone and the reduced acceptor.

The term "alpha-xylosidase" refers to an enzyme that catalyses the hydrolysis of alpha-xylose residues that are not terminal reducers in alpha-xylosides.

In another preferred embodiment, the composition of the invention comprises at least one of the polypeptides of the invention and a cellulolytic mixture consisting of: endoglucanase, beta-glucosidase, celobiohydrolase I and celobiohydrolase II. More preferably, these cellulolytic enzymes come from *M. thermophila.*

In a more preferred embodiment, the composition of the invention also includes Cu.

The composition of the invention can be prepared according to known procedures in the technique and can be in liquid form or in a solid or semi-solid dry composition. For example, the composition may be in the form of a granulate or a microgranulate. The enzymes to be included in the composition may be stabilised according to the procedures known in the technique.

As indicated above, the host cell of the invention expresses at least one PMO polypeptide of the invention, which is capable of oxidising cellulose when secreted into the extracellular medium. This host cell is able to secrete this enzyme(s) into the medium along with another/other cellulolytic enzyme(s) produced naturally or recombinantly, thus being useful for the optimisation of the biomass hydrolysis stage in fermentable sugars.

Therefore, another aspect of the invention concerns the use of the host cell of the invention, the polypeptide(s) of the invention, or the composition of the invention, for the enzymatic degradation or hydrolysis of cellulosic biomass.

The term "biomass" refers the biodegradable fraction of products, waste and residues of biological origin from agriculture (including plant substances, such as crop residues, and animal substances), forest industries (such as timber resources) and related industries including fisheries and aquaculture, as well as the biodegradable fraction of industrial and urban waste, such as municipal solid waste or paper residues. In a preferred embodiment, the biomass is straw or the organic fraction of urban solid waste. In a more preferred embodiment, the biomass is vegetable biomass, more preferably selected from the list consisting of: biomass rich in fermentable sugars, such as sugar cane; starch biomass, for example, grains or wheat straw; or maize or maize straw or maize grain or maize fibre; or barley grains or straw; or sorghum grains or straw. Biomass can also include rice, grass, branches, etc. In a more preferred embodiment, the cellulosic biomass employed in this invention comprises corn and/or sugar cane.

The polypeptide of the invention, as well as the host cell or composition of this invention, may be used in the production of monosaccharides, disaccharides and polysaccharides as chemical or fermentation raw materials, from biomass for the production of ethanol, butanol, plastics, alkanes, alkenes, or other products or intermediates.

The host cell of this invention can be used as a source of the polypeptide of the invention, and other cellulolytic enzymes, in a fermentation process with biomass.

The predominant polysaccharide in the primary cell wall of the plant biomass is cellulose, the second most abundant is hemicellulose, and the third, depending on the biomass in question, may be pectin. The secondary cell wall, produced after the cell has stopped growing, also contains polysaccharides and is reinforced by covalently cross-linked polymer lignin with hemicellulose. Cellulose is an anhydrocelobic homopolymer and thus is a linear beta-(1-4)-D-glucan, while hemicellulose includes a variety of compounds, such as xylans, xyloglucans, arabinoxylans, and mannan in complex branched structures with a range of substitutes. Although generally polymorphous, cellulose is found in plant tissue mainly as an insoluble crystalline matrix of parallel glucan chains. Hemicelluloses are normally bound by hydrogen bonds to cellulose, as well as to other hemicelluloses, which helps to stabilise the cell wall matrix. The polypeptides of the invention can be used together with other cellulolytic enzymes to degrade the cellulose component of the biomass substrate.

The degradation or hydrolysis of biomass to fermentable sugars, also known as "saccharification", by means of the polypeptide of the invention, the host cell of the invention or the composition of the invention, may be followed by a fermentation process in which the fermentable sugars obtained are used in order to finally obtain a bioproduct such as bioethanol.

Thus, another preferred embodiment of this aspect of the invention refers the use of at least one polypeptide of the invention, of the host cell of the invention or of the composition of the invention, for the degradation of biomass in a process for the production of a bioproduct.

The term "bioproduct" or "biobased products" refers to the materials, chemicals and energy derived from renewable biological resources. Examples of these bioproducts are, but are not limited to, hydrocarbon compounds in their different forms, such as aliphatic (saturated, unsaturated, cyclic) or aromatic compounds, such as alkanes, alkenes, alkyls, cyclic forms of these compounds or aromatic hydrocarbons; oxygenated substances such as alcohols (such as ethanol, butanol, sorbitol), ethers, aldehydes, ketones or carboxylic acids; nitrogenous substances such as amines, amides, nitrocompounds or nitriles; halogenated substances such as halides; organic acids (such as lactic acid, acrylic acid, acetic acid, succinic acid, glutamic acid, citric acid or propionic acid). The term "bioproducts" also includes any combination of the compounds described above, compounds derived in addition to the compounds described above by any type of physical, chemical or biological treatment, polymers of the compounds described above, compounds described above replaced by any group or functional element in one or more of their linked and branched forms.

Ethanol can be produced by the enzymatic degradation of biomass and the conversion of saccharides released into ethanol. This type of ethanol is often referred to as bioethanol. It can be used as a fuel additive or extender in mixtures of less than 1% up to 100% (a fuel substitute).

In a more preferred embodiment, the bioproduct is a biofuel. The term "biofuel", as used herein, refers to a hydrocarbon, or one of its mixtures, that can be used as a fuel and is obtained using fermentable biomass as the starting material. Examples of biofuels include, but are not limited to, ethanol or bioethanol, butanol or biobutanol and biodiesel. In a more preferred embodiment, biofuel is bioethanol.

The term "bioethanol" refers to a alcohol prepared by fermentation from fermentable biomass such as carbohydrates produced in sugar or starch crops such as maize or sugar cane.

In another aspect, this invention refers to a process to produce fermentable sugars from cellulosic biomass, referred to herein as the "first procedure in the invention", which comprises the following stages:
a) incubation of the cellulosic biomass, preferably pre-treated biomass, with the composition of the invention, with at least one polypeptide of the invention or with the host cell of the invention, and
b) recovering of fermentable sugars obtained after incubation of stage (a).

A biomass pre-treatment procedure is often required to increase enzyme access to their substrates and consequent effective hydrolysis. Pre-treatment uses a variety of techniques, including but not limited to chemical treatment (e.g. ammonium fibre explosion or exposure to a solvent), physical treatment (e.g. steam explosion at high temperatures), mechanical treatment (e.g. crushing or grinding), biological treatment, or any combination thereof, to alter the structure of cellulosic biomass and make cellulose more accessible.

The term "fermentable sugar" as used herein refers to simple sugars (monosaccharides, disaccharides, and short oligosaccharides), such as glucose, xylose, arabinose, galactose, mannose, rhamnose, sucrose, or fructose, etc. A fermentable sugar is any one that can use or ferment a microorganism. Preferably, the fermentable sugars referred to in the invention comprise at least glucose.

Another aspect of this invention refers a process for producing a bioproduct from cellulosic biomass, hereinafter referred to as the "second procedure in the invention", which comprises the following stages:
a) incubation of the cellulosic biomass, preferably pre-treated biomass, with the composition of the invention, with at least one polypeptide of the invention or with the host cell of the invention,
b) fermenting of the fermentable sugars obtained after incubation of stage (a) with at least one fermenting micro-organism, and
c) recovering the bioproduct obtained after fermentation of stage (b).

The term "fermenting" or "fermentation" as used herein refers to a biological transformation process produced by the activity of some microorganisms in which sugars such as glucose, fructose, and sucrose are converted into ethanol. The microorganisms used in this way are fermenting microorganisms that have fermentation capacity, such as yeasts of the genera *Saccharomyces, Pichia* or *Kluyveromyces,* preferably *Saccharomyces cerevisiae,* both natural and genetically modified strains for the conversion of pentoses.

The term "recovery" as used herein refers to the collection of fermentable sugars obtained after the incubation of stage (a) of the first procedure of the invention or of the bioproduct obtained after the fermentation of stage (b) of the second procedure of the invention. Recovery may be performed by any procedure known in the technique, including mechanics or manuals.

In some embodiments, the first and/or second procedure of the invention comprises, preferably, a process of pre-treatment of the biomass before stage (a). In general, a pre-treatment process will result in the components of the cellulosic material being more accessible for later stages or more digestible by enzymes after treatment in the absence of hydrolysis. Pre-treatment can be chemical, physical, mechanical or biological pre-treatment, or any mixture thereof. Preferably, the pre-treatment of the biomass to which this invention refers is done by steam explosion.

Before (i.e. in stage (a)) and/or simultaneously with the fermentation of stage (b) of the second method of invention, biomass, preferably pre-treated biomass, is hydrolysed to degrade cellulose and hemicellulose into sugars and/or oligosaccharides. The solid content during hydrolysis can be, but not be limited to, between 10-30% of the total weight, preferably between 15-25% of the total weight, more preferably between 18-22% of the total weight. Hydrolysis is performed as a process in which biomass, preferably pre-treated biomass, is incubated with at least one polypeptide of the invention, with the host cell of the invention or with the composition of the invention and thus forming the hydrolysis solution. The correct process time, temperature and pH conditions can easily be determined by a technical expert. Preferably, this hydrolysis is carried out at a temperature of between 25°C and 60 °C, preferably between 40°C and 60 °C, specifically around 50°C. The process is preferably carried out at a pH in the range 4-6, preferably pH 4.5-5.5, especially around pH 5.2. Preferably, hydrolysis is performed between 12 and 144 hours, preferably between 16 and 120 hours, more preferably between 24 and 96 hours, even more preferably between 32 and 72 hours.

Hydrolysis (stage (a)) and fermentation (stage (b) of the second method of the invention) can be performed simultaneously (SSF process) or sequentially (SHF process). According to the invention, hydrolysed biomass, and preferably pre-treated, is fermented by at least one fermenting microorganism capable of fermenting fermentable sugars, such as glucose, xylose, mannose and galactose, directly or indirectly in the desired fermentation product. Fermentation takes place preferably between 8 and 96 hours, preferably between 12 and 72 hours, more preferably between 24 and 48 hours. In another preferred embodiment, fermentation takes place at a temperature of between 20°C and 40 °C, preferably 26°C to 34 °C, particularly around 32°C. In another preferred embodiment, the pH is 3 to 6 units, preferably 4 to 5. A yeast of the *Saccharomyces cerevisiae* species is preferred for ethanolic fermentation, preferably strains that are resistant to high levels of ethanol, up to, for example, 5 or 7% vol. of ethanol or more, such as 10-15% vol. of ethanol.

In a preferred embodiment of the second process of the invention, the bioproduct is biofuel, more preferably bioethanol.

Throughout the description and the claims, the word "comprises", "consists" and its variants are not intended to exclude other technical characteristics, additives, components or steps. For experts in the art, other objects, advantages and characteristics of the invention will emerge partly from the description and partly from the practice of the invention. The following examples and figures are provided by way of illustration, and are not intended to be exhaustive of this invention.

### DESCRIPTION OF THE FIGURES

**Figure 1****.** Analysis of glucose release from pre-treated corn biomass (PCS) subjected to different cellulolytic enzymatic compositions obtained from a strain of *M. thermophila* control (C) and strains of *M. thermophila* transformed with expression vectors codifying for each of the proteins of the invention.
**Figure 2****.** Analysis of glucose release from pre-treated sugar cane biomass (PSCS) subjected to different cellulolytic enzymatic compositions obtained from a strain of *M. thermophila* control (C) and from strains of *M. thermophila* transformed with expression vectors codifying for each of the proteins of the invention.

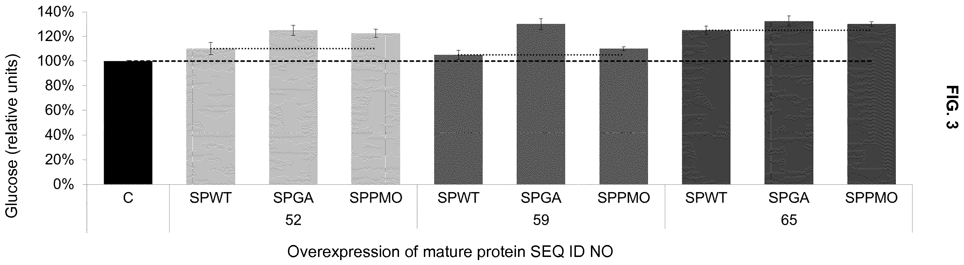

**Figure 3**. Analysis of glucose release from pre-treated corn biomass (PCS) subjected to different cellulolytic enzymatic compositions obtained from a strain of *M. thermophila* control (C) and from strains of *M. thermophila* transformed with expression vectors generated to code for different signal peptide combinations (SPWT, native signal peptide; SPGA, signal peptide of glucoamylase A; SPPMO, signal peptide of PMO-06230) and mature protein for proteins of SEQ ID NO: 52, SEQ ID NO: 59 and SEQ ID NO: 65 of the invention.

### EXAMPLES

### Example 1. Construct of the codifying expression vectors for the different polysaccharide monooxygenases of the invention together with the different signal peptides, for their expression in M. thermophila C1.

The genes of different polysaccharide monooxygenases listed in Table 1 were used to overexpress enzymes in the host cell *M. thermophila* C1 and to test the hydrolysis yields with the resulting enzymatic cocktails (secreted by the transformed cells).

Genes were synthesised *in vitro* after optimisation to eliminate restriction sites of the most common enzymes without altering the encoded amino acid sequence. The resulting genes and deduced sequences are referenced according to Table 1.

Using the SignalP tool (Petersen et al., 2011, Signal IP 4.0, Nature Methods, 8:785-786), signal peptides of all proteins were identified. The sequences of the mature proteins are referenced according to Table 1.

Prior to *in vitro* synthesis, the signal peptide of the native protein was replaced to generate variants with the signal peptide of *Aspergillus niger* glucoamylase A (glaA, Uniprot:A2QHE1) or with the signal peptide of *M. thermophila*'s own polysaccharide monooxygenase 06230 (Uniprot:G2QCJ3).

The cloning and expression of each gene followed a procedure similar to that described in the patent (PCT/ES2013/070318).

### Example 2. Cocktail effect produced by transforming strains on glucose release during enzymatic hydrolysis of different lignocellulosic substrates.

The release of fermentable sugars by cocktails produced by the parental strain of *M. thermophila* C1 (control) and by selected clones resulting from transformation with expression vectors containing coding sequences of enzymes bound to the Glucoamylase A signal peptide was compared by enzymatic hydrolysis. Pre-treated Corn Stover (PCS) or Pre-treated Sugar Cane Straw (PSCS) biomass was used as the substrate. Biomass pre-treatment was performed by steam explosion in the presence of dilute acid (Nguyen et al., 1998, Appl. Biochem. Biotechnol. 70-72; Alcantara et al. 2016, Biotechnology for Biofuels. 9:207), and the compositional analysis of the resulting biomass was performed according to the procedures described by NREL in "Standard Biomass Analytical Procedures" (http://www.nrel.gov/biomass/analytical_procedures.htmL). In order to be used in hydrolysis, the biomass was previously neutralised at a pH of 6.5. For the enzymatic hydrolysis process, 100 ml ISO vials were used with 20 g of the reaction mixture at 20% (w/w) of total solids and supplemented with 8 mg of protein per g of glucan from the cocktail coming from the strains in question. The vials with the mixture were incubated for 72 hrs at 50 °C with 150 rpm agitation in a 25 mm diameter orbital incubator (Infors HT). At the end of the process, the glucose content in the slurry samples was analysed by HPLC (Agilent Technologies, 1200 Series) using a refractive index (DIR) detector and an Aminex HPX-87 H column.

The results obtained with PCS are shown in Figure 1 and those obtained with PSCS in Figure 2, where it can be seen that all enzymes overexpressed with the glucoamylase signal peptide release more glucose than the cocktail produced by the control strain, the enzymes being of SEQ ID NO: 52, SEQ ID NO: 53, SEQ ID NO: 59, SEQ ID NO: 60 and SEQ ID NO: 65 the highest glucose release yields provided.

### Example 3. Effect of the different signal peptides on the performance of cocktails overexpressing the polysaccharide monooxygenases of the invention.

In this example was compared the cocktail yields obtained by overexpressing 3 polysaccharide monooxygenases (SEQ. ID NOs: 52, 59 and 65) fused to the different signal peptides of this invention: its native or original signal peptide (SPWT), that of *A. niger's* glucoamylase A (SPGA) and that of *M. thermophila*'s PMO-06230 (SPPMO). The enzymatic cocktail obtained from the parental strain of untransformed *M. thermophila* C1 was used as a control.

Enzymatic hydrolysis and analysis of the results were performed as explained in the previous example using Pre-treated Corn Stover (PCS) biomass.

The results obtained, as shown in Figure 3, show that the increase in sugar release also depends on the signal peptide used (and therefore on the level of overexpression obtained) with each of the enzymes tested, although they all surpassed their parental strain. The signal peptide that provided the highest yields in glucose release was SPGA, followed by SPPMO and finally SPWT.

**Table 1. Identification of the DNA and protein sequences corresponding to each of the PMOs of the invention.**

| Nucleic acid SEQ. ID NO. | Protein sequence of the immature enzyme SEQ. ID NO. | Protein sequence of the mature enzyme SEQ. ID NO. |
|---|---|---|
| 1 | 23 | 49 |
| 2 | 24 | 50 |
| 3 | 25 | 51 |
| 4 | 26 | 52 |
| 5 | 27 | 53 |
| 6 | 28 | 54 |
| 7 | 29 | 55 |
| 8 | 30 | 56 |
| 9 | 31 | 57 |
| 10 | 32 | 58 |
| 11 | 33 | 59 |
| 12 | 34 | 60 |
| 13 | 35 | 61 |
| 14 | 36 | 62 |
| 15 | 37 | 63 |
| 16 | 38 | 64 |
| 17 | 39 | 65 |
| 18 | 40 | 66 |
| 19 | 41 | 67 |
| 20 | 42 | 68 |
| 21 | 43 | 69 |
| 22 | 44 | 70 |

## Claims

1. Isolated polypeptide with polysaccharide monooxygenase activity comprising an amino acid sequence with at least 80% identity with the amino acid sequence SEQ ID NO: 65.

2. Polypeptide according to Claim 1, comprising an amino acid sequence having at least 90% identity with the amino acid sequence SEQ ID NO: 65.

3. Polypeptide according to Claim 2, comprising the amino acid sequence SEQ ID NO: 65.

4. Polypeptide according to any one of Claims 1 to 3, which further comprises attached to its N-terminal end a signal peptide, preferably the signal peptide with SEQ ID NO: 46.

5. Polypeptide according to Claim 3, consisting of the amino acid sequence SEQ ID NO: 65.

6. Polypeptide according to Claim 3, consisting of the amino acid sequence SEQ ID NO: 39.

7. Isolated polynucleotide encoding the polypeptide according to any one of Claims 1 to 6.

8. Gene construct comprising the polynucleotide according to Claim 7.

9. Gene construct according to Claim 8, wherein such gene construct is an expression vector.

10. Host cell comprising the polynucleotide according to Claim 7 or the gene construct according to any of Claims 8 or 9.

11. Host cell according to Claim 10, wherein such cell is *Myceliophthora thermophila* C1.

12. Enzymatic composition comprising the polypeptide according to any one of Claims 1 to 6.

13. Enzymatic composition according to Claim 12, which further comprises another cellulolytic enzyme.

14. Enzymatic composition according to Claim 13, wherein the cellulolytic enzyme is selected from the list consisting of: endoglucanase, beta-glucosidase, celobiohydrolase, celobiose dehydrogenase, beta-xylosidase, alpha-xylosidase, endoxylanase, endoxyloglucanase, polysaccharide monooxygenase or any combination thereof.

15. Enzymatic composition according to any one of Claims 12 to 14, wherein such composition is an enzymatic mixture expressed by the cell according to any of Claims 10 or 11.

16. Use of the polypeptide according to any one of Claims 1 to 6, of the host cell according to any of Claims 10 or 11, or of the composition according to any one of Claims 12 to 15, for the degradation of cellulosic biomass.

17. Use according to Claim 16, wherein the degradation of cellulosic biomass takes place in a bioproduct production process.

18. Use according to Claim 17, wherein the bioproduct is a biofuel.

19. Use according to Claim 18, wherein the biofuel is bioethanol.

20. Process for producing fermentable sugars from cellulosic biomass comprising the following stages:
a) incubation of cellulosic biomass with the composition according to any one of Claims 12 to 15, and
b) recovering the fermentable sugars obtained after the incubation of stage (a).

21. Process for producing a bioproduct from cellulosic biomass comprising the following stages:
a) incubation of cellulosic biomass with the composition according to any one of Claims 12 to 15,
b) fermenting the fermentable sugars obtained after the incubation of stage (a) with at least one fermenting microorganism, and
c) recovering the bioproduct obtained after the fermentation of stage (b).

22. The process according to Claim 21, wherein the bioproduct is a biofuel.

23. The process according to Claim 22, wherein the biofuel is bioethanol.
